# EUROPÄISCHE PATENTSCHRIFT

(11) **EP 0 993 306 B1**
(45) Veröffentlichungstag und Bekanntmachung des Hinweises auf die Patenterteilung: **10.09.2003**
(21) Anmeldenummer: 98937424.4
(22) Anmeldetag: 28.05.1998
(51) Int. Cl.: A61K 49/00, A61K 49/04, A61P 43/00

(54) **OLIGOMERE, PERFLUORALKYLHALTIGE VERBINDUNGEN, VERFAHREN ZU DEREN HERSTELLUNG UND IHRE VERWENDUNG IN DER NMR-DIAGNOSTIK**
PERFLUOROALKYLATED OLIGOMER COMPOUNDS, PROCESS FOR PREPARING THE SAME AND THEIR USE IN NMR DIAGNOSIS
COMPOSES OLIGOMERES PERFLUORALKYLES, LEUR PROCEDE DE PREPARATION ET LEUR UTILISATION DANS LE DIAGNOSTIC RMN

(30) Priorität: 03.07.1997 DE 19729013
(43) Veröffentlichungstag der Anmeldung: 19.04.2000
(73) Patentinhaber: SCHERING AKTIENGESELLSCHAFT, 13342 Berlin (DE)
(72) Erfinder: PLATZEK, Johannes, Dr., D-12621 Berlin (DE); NIEDBALLA, Ulrich, Dr., D-14195 Berlin (DE); RADÜCHEL, Bernd, Dr., D-13465 Berlin (DE); SCHLECKER, Wolfgang, Dr., D-12047 Berlin (DE); WEINMANN, Hanns-Joachim, Dr., D-14129 Berlin (DE); FRENZEL, Thomas, Dr., D-12247 Berlin (DE); MISSELWITZ, Bernd, Dr., D-16548 Glienicke (DE); EBERT, Wolfgang, Dr., D-15831 Mahlow (DE)
(86) Internationale Anmeldenummer: EP9803143
(87) Internationale Veröffentlichungsnummer: WO99001161

(56) Entgegenhaltungen:
- DE-A- 4 317 588
- DE-A- 19 521 945
- DE-A- 19 525 924
- DE-A- 19 603 033
- DE-A- 19 608 278

## Beschreibung

Die Erfindung betrifft die in den Patentansprüchen gekennzeichneten Gegenstände, d. h. neue, oligomere, perfluoralkylhaltige Verbindungen, pharmazeutische Mittel enthaltend diese Verbindungen, Verfahren zu deren Herstellung, und ihre Verwendung als Kontrastmittel in der ¹H-NMR-Diagnostik und -Spektroskopie, Röntgendiagnostik, Radiodiagnostik sowie als Radiotherapeutika.

Die kerrmagnetische Resonanz (NMR) ist heute eine breit angewendete, für die in vivo-Bildgebung ausgenutzte Methode der medizinischen Diagnose, mit der über die Messung der magnetischen Eigenschaften der Protonen im Körperwasser Körpergefäße und Körpergewebe (einschließlich Tumore) dargestellt werden können. Es werden hierzu z. B. Kontrastmittel eingesetzt, die durch Beeinflussung bestimmter NMR-Parameter der Körperprotonen (z. B. der Relaxationszeiten T¹ und T²) eine Kontrastverstärkung in den resultierenden Bildern bewirken bzw. diese Bilder erst lesbar machen. Vor allem kommen Komplexe paramagnetischer Ionen, wie z. B. Gadolinium-enthaltende Komplexe (z. B. Magnevist®) aufgrund des Effektes der paramagnetischen Ionen auf die Verkürzung der Relaxationszeiten zur Anwendung. Ein Maß für die Verkürzung der Relaxationszeit ist die Relaxivity, die in mM⁻¹ · sec⁻¹ angegeben wird.

Paramagnetische Ionen, wie z. B. Gd³⁺, Mn²⁺, Cr³⁺, Fe³⁺ und Cu²⁺ können nicht in freier Form als Lösungen verabreicht werden, da sie hoch toxisch sind. Um diese Ionen für eine in vivo-Anwendung geeignet zu machen, werden sie in der Regel komplexiert, was erstmalig in EP 0 071 564 A1 beschrieben wird (Komplexierung mit Aminopolycarbonsäuren, z. B. mit Diethylentriamin-pentaessigsäure [DTPA]). Das Di-N-methylglucaminsalz des Gd-DTPA-Komplexes ist unter dem Namen Magnevist® bekannt und wird u.a. zur Diagnose von Tumoren im menschlichen Gehirn und in der Niere angewandt.

Das in der französischen Patentschrift 25 39 996 beschriebene Megluminsalz der Gd-DOTA (Gadolinium-III-Komplex des 1,4,7,10-Tetracarboxymethyl-1,4,7,10-tetraazacyclododecans) ist ein weiteres Kontrastmittel, das sich in der Kernspintomographie ebenfalls sehr gut bewährt hat und unter dem Namen Dotarem® registriert wurde.

Diese Kontrastmittel sind jedoch nicht für alle Anwendungsfälle befriedigend einzusetzen. So verteilen sich die zur Zeit klinisch eingesetzten Kontrastmittel für die modernen bildgebenden Verfahren Kernspintomographie (MRI) und Computertomographie (CT) wie z.B. Magnevist®, Pro Hance®, Ultravist® und Omniscan® im gesamten extrazellulären Raum des Körpers (im Intravasalraum und im Interstitium).

Besonders zur Darstellung von Gefäßen sind jedoch Konstrastmittel wünschenswert, die sich bei Applikation in den vasalen Raum (Gefäßraum) auch ausschließlich in diesem verteilen und ihn damit markieren (sog. blood-pool-agents).

Es wurde versucht, diese Probleme durch Verwendung von Komplexbiianern, die an Makro- oder Biomoleküle gebunden sind, zu lösen. Damit war man bisher nur sehr begrenzt erfolgreich.

So ist beispielsweise die Anzahl paramagnetischer Zentren in den Komplexen, die in den EP 0 088 695 A1 und EP 0 150 844 A1 beschrieben sind, für eine zufriedenstellende Bildgebung nicht ausreichend.

Erhöht man die Anzahl der benötigten Metallionen durch mehrfache Einführung komplexierender Einheiten in ein makromolekulares Biomolekül, so ist das mit einer nicht tolerierbaren Beeinträchtigung der Affinität und/oder Spezifizität dieses Biomoleküls verbunden [J. Nucl. Med. 24, 1158 (1983)].

Makromolekulare Kontrastmittel für die Angiographie, wie Albumin-Gd-DTPA, werden in Radiology 1987; 162: 205 beschrieben. Albumin-Gd-DTPA zeigt jedoch 24 Stunden nach intravenöser Injektion bei der Ratte eine Anreicherung im Lebergewebe, die fast 30 % der Dosis ausmacht. Außerdem werden in 24 Stunden nur 20 % der Dosis eliminiert.

Das Makromolekül Polylysin-Gd-DTPA (EP 0 233 619 A1) kann ebenfalls als blood-pool-agent eingesetzt werden. Diese Verbindung besteht jedoch herstellungsbedingt aus einem Gemisch von Molekülen verschiedener Größe. Bei Ausscheidungsversuchen bei der Ratte konnte gezeigt werden, daß dieses Makromolekül unverändert durch glomeruläre Filtration über die Niere ausgeschieden wird. Synthesebedingt enthält Polylysin-Gd-DTPA aber auch Makromoleküle, die so groß sind, daß sie bei der glomerulären Filtration die Kapillaren der Niere nicht passieren können und somit im Körper zurückbleiben.

Auch makromolekulare Kontrastmittel auf der Basis von Kohlenhydraten, z.B. Dextran, sind beschrieben worden (EP 0 326 226 A1). Der Nachteil dieser Verbindungen liegt darin, daß diese in der Regel nur ca. 5 % des signalverstärkenden paramagnetischen Kations tragen.

Aufgabe der Erfindung war es daher, neue Kontrastmittel zur Verfügung zu stellen, die die genannten Nachteile nicht aufweisen und insbesondere bei der ¹H-NMR-Diagnostik eine höhere Protonen-Relaxivity zeigen und damit bei einer Steigerung der Signalintensität eine Reduzierung der Dosis erlauben. Ferner sollen die Kontrastmittel stabil sein, gut verträglich und vor allem organspezifische Eigenschaften aufweisen, wobei einerseits ihre Retention in den zu untersuchenden Organen ausreichend sein soll, um bei geringer Dosierung die für eine zweifelsfreie Diagnose notwendige Anzahl an Bildern zu erhalten, andererseits aber anschließend eine möglichst schnelle und weitestgehend vollständige Ausscheidung der Metalle aus dem Körper gewährleistet sein soll.

Die Aufgabe der Erfindung wird mit den oligomeren, perfluoralkylhaltigen Verbindungen der allgemeinen Formel I gelöst:

A-R^{F} (I)

worin
- A ein Molekülteil ist, der 2 - 6 Metallkomplexe enthält, die direkt oder über einen Linker an ein Stickstoffatom einer ringförmigen Gerüstkette gebunden sind
   und
- R^{F} eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4 - 30 steht,
**dadurch gekennzeichnet**,
daß das Molekülteil A die folgende Struktur aufweist: 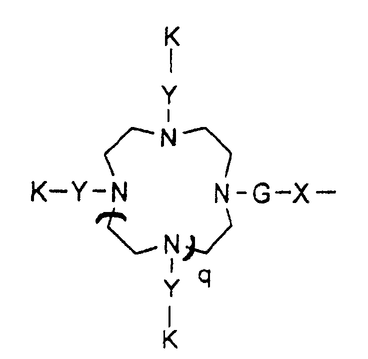 wobei
- q eine Zahl 0,1,2 oder 3 ist,
- K für einen Komplexbildner oder Metallkomplex oder deren Salze organischer und/oder anorganischer Basen oder Aminosäuren oder Aminosäureamide steht,
- X eine direkte Bindung zur Perfluoralkylgruppe, eine Phenylengruppe oder eine C₁-C₁₀-Alkylenkette ist, die gegebenenfalls 1 - 15 Sauerstoff-, 1 - 5 Schwefelatome, 1 - 10 Carbonyl-, 1 - 10 (NR)-, 1 - 2 NRSO₂-, 1 - 10 CONR-, 1 Piperidin- 1 - 3 SO₂-, 1 - 2 Phenylengruppen enthält oder gegebenenfalls durch 1 - 3 Reste R^{F} substituiert ist, worin R fiir ein Wasserstoffatom, eine Phenyl-, Benzyl- oder eine C₁-C₁₅-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1 - 5 Methoxy-, 1 - 3 Carboxy-, 1 - 3 R^{F}-Reste substituiert ist
- Y eine direkte Bindung oder eine Kette der allgemeinen Formel II oder III ist: 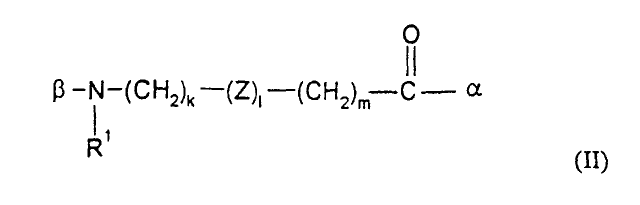 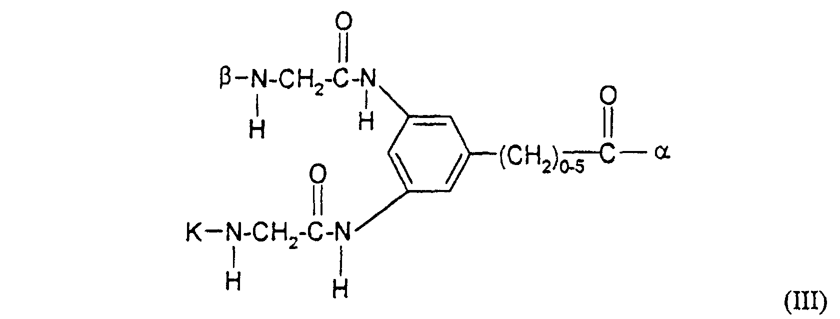 worin
   ■ R¹ ein Wasserstoffatom, eine Phenylgruppe, eine Benzylgruppe oder eine C₁-C₇ Alkylgruppe ist, die gegebenenfalls substituiert ist mit einer Carboxy-, einer Methoxy- oder einer Hydroxygruppe,
   ■ Z eine direkte Bindung, eine Polyglycolethergruppe mit bis zu 5 Glycoleinheiten oder ein Molekülteil der allgemeinen Formel IV ist

      ―CH(R²)- (IV)

      worin R² eine C₁-C₇-Carbonsäure, eine Phenylgruppe, eine Benzylgruppe oder eine -(CH₂)₁₋₅-NH-K-Gruppe ist,
   ■ α die Bindung an das Stickstoffatom der Gerüstkette, β die Bindung zum Komplexbildner oder Metallkomplex K darstellt,
   ■ und in der die Variablen k und m für natürliche Zahlen zwischen 0 und 10 und 1 fiir 0 oder 1 stehen,
   und wobei
- G eine CO- oder SO₂-Gruppe ist.

Diese Verbindungen weisen eine überraschend hohe Protonen-Relaxivity im Vergleich zu den im Handel befindlichen ¹H-NMR-Kontrastmitteln Magnevist®, Dotarem®, Omniscan® und Pro Hance® auf (hier liegen die Werte für die T¹-Relaxivity zwischen 3,5 - 4,9 [mM⁻¹ · sec⁻¹, 39 °C, 0,47 T], siehe dazu Tabelle 1 vor Beispiel 28).

Daneben sind die erfindungsgemäßen Verbindungen in hervorragender Weise zur Erkennung und Lokalisierung von Gefäßkrankheiten geeignet, da sie sich bei Applikation in den Intravasalraum ausschließlich in diesem verteilen. Die erfindungsgemäßen Verbindungen ermöglichen es, mit Hilfe der Kernspintomographie gut durchblutetes von schlecht durchblutetem Gewebe abzugrenzen und somit eine Ischämie zu diagnostizieren. Auch infarziertes Gewebe läßt sich aufgrund seiner Anämie vom umliegenden gesunden oder ischämischen Gewebe abgrenzen, wenn die erfindungsgemäßen Kontrastmittel angewandt werden. Dies ist von besonderer Bedeutung, wenn es z.B. darum geht, einen Herzinfarkt von einer Ischämie zu unterscheiden.

Gegenüber den bisher als blood-pool-agents eingesetzten makromolekularen Verbindungen, wie beispielsweise Gd-DTPA-Polylysin, zeigen die erfindungsgemäßen Verbindungen ebenfalls eine höhere T¹-Relaxivity und zeichnen sich somit durch eine Steigerung der Signalintensität bei der NMR-Bildgebung aus. Da sie daneben eine verlängerte Retention im Blutraum haben, können sie auch in relativ kleinen Dosierungen (von z. B. ≤ 50 µmol Gd/kg Körpergewicht) appliziert werden. Vor allem aber werden die erfindungsgemäßen Verbindungen rasch und weitestgehend vollständig aus dem Körper eliminiert.

Ferner zeigte sich, daß sich die erfindungsgemäßen Verbindungen in Gebieten mit erhöhter Gefäßpermeabilität, wie z.B. in Tumoren, anreichern; sie erlauben Aussagen über die Perfusion von Geweben, geben die Möglichkeit, das Blutvolumen in Geweben zu bestimmen, die Relaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA (Magnevist®), zu erhalten. Aus diesen Gesichtspunkten ergeben sich auch die Einsatzgebiete bei den modernen bildgebenden Verfahren Kernspintomographie und Computertomographie: spezifischere Diagnose von malignen Tumoren, frühe Therapiekontrolle bei zytostatischer, antiphlogistischer oder vaso-dilatativer Therapie, frühe Erkennung von minderperfundierten Gebieten (z.B. im Myokard), Angiographie bei Gefäßerkrankungen, und Erkennung und Diagnose von (sterilen oder infektiösen) Entzündungen.

Außerdem zeigte sich, daß die Verbindungen der vorliegenden Erfindung nicht nur als blood-pool-agents geeignet sind, sondern auch als lymphspezifische NMR-Kontrastmittel (Lymphographika) in hervorragender Weise eingesetzt werden können.

Die Darstellung von Lymphknoten ist von zentraler Bedeutung für die frühe Erkennung des metastatischen Befalls bei Krebspatienten. Die erfindungsgemäßen Kontrastmittel erlauben es, kleine Metastasen in nicht-vergrößerten Lymphknoten (<2 cm) von Lymphknotenhyperplasien ohne malignen Befall zu unterscheiden. Dabei können die Kontrastmittel intravasal oder interstitiell/intrakutan appliziert werden. Die Applikation interstitiell/intrakutan hat den Vorteil, daß die Substanz direkt vom streuenden Herd (z.B. Primärtumor) durch die entsprechenden Lymphwege in die potentiell betroffenen, regionalen Lymphknotenstationen transportiert wird. Gleichfalls kann mit einer geringen Dosis eine hohe Konzentration des Kontrastmittels in den Lymphknoten erreicht werden.

Die erfindungsgemäßen Verbindungen erfüllen alle Voraussetzungen, die von Kontrastmitteln in der indirekten NMR-Lymphographie verlangt werden: gute lokale Verträglichkeit, rascher Abtransport vom Injektionsort, rasche und weitestgehend vollständige Ausscheidung aus dem Gesamtorganismus. Ferner zeigen sie eine hohe Anreicherung über mehrere Lymphknotenstationen und erlauben damit relevante diagnostische Aussagen. So konnte am Meerschweinchenmodell eine hohe Anreicherung über mehrere Lymphknotenstationen (popliteal, inguinal, iliakal) nach s.c. Gabe (2,5-10 µmol/kg Körpergewicht, Injektion in Zwischenzehenräume der Hinterpfote) gezeigt werden. In besonders geeigneten Fällen wurden so in der zweiten (inguinal) und dritten (iliakal) Station noch Gadoliniumkonzentrationen von jeweils ≥ 200 bzw. ≥ 300 µmol/l erhalten. Üblicherweise sind mit den erfindungsgemäßen Verbindungen Lymphknotenkonzentrationen bis zu 1000 µmol/l zu erhalten.

Beim Menschen können die erfindungsgemäßen Verbindungen lokal (entweder subkutan oder direkt perkutan in das interessierende Gewebe) injiziert werden. Es sind mehrere Injektionsorte (Quaddeln) mit einem jeweiligen Injektionsvolumen von 0,2 bis 1 ml gruppiert um den zu untersuchenden Bereich herum (z.B. Tumor) möglich. Das injizierte Gesamtvolumen sollte dabei 5 ml in keinem Fall übersteigen. Das bedeutet, daß in der Formulierung eine Metallkonzentration von 75 - 100 mmol/l vorliegen muß, damit eine potentielle klinische Dosis von 5 - 10 µmol/kg Körpergewicht mit diesem Volumen appliziert werden kann. Der Applikationsort hängt davon ab, ob ein bestimmtes Lymphabflußgebiet aus dem dazu zugeordneten Gewebe spezifisch angefärbt werden soll (z.B. bei gynäkologischen oder Rektumtumoren), oder ob das unbekannte Abflußgebiet einer bestimmten Läsion (ergo der Bereich für eine mögliche therapeutische Intervention z.B. beim Melanom oder Mammakarzinom) dargestellt werden soll.

Für die MR-Bildgebung werden im normalen Lymphknotengewebe, wo die Anreicherung der Verbindung erfolgt, Gadoliniumkonzentrationen von mindestens 40 µmol/l und höchstens 2500 µmol/l benötigt. Die Bildgebung kann (je nach Injektionsort und Gewebe) nach 30 Minuten oder bis zu 4 - 6 Stunden nach Injektion der erfindungsgemäßen Verbindungen erfolgen. Da mit den erfindungsgemäßen Verbindungen von Gadoliniumkomplexen vor allem die T¹-Relaxationszeiten der Protonen des Wassers des Lymphknotengewebes beeinflußt werden, sind T¹-gewichtete Sequenzen am besten in der Lage, ein NMR-Enhancement der Lymphknotenstationen nachzuweisen. Da Lymphknoten sehr häufig in Fettgewebe eingebettet sind, und dieses eine sehr hohe Signalintensität auf solchen Sequenzen besitzt, bieten sich fett-unterdrückte Meßmethoden an. Paramagnetische Gadoliniumkomplexe in Verbindung mit fett-unterdrückten, T¹-gewichteten Meßsequenzen haben gegenüber Formulierungen von superparamagnetischen Eisenoxidpartikeln den großen Vorteil, daß sie NMR-Bilder mit höherer räumlicher Auflösung, mit geringeren Distorsionsartefakten (aufgrund von Suszeptibilitätsartefakten) und mit kürzerer Aufnahmezeit erlauben.

Da eine positive Markierung der Lymphknoten erfolgt (d.h. Signalanstieg), sind auch NMR-Aufnahmen ohne Kontrastmittel zum Vergleich nicht mehr zwingend notwendig, und die Gesamt-Untersuchungszeit pro Patient kann verkürzt werden.

Von den neuen erfindungsgemäßen oligomeren, perfluoralkylhaltigen Verbindungen der allgemeinen Formel I sind diejenigen mit q in der Bedeutung der Ziffer 1 bevorzugt.

Als Molekülteil X in der Bedeutung einer Alkylenkette seien die folgenden Strukturen beispielhaft genannt:

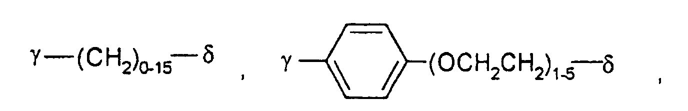

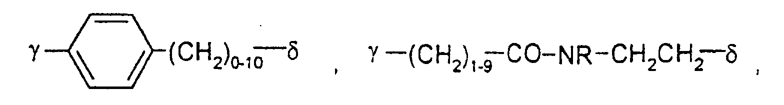

γ ―(CH₂)₁₋₁₅-S-CH₂CH₂―δ , γ ―(CH₂)₁₋₁₀-NR-SO₂-δ,

γ―(CH₂)₁₋₁₀-OCH₂CH₂―δ , γ-CH₂NH(COCH₂NH)₁₋₅-CO-CH₂-OCH₂CH₂-δ

γ―(CH₂)₁₋₁₀―N(COR)-CH₂CH₂―δ,

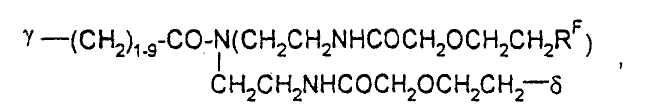

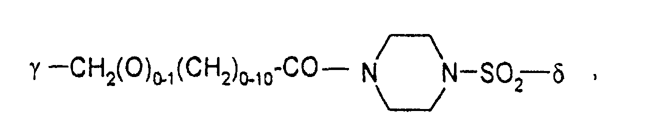

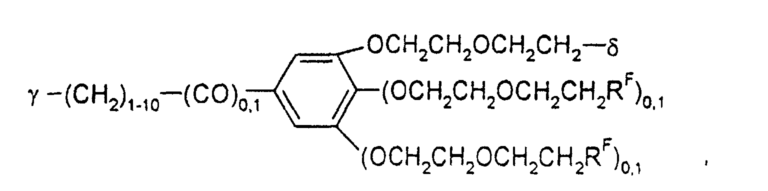

wobei γ an G und δ an R^{F} bindet R für ein Wasserstoffatom, eine Phenyl-, Benzyl- oder eine C₁-C₁₅-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1 - 5 Methoxy-, 1 - 3 Carboxy-, 1 - 3 R^{F}-Reste substituiert ist.

Bevorzugte Molekülteile X sind Alkylenketten, die 1-10 CH₂CH₂O oder 1-5 COCH₂NH-Gruppen enthalten.

Besonders bevorzugt für X sind die direkte Bindung sowie die folgenden Strukturen:

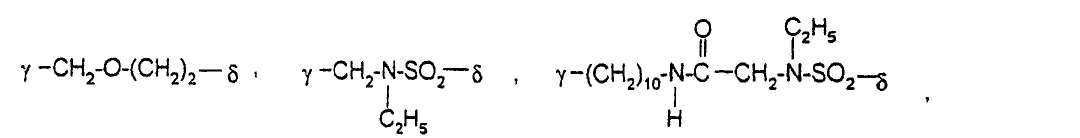

γ―(CH₂)₁₀-O-(CH₂)₂―δ ,

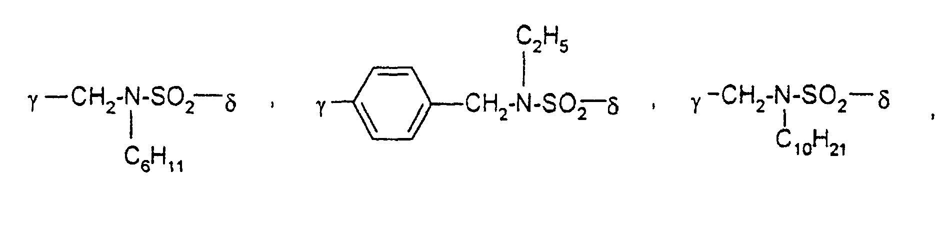

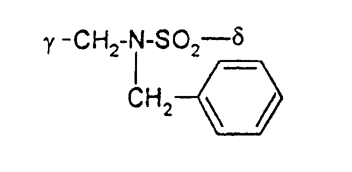

Als Molekülteil Y seien die folgenden Strukturen genannt:

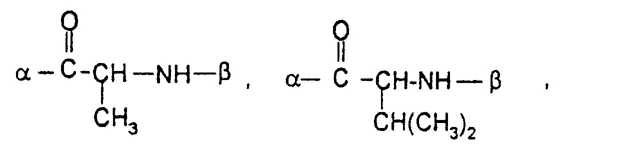

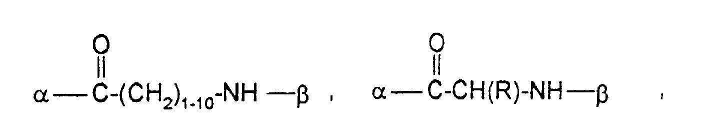

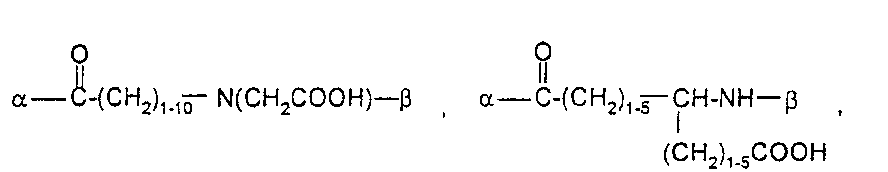

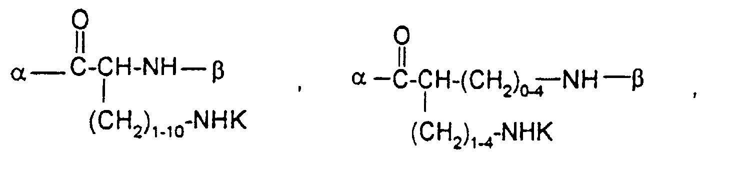

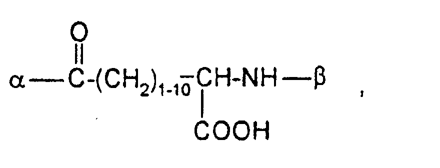

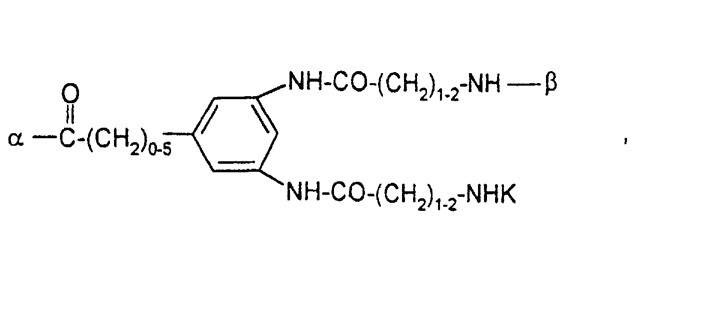

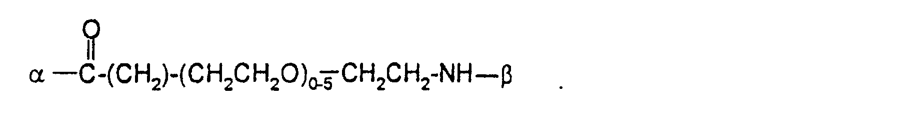

Bevorzugte Molekülteile Y sind die direkte Bindung sowie die folgenden Strukturen:

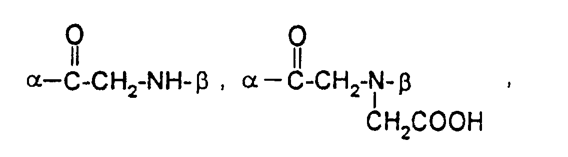

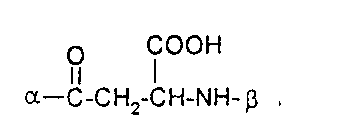

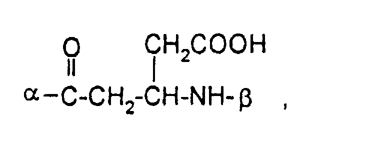

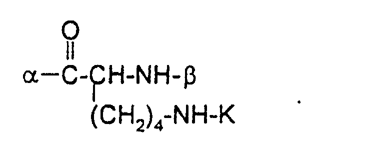

Die Komplexbildner bzw. Metallkomplexe haben die folgenden Strukturen: 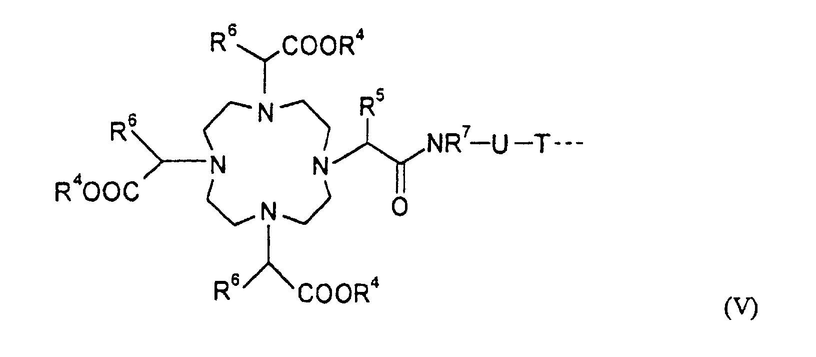 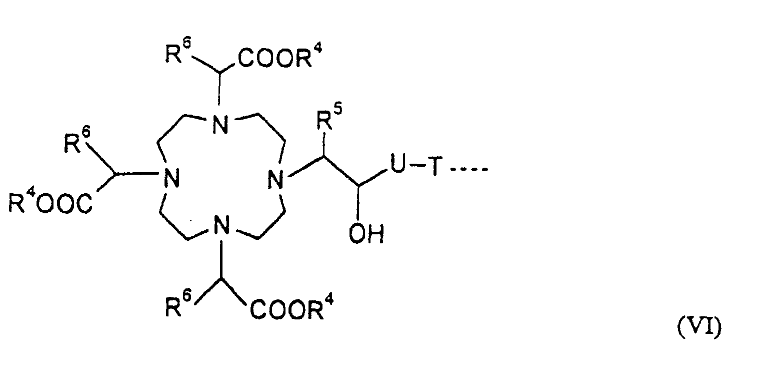 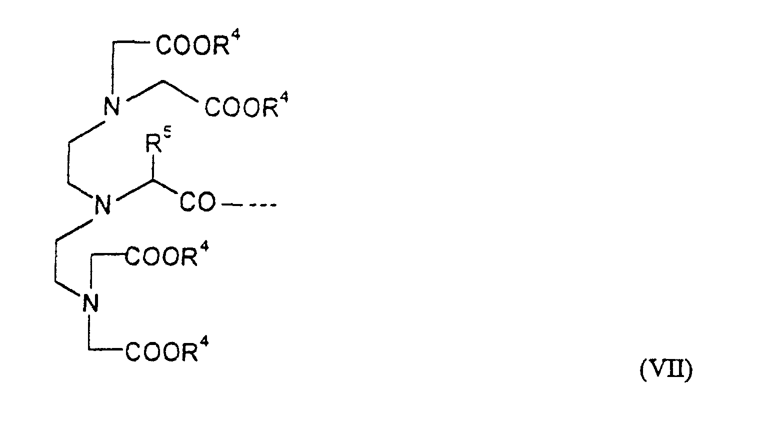 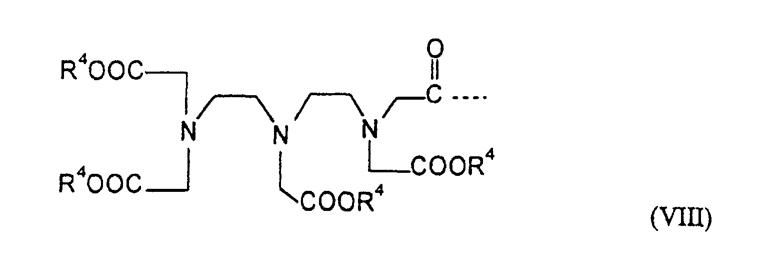 wobei
- R⁴ unabhängig voneinander ein Wasserstoffatom oder ein Metallionenäquivalent der Elemente der Ordnungszahlen 20 - 32, 37 - 39, 42 - 44, 49 oder 57 - 83 ist,
- R⁵ ein Wasserstoffatom oder eine geradkettige, verzweigte, gesättigte oder ungesättigte C₁-C₃₀-Alkylkette ist, die gegebenenfalls substituiert ist durch 1 -5 Hydroxy-, 1 - 3 Carboxy- oder 1 Phenylgruppe(n) und/oder gegebenenfalls durch 1 - 10 Sauerstoffatome, 1 Phenylen- oder 1 Phenylenoxygruppe unterbrochen ist
- R⁶ ein Wasserstoffatom, ein geradkettiger oder verzweigter C₁-C₇-Alkylrest, ein Phenyl- oder Benzylrest ist,
- R⁷ ein Wasserstoffatom, eine Methyl- oder Ethylgruppe ist, die gegebenenfalls substituiert ist durch eine Hydroxy- oder Carboxygruppe,
- U eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1 Thioharnstoff-, 1 - 2 Carboxyalkylimino-, 1 - 2 Estergruppen, 1 - 10 Sauerstoff-, 1 - 5 Schwefel- und/oder 1 - 5 Stickstoffatome enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppen substituierte, geradkettige, verzweigte, gesättigte oder ungesättigte C₁-C₂₀-Alkylengruppe ist, wobei die gegebenenfalls enthaltenen Phenylengruppen durch 1 - 2 Carboxy-, 1 - 2 Sulfon- oder 1 - 2 Hydroxygruppen substituiert sein können
- T für eine -CO-β, -NHCO-β oder -NHCS-β-Gruppe steht, wobei β die Bindungsstelle an Y darstellt.

Als bevorzugte Substituenten R⁵ seien genannt:
das Wasserstoffatom, die Methyl-, Ethyl-, Propyl-, Isopropyl-, Benzyl-, Phenyl-, -CH₂CH₂OH-,
   -CH₂OH-, -CH₂-COOH-, -COO_{H}-, -CH₂CHOHCH₂OH-, -CH₂O-CH₂CH₂OCH₃-, -CH₂OCH₃-, -CH₂-O-C₆H₄-COOH-Gruppe.

Als bevorzugte Substituenten R⁶ seien genannt:
das Wasserstoffatom, die Methyl-, Ethyl-, Propyl-, Isopropyl-, Benzyl- und Phenylgruppe.

Als bevorzugte Substituenten R7 seien genannt:
das Wasserstoffatom, die Methyl-, Ethyl-, -CH₂CH₂OH-, -CH₂-COOH-gruppe.

Bevorzugt enthält die für U stehende C₁-C₁₀-Alkylenkette die folgenden Gruppen:
-CH₂NHCO-, -NHCOCH₂O-, -NHCOCH₂OC₆H₄-, -N(CH₂CO₂H)-,
-CH₂OCH₂-,
-NHCOCH₂C₆H₄-, -NHCSNHC₆H₄-, -CH₂OC₆H₄-, -CH₂CH₂O-
und/oder ist durch die Gruppen -COOH, -CH₂COOH substituiert.

Als Beispiele für U seien folgende Gruppen angeführt:
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-, 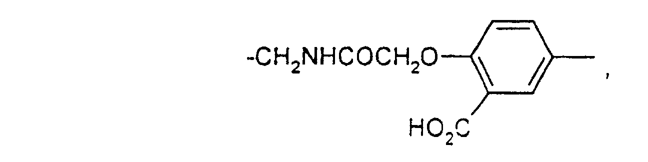 -CH₂NHCSNH-C₆H₄-CH(CH₂COOH)CH₂-,
-CH₂OC₆H₄-N(CH₂COOH)CH₂-,
-CH₂NHCOCH₂O(CH₂CH₂O)₄-C₆H₄-,
-CH₂O-C₆H₄-,
-CH₂CH₂-O-CH₂CH₂-, -CH₂CH₂-O-CH₂CH₂-O-CH₂CH₂-,

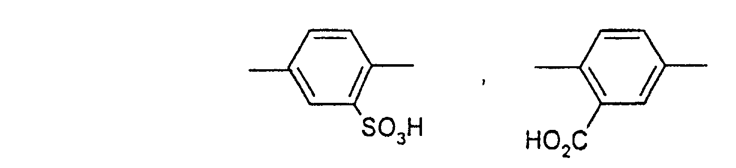

Als Beispiele für K seien die folgenden Strukturen aufgeführt: 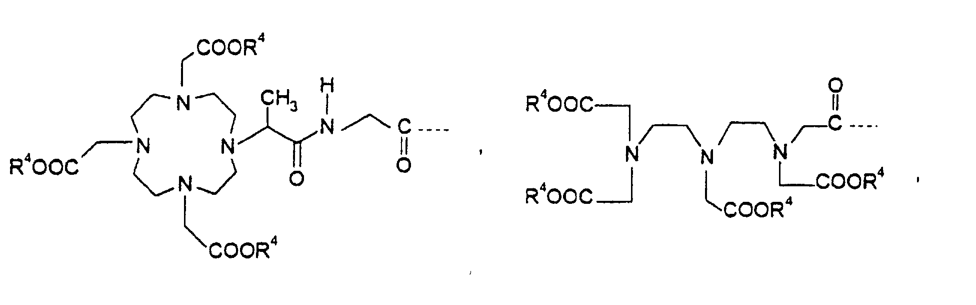 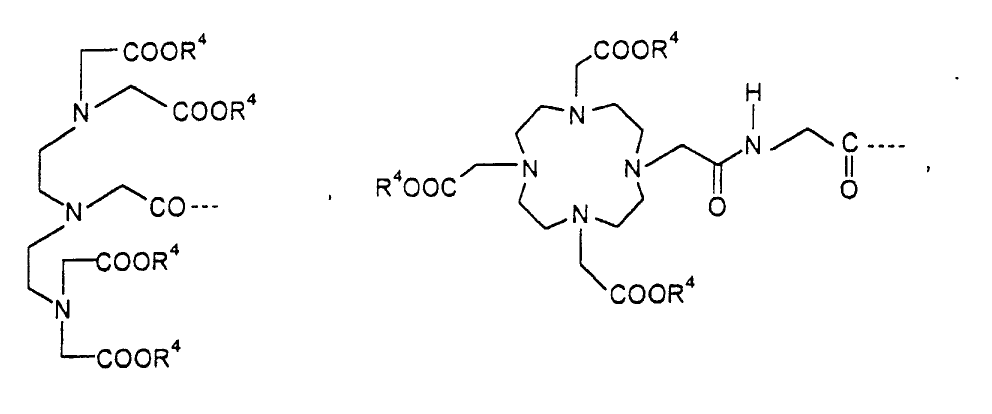 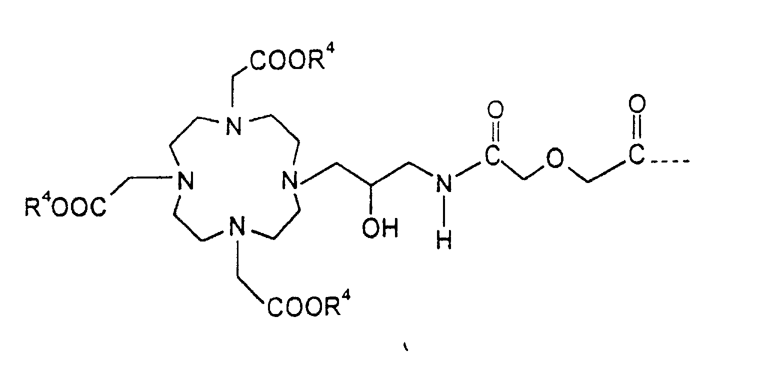 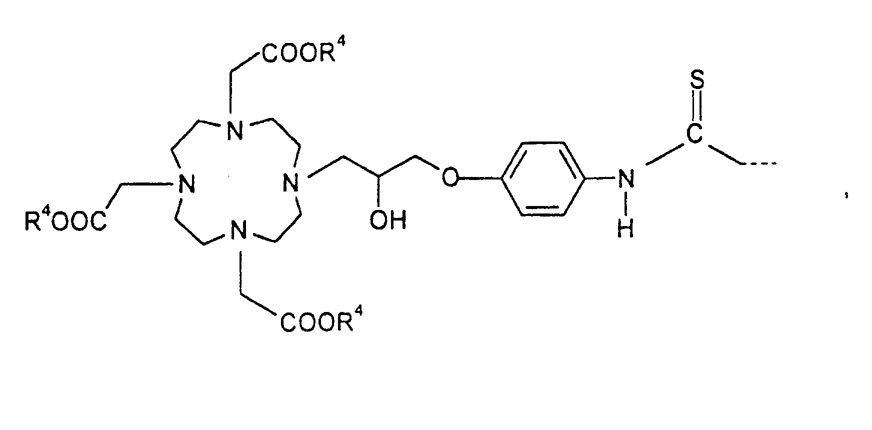 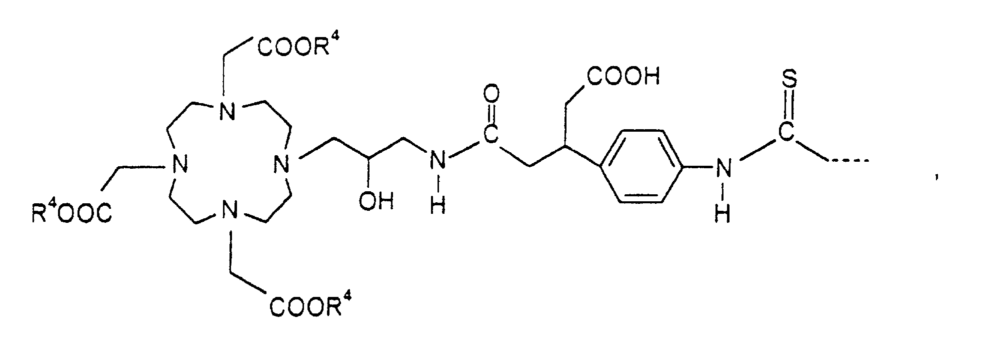 wobei die fünf erstgenannten bevorzugt sind.

R^{F} steht fiir eine -CₙF₂ₙE-Kette, in der E für ein endständiges F-, Cl, Br, I oder H-atom, bevorzugt für ein F-atom und n für die Zahlen 4-30, bevorzugt 6-12, steht. Bevorzugte R^{F}-Ketten sind die folgenden:
-C₆F₁₃, -C₈F₁₇, -C₁₀F₂₁ und -C₁₂F₂₅.

Ist das erfindungsgemäße Mittel zur Anwendung in der NMR-Diagnostik bestimmt, so muß das Zentralion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)-, Mangan(II)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21 - 29, 39, 42, 44, 57 - 83 enthalten, geeignet sind; dies sind beispielsweise das Lanthan(III)-ion und die oben genannten Ionen der Lanthanidenreihe.

Die restlichen aciden Wasserstoffatome, das heißt diejenigen, die nicht durch das Zentralion substituiert worden sind, können gegebenenfalls ganz oder teilweise durch Kationen von anorganischen und/oder organischen Basen, Aminosäuren oder Aminosäureamiden ersetzt sein.

Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der erfindungsgemäßen Verbindungen der allgemeinen Formel I erfolgt dadurch, daß man Verbindungen der allgemeinen Formel I'

A'-R^{F} (I')

worin A' für einen Rest 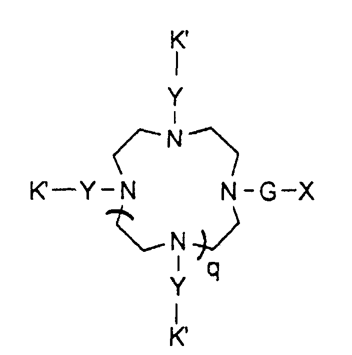 steht und K' für K steht mit R⁴ in der Bedeutung eines Wasserstoffatoms oder einer Carboxyschutzgruppe,
nach Abspaltung der gegebenenfalls vorhandenen Schutzgruppen in an sich bekannter Weise mit mindestens einem Metalloxid oder Metallsalz eines Elementes der Ordnungszahlen 20 - 32, 37 - 39, 42 - 44, 49 oder 57 - 83 umsetzt und gegebenenfalls anschließend in den so erhaltenen Komplexverbindungen noch vorhandene acide Wasserstoffatome ganz oder teilweise durch Kationen von anorganischen und/oder organischen Basen, Aminosäuren oder Aminosäureamiden substituiert.

Steht K für einen Tetraazamakrocyclus, kann die Synthese der erfindungsgemäßen Verbindungen auch in der Weise erfolgen, daß man eine Verbindung der allgemeinen Formel I" 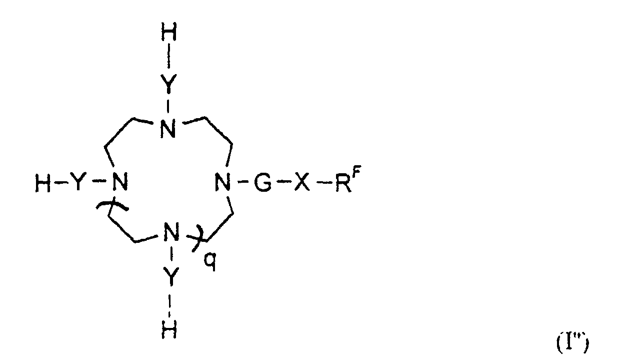 mit einem Komplex V' oder VI' umsetzt, 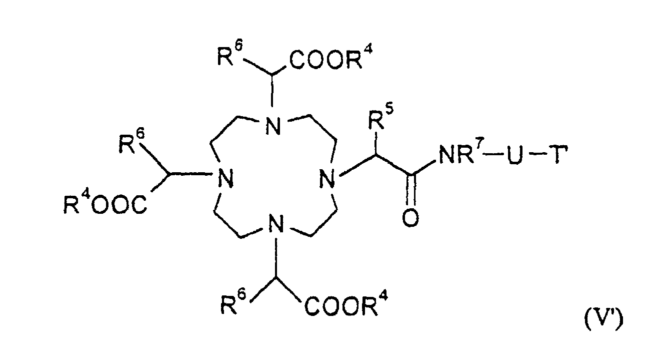 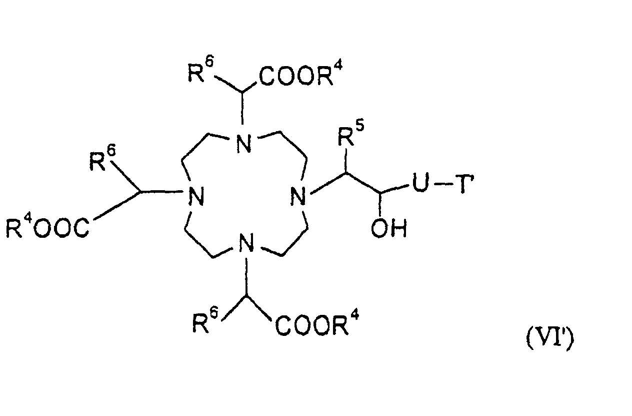 wobei T' für eine -C*O-, -COOH, -N=C=O- oder -N=C=S-Gruppe und -C*O fiir eine aktivierte Carboxylgruppe steht,
mit der Maßgabe, daß mindestens zwei (bei zweiwertigen Metallen) bzw. drei (bei dreiwertigen Metallen) der Substituenten R⁴ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen.

Als Beispiele für eine aktivierte Carbonylgruppe C*O seien Anhydrid, p-Nitrophenylester, N-Hydroxysuccinimidester, Pentafluorphenylester und Säurechlorid genannt.

Falls R⁴ für eine Säureschutzgruppe steht, kommen niedere Alkyl-, Aryl- und Aralkylgruppen, beispielsweise die Methyl-, Ethyl-, Propyl-, Butyl-, Phenyl-, Benzyl-. Diphenylmethyl-, Triphenylmethyl-, bis-(p-Nitrophenyl)-methylgruppe, sowie Trialkylsilylgruppen in Frage.

Die gegebenenfalls gewünschte Abspaltung der Schutzgruppen erfolgt nach den dem Fachmann bekannten Verfahren (s. z.B. E. Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester in wäßrig-alkoholischer Lösung bei Temperaturen von 0 °C bis 50 °C, saure Verseifung mit Mineralsäuren oder im Fall von tert.-Butylestern mit Hilfe von Trifluoressigsäure.

Die Einführung der gewünschten Metallionen erfolgt in der Weise, wie sie z.B. in der Deutschen Offenlegungsschrift 34 01 052 offenbart worden ist, indem man das Metalloxid oder ein Metallsalz (beispielsweise das Nitrat, Acetat, Carbonat, Chlorid oder Sulfat) des Elements der Ordnungszahlen 20 - 32, 37-39, 42-44, 57 - 83 in Wasser und/oder einem niederen Alkohol (wie Methanol, Ethanol oder Isopropanol) löst oder suspendiert und mit der Lösung oder Suspension der äquivalenten Menge des komplexbildenden Liganden umsetzt und anschließend, falls gewünscht, vorhandene acide Wasserstoffatome der Säuregruppen durch Kationen von anorganischen und/oder organischen Basen, Aminosäuren oder Aminosäureamiden substituiert.

Die Einführung der gewünschten Metallionen kann sowohl auf der Stufe der allgemeinen Formel I' oder vor der Kopplung der Strukturteile K, d.h. auf der Stufe der Herstellung der Komplexe V' oder VI' erfolgen.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren, wie zum Beispiel Glycinamid.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Reinigung der so erhaltenen Komplexe erfolgt, gegebenenfalls nach Einstellung des pH-Wertes durch Zusatz einer Säure oder Base auf pH 6 bis 8, bevorzugt ca. 7, vorzugsweise durch Ultrafiltration mit Membranen geeigneter Porengröße (z.B. Amicon® YM1, Amicon®YM3), Gelfiltration an z.B. geeigneten Sephadex®-Gelen oder durch HPLC an Kieselgel oder reverse-phase Material.

Im Falle von neutralen Komplexverbindungen ist es häufig von Vorteil, die oligomeren Komplexe über einen Anionenaustauscher, beispielsweise IRA 67 (OH⁻-Form) und gegebenenfalls zusätzlich über einen Kationenaustauscher, beispielsweise IRC 50 (H⁺-Form) zur Abtrennung ionischer Komponenten zu geben.

Die Synthese der Verbindungen der allgemeinen Formel I', d.h. die Verknüpfung von Komplexbildnern an Verbindungen der Formel I", erfolgt ebenso wie die Kopplung der Metallkomplexe der allgemeinen Formel K' an die Verbindungen der allgemeinen Formel I" analog literaturbekannter Methoden, wie sie z.B. beschrieben sind in US-5,135,737, H. Takalo et al, Bioconjugate Chem. 1994, 5, 278; EP 0430863; EP 0331616; WO 96/01655; EP 0271 180; US-5,364,613; WO 95/17451 und WO 96/02669. Sie wird in Lösungsmitteln wie z.B. Wasser. Methylenchlorid. Acetonitril, Chloroform. DMS0, Pyridin, Ethanol/Wasser, Ethanol/Acetonitril, Dioxan, DMF, THF, niedere Alkohole, Tetramethylharnstoff, N-Methylpyrrolidon, Polyethylenglykole, 1,2-Dimethoxyethan, Dimethylacetamid, Formamid, 1,2-Dichlorethan oder - falls möglich - deren Mischungen mit Wasser bei Temperaturen von -10 °C bis 100 °C, bevorzugt 0 bis 50 °C, besonders bevorzugt bei Raumtemperaturen innerhalb von 5 Minuten bis 72 Stunden, bevorzugt 1 bis 24 Stunden, besonders bevorzugt 1 bis 14 Stunden, gegebenenfalls unter Zusatz einer organischen oder anorganischen Base, wie z.B. aromatischen oder aliphatischen Aminen, Alkali- oder Erdalkali-hydroxiden, -carbonaten oder -hydrogencarbonaten und quartären Ammoniumhydroxiden durchgeführt. Beispielhaft genannt seien Triethylamin, Diisopropyl-N-ethylamin (Hünig-Base), N-Methylmorpholin, Tributylamin, Tetramethylethylendiamin, Pyridin, Lutedin, 2,4,6-Trimethylpyridin, 4-Dimethylaminopyridin, N-Methylimidazol, Tetramethylguanidin, DBU, Lithium-, Natrium-, Kalium, Calcium-, Magnesium-, Bariumhydroxid, -carbonat, -hydrogencarbonat. Die Umsetzung kann auch in den dem Fachmann bekannten Pufferlösungen, vorzugsweise bei pH 8 bis 11, besonders bevorzugt bei pH 8,5 bis 9 erfolgen. Die pH-Wert-Einhaltung erfolgt bevorzugt unter Verwendung eines pH-Staten.

Erfolgt die Kopplung mit einem Metallkomplex so wird bevorzugt Dimethylsulfoxid als Lösungsmittel verwendet. Es hat sich hierbei als vorteilhaft erwiesen, Salze wie z. B. Lithiumchlorid, Natriumbromid, Lithiumbromid und Lithiumjodid als löslichkeitsverbessernde Zusätze zu verwenden.

Wird die Kopplung mit einer in situ aktivierten Carboxylgruppe durchgeführt, so können dem Fachmann bekannte Kupplungs-Reagenzien wie z. B. DCCI, EEDQ, Staab-Reagenz, BOP, PyBOP, TBTU, TDBTU, HBTU (s. z.B. Fournic-Zaluski et al., J. Med. Chem. 1996, 39, 2596; Houben-Weyl, Band XV/2, Teil II, 1974; Y.M. Angell et al, Tetrahedron Letters 1994, 35, 5981; L.A. Carpino et at, J. Chem. Soc. Commun. 1994, 201; H-O. Kim et al, Tetrahedron Letters 1995, 36, 6013; D. Papaioannou et al, Tetrahedron Letters, 1995, 36, 5187, G. Stemple et al, Bioorg. Med. Letters 1996, 6, 55; verwendet werden.

Die als Ausgangssubstanzen verwendeten aktivierten Komplexe bzw. Komplexbildner V', VI', VII', VIII' und VIII' a 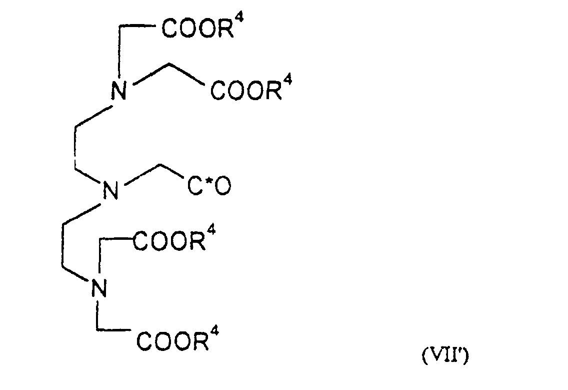 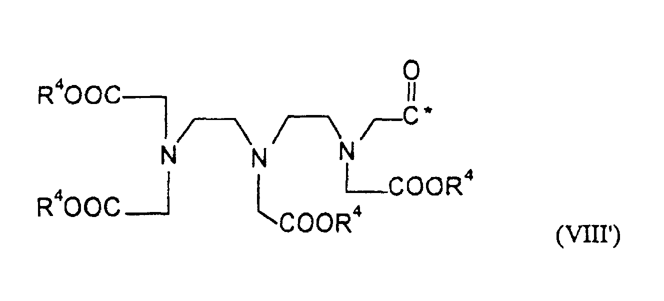 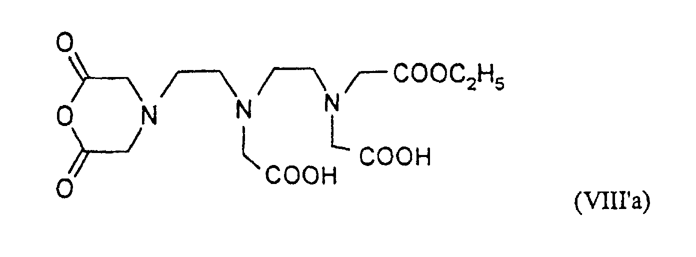 sind literaturbekannt oder analog literaturbekannten Methoden erhältlich:
VIII' und VIII'a s. z.B. EP 263 059,
VII' s. z.B. DE 19507822, DE 19580858, DE 19507819,
V' und VI' s. z.B. US-5,053,503, WO 96/02669, WO 96/01655, EP 0430863, EP 255471, US-5,277,895, EP 0232751, US-4,885,363.

Die Ausgangssubstanzen der allgemeinen Formel I" werden aus Verbindungen der allgemeinen Formel I A 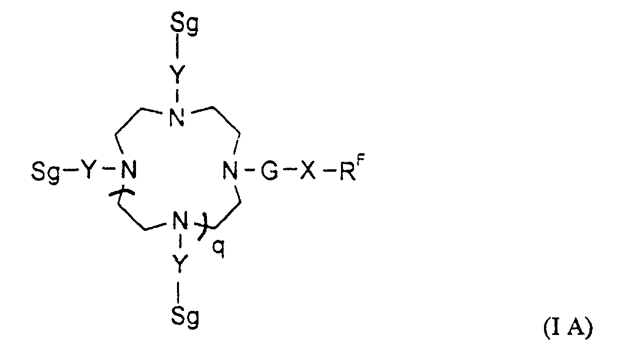 worin Sg für eine Aminoschutzgruppe steht, erhalten.

Als Aminoschutzgruppen seien die dem Fachmann geläufigen Benzyloxycarbonyl-, tertiär-Butoxycarbonyl-, Trifluoracetyl-, Fluorenylmethoxycarbonyl-, Benzyl-, Formyl-, 4-Methoxybenzyl-, 2,2,2-Trichlorethoxycarbonyl-, Phthaloyl-, 1,2-Oxazolin-, Tosyl-, Dithiasuccinoyl-, Allyloxycabonyl-, Sulfat-, Pent-4-encarbonyl-, 2-Chloracetoxymethyl (bzw.-ethyl) benzoyl-, Tetrachlorphthaloyl-, Alkyloxycarbonylgruppen genannt [Th. W. Greene, P.G.M. Wuts, Protective Groups in Organic Syntheses, 2nd ed, John Wiley and Sons (1991), S. 309- 385; E. Meinjohanns et al, J. Chem. Soc. Pekin Trans 1, 1995, 405; U. Ellensik et al, Carbohydrate Research 280, 1996, 251; R. Madsen et al, J. Org. Chem. 60, 1995, 7920; R.R. Schmidt, Tetrahedron Letters 1995, 5343].

Die Abspaltung der Schutzgruppen erfolgt nach den dem Fachmann bekannten Verfahren (s. z.B. E. Wünsch, Methoden der Org. Chemie, Houben-Weyl, Bd XV/1, 4. Auflage 1974, S. 315), beispielsweise durch Hydrolyse, Hydrogenolyse, alkalische Verseifung der Ester mit Alkali in wäßrig-alkoholischer Lösung bei Temperaturen von 0 °C bis 50 °C, saure Verseifung mit Mineralsäuren oder im Fall von Boc-Gruppen mit Hilfe von Trifluoressigsäure.

Die Herstellung der geschützten Makrocyclen der allgemeinen Formel I A kann durch Acylierung von Verbindungen der allgemeinen Formel I B 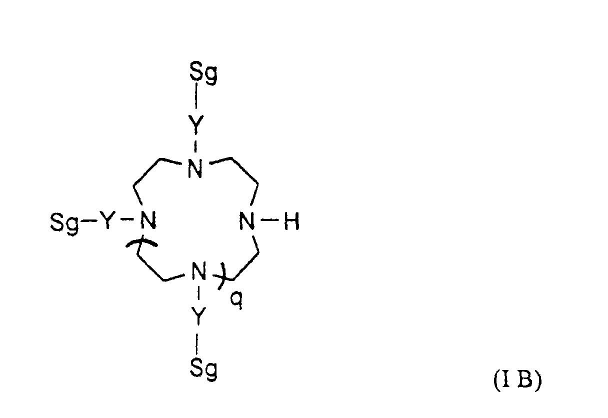 mit einem den gewünschten Substituenten einführenden Substrat der allgemeinen Formel IC

Nu-G-X-R^{F} (IC)

worin Nu für ein Nucelofug steht, erfolgen.

Als Nucleofug dienen vorteilhafterweise die Reste:
F, Cl, Br, I, ―OTs , ―OMs , OH,

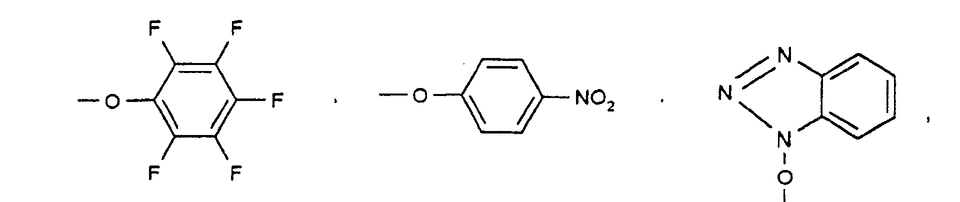

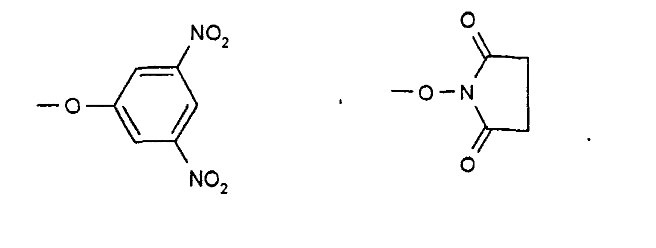

Die Umsetzung wird im Gemisch von Wasser und organischen Lösungsmitteln wie Isopropanol, Ethanol, Methanol, Butanol, Dioxan, Tetrahydrofuran, Dimethylformamid, Dimethylacetamid, Formamid oder Dichlormethan durchgeführt. Bevorzugt sind ternäre Gemische aus Wasser, Isopropanol und Dichlormethan.

Die Umsetzung wird in einem Temperaturintervall zwischen -10 °C - 100 °C, vorzugsweise zwischen 0 °C - 30 °C durchgeführt.

Als Säurefänger dienen anorganische und organische Basen wie Triethylamin, Pyridin, N-Methylmorpholin, Diisopropylethylamin, Dimethylaminopyridin, Alkali- und Erdalkalihydroxyde, ihre Carbonate oder Hydrogencarbonate wie Lithiumhydroxid, Natriumhydroxid, Kaliumhydroxid, Natriumcarbonat, Natriumhydrogencarbonat, Kaliumhydrogencarbonat.

Steht Nu für eine OH-Gruppe, so können dem Fachmann bekannte Kupplungs-Reagenzien wie z.B. DCCI, EEDQ, Staab-Reagenz, BOP, PyBOP, TBTU, TDBTU, HBTU (s. z.B. Fournic-Zaluski et al., J. Med. Chem. 1996, 39, 2596; Houben-Weyl, Band XV/2, Teil II, 1974; Y.M. Angell et al, Tetrahedron Letters 1994, 35, 5981; L.A. Carpino et at, J. Chem. Soc. Commun. 1994, 201; H-O. Kim et al, Tetrahedron Letters 1995, 36, 6013; D. Papaioannou et al, Tetrahedron Letters, 1995, 36, 5187, G. Stemple et al, Bioorg. Med. Letters 1996, 6, 55; verwendet werden.

Die Herstellung der als Edukte für die oben genannte Acylierungsreaktion benötigten Verbindungen IB ist in der DE-OS 19549286 beschrieben.

Die Synthese der Verbindungen der allgemeinen Formel IC erfolgt nach den dem Fachmann bekannten Methoden und ist in den Beispielen hinreichend beschrieben.

Eine weitere Möglichkeit der Synthese von Makrocyclen der allgemeinen Formel IA besteht darin, daß man Verbindungen der allgemeinen Formel IE 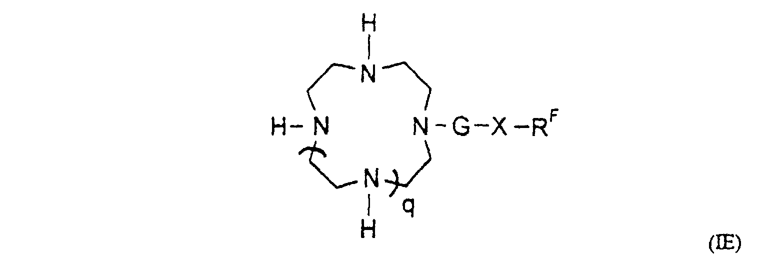 mit literaturbekannten Verbindungen der allgemeinen Formel ID

Nu-Y-Sg (ID)

in einer dem Fachmann bekannten Weise, wie bei der Reaktion von IB mit IC beschrieben, umsetzt.

Die oben genannte Verbindung der allgemeinen Formel IE erhält man durch Abspaltung von Aminoschutzgruppen aus Verbindungen der allgemeinen Formel IF, 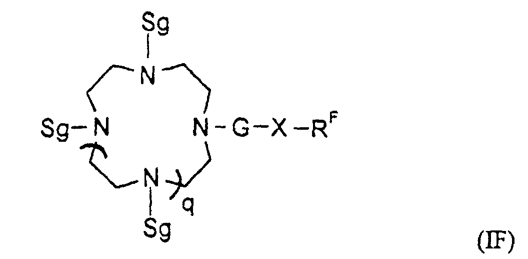 die zugänglich sind aus den literaturbekannten Verbindungen der allgemeinen Formel IG.

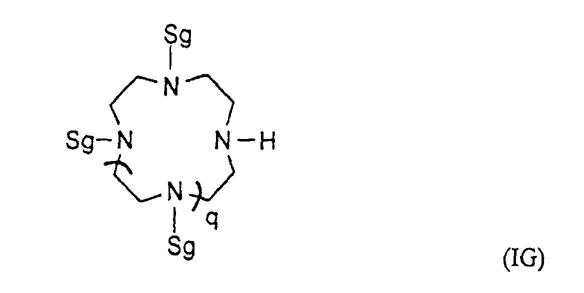

Diese sind durch Acylierung mit Substraten der allgemeinen Formel IC analog der oben beschriebenen Umsetzung von IB mit IC erhältlich.

Die Herstellung der erfindungsgemäßen pharmazeutischen Mittel erfolgt in an sich bekannter Weise, indem man die erfindungsgemäßen Komplexverbindungen-gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Oligomer-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methylcellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Die erfindungsgemäßen pharmazeutischen Mittel enthalten vorzugsweise 0,1 µMol - 1 Mol/l des Komplexes und werden in der Regel in Mengen von 0,0001 - 5 mMol/kg dosiert. Sie sind zur enteralen und parenteralen Applikation bestimmt. Die erfindungsgemäßen Komplexverbindungen kommen zur Anwendung
1. für die NMR- und Röntgen-Diagnostik in Form ihrer Komplexe mit den Ionen der Elemente mit den Ordnungszahlen 21 - 29, 39, 42, 44 und 57 - 83;
2. für die Radiodiagnostik und Radiotherapie in Form ihrer Komplexe mit den Radioisotopen der Elemente mit den Ordnungszahlen 27, 29, 31, 32, 37 - 39, 43, 49, 62, 64, 70, 75 und 77.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen mittel für die Anwendung als NMR-Diagnostika in Mengen von 0,0001 - 5 mMol/kg, vorzugsweise 0,005 - 0,5 mMol/kg, dosiert. Details der Anwendung werden zum Beispiel in H.-J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als

Radiodiagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomo-draphie, die positronenemittierende Isotope wie z.B. ⁴³Sc, ⁴⁴Sc, ⁵²Fe, ⁵⁵Co und ⁶⁸Ga verwendet (Heiss, W.D.; Phelps, M.E.; Positron Emission Tomography of Brain, Springer Verlag Berlin, Heidelberg, New York 1983).

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, daß sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung fiir gesunde Gewebe erheblich reduziert und damit belastende Nebenwirkungen fiir die Patienten vermieden werden. Die erfindungsgemäßen Verbindungen eignen sich deshalb auch als ^{.}radiosensibilisierende Substanz bei Strahlentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronenemittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen therapeutischen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen therapeutischen Mittel werden parenteral, vorzugsweise i.v., appliziert.

Details der Anwendung von Radiotherapeunka werden z.B. in R.W. Kozak et al. TIBTEC, Oktober 1986, 262, diskutiert.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemischpharmakologischen Untersuchungen erkennen lassen. Besonders wertvoll sind sie wegen der günstigen Absorptionseisenschaften in Bereichen höherer Röhrenspannungen für digitale Substraktionstechniken.

Im allgemeinen werden die erfindungsgemäßen Mittel für die Anwendung als Röntgenkontrastmittel in Analogie zu zum Beispiel Meglumin-Diatrizoat in Mengen von 0,1 - 5 mMol/kg, vorzugsweise 0,25 - 1 mMol/kg, dosiert.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Insgesamt ist es gelungen, neue Verbindungen zu synthetisieren, die neue Möglichkeiten in der diagnostischen und therapeutischen Medizin erschließen.

Die nachfolgenden Beispiele dienen zur näheren Erläuterung des Erfindungsgegenstands:

Dabei werden folgende Abkürzungen benutzt:
a) DTPA-monoanhydrid-ethylester: 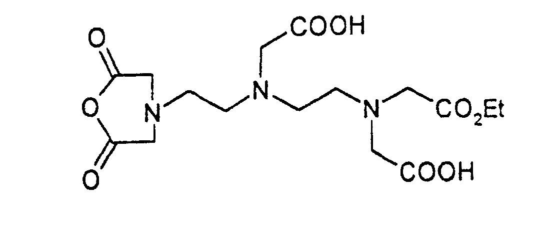
b) sym-DTPA-tetra-t.butylester:

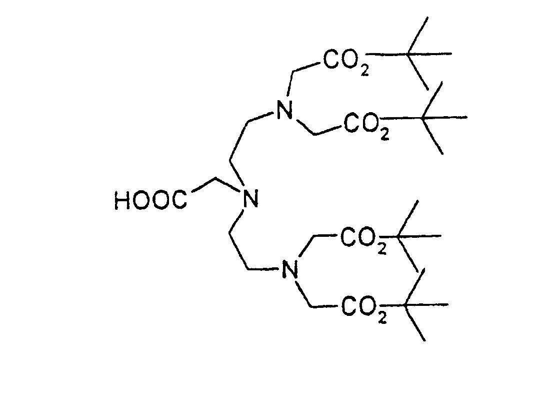

### Beispiel 1

a) 2H,2H,4H,4H,5H,5H-3-oxa-perfluor-tridecansäure-t.-butylester
Zu einer Mischung aus 10 g (21,55 mmol) 1H, 1H, 2H, 2H-Perfluordecan-1-ol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäuretert.-butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/ Dichlormethan/Aceton= 20:10:1).
Ausbeute: 9,72 g (78 % d. Th.) eines farblosen viskosen Öls

| Elementaranalyse: | | | |
|---|---|---|---|
| ber. | C 33,23 | H 2,61 | F 55,85 |
| gef. | C 33,09 | H 2,78 | F 55,71 |

b) 2H,2H,4H,4H,5H,5H-3-oxa-perfluor-tridecansäure
9,0 g (15,56 mmol) der Titelverbindung aus Beispiel 1a) werden in 180 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 7,80 g (96 % d. Th.) eines farblosen Feststoffes

| Elementaranalyse: | | | |
|---|---|---|---|
| ber. | C 27,60 | H 1,35 | F 61,85 |
| gef. | C 27,48 | H 1,49 | F 61,66 |

c) 1,4,7-Tris[N-(2-benzyloxycarbonylamino)-acetyl]-1,4,7,10-tetraazacyclododecan
Zu 10 g (58 mmol) 1,4,7,10-Tetraazacyclododecan (= Cyclen) in 400 ml Toluol gibt man 53,33 g (174 mmol) N-(Benzyloxycarbonyl)-glycin-N-hydroxysuccinimidester und 17,60 g (174 mmol) Triethylamin und erhitzt 12 Stunden unter Rückfluß. Man dampft im Vakuum zur Trockne ein, nimmt den Rückstand in 400 ml Dichlormethan auf und wäscht die organische Phase 2 mal mit 5 %iger aqu. Natriumcarbonat-Lösung Man trocknet die organische Phase über Magnesiumsulfat und engt im Vakuum zur Trockne ein. Der Rückstand wird an Kieselgel chromatographiert (Laufmittel: Essigsäureethylester/Ethanol= 20:1).

| Elementaranalyse: | | | |
|---|---|---|---|
| ber. | C 61,20 | H 6,35 | N 13,15 |
| gef. | C 61,03 | H 6,48 | N 13,02 |

d) 1,4,7-Tris[N-(2-benzyloxycarbonylamino)-acetyl-]-10-(N-2H,2H,4H,4H,5H,5H-3-oxa-perfluor-tridecanoyl)-1,4,7,10-tetraazacyclododecan
20 g (26,82 mmol) der Titelverbindung aus Beispiel 1c) und 28.0 g (53,63 mmol) der Titelverbindung aus Beispiel 1b) werden in 200 ml Dimethylformamid gelöst und bei 0°C 13,26 g (53,63 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 28,16 (84 % d. Th.) eines farblosen, zähen Öls

| Elementaranalyse: | | | | |
|---|---|---|---|---|
| ber. | C 48,05 | H 4,19 | N 7,84 | F 25,84 |
| gef. | C 47,91 | H 4,38 | N 7,71 | F 25,67 |

e) 1,4,7-Tris-[N-(2-amino)-acetyl]-10-(N-2H,2H,4H,4H,5H,5H-3-oxa-perfluor-tridecanoyl)-1,4,7,10-tetraazacyclododecan, Trihydrobromid
20 g (16 mmol) der Titelverbindung aus Beispiel 1d) werden in 100 ml Essigsäure gelöst und zu einer 60°C heißen Lösung aus 200 ml Bromwasserstoff in Eisessig (33 %ig) getropft. Man rührt 1 Stunde bei 60°C. Es wird auf 0°C abgekühlt und unter Rühren 1500 ml Diethylether langsam zugetropft. Man filtriert den ausgefallenen Niederschlag ab, wäscht 2 mal mit 200 ml Ether nach und trocknet im Vakuumofen (60°C).
Ausbeute: 16,57 g (95 % d. Th.) cremefarbener, kristalliner Feststoff

| Elementaranalyse: | | | | | |
|---|---|---|---|---|---|
| ber. | C 28,64 | H 3,42 | N 8,99 | F 29,62 | Br 21,99 |
| gef. | C 28,51 | H 3,60 | N 8,72 | F 29,41 | Br 21,65 |

f) Gd-Komplex des 1,4,7-Tris(N-carboxylatomethyl)-10-(N-1-methyl-2-oxo-3-aza-4-carboxy-butyl)-1,4,7,10-tetraazacyclododecan
77 g (103,1 mmol) 10-[4-carboxy-1-methyl-2-oxo-3-azabutyl]-1,4,7-tris(tert.butoxycarbonyl)-1,4,7,10-tetraazacyclododecan werden in 500 ml Trifluoressigsäure gelöst und 3 Stunden bei Raumtemperatur gerührt. Man dampft zur Trockne ein, nimmt den Rückstand in 300 ml Wasser auf und gibt die Lösung auf eine Säule, gefüllt mit Reillex® 425 PVP. Man eluiert mit Wasser. Die produkthaltigen Fraktionen werden vereinigt und zur Trockne eingedampft, der Rückstand wird aus Methanol/Aceton umkristallisiert. Ausbeute: 44,04 g (84 % d. Th.) eines farblosen, hygroskopischen Feststoffes Wassergehalt: 6,5 %

| Elementaranalyse (berechnet auf wasserfreie Substanz): | | | |
|---|---|---|---|
| ber. | C 47,99 | H 6,99 | N 14,73 |
| gef. | C 47,83 | H 7,12 | N 14,55 |

Zu 40 g (84,12 mmol) der oben erhaltenen Tetrasäure, gelöst in 400 ml Wasser, gibt man 15,27 g (42,06 mmol) Gadoliniumoxid und erwärmt 3 Stunden auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden). Ausbeute: 50,53 g (93 % d. Th.) eines farblosen, kristallinen Pulvers
Wassergehalt: 2,5 %

| Elementaranalyse (berechnet auf wasserfreie Substanz): | | | | |
|---|---|---|---|---|
| ber. | C 36,24 | H 4,80 | N 11,12 | Gd 24,97 |
| gef. | C 36,35 | H 4,95 | N 10,98 | Gd 24,80 |

g) 1,4,7-Tris {1,4,7-tris(N-carboxylatomethyl)-10-(N-1-methyl-3,6-diaza-2,5,8-trioxooctan-1,8-diyl)-1,4,7,10-tetraazacyclododecan, Gd-Komplex}-10-(N-2H,2H,4H,4H,5H,5H-3-oxa-perfluor-tridecanoyl)-1,4,7,10-tetraazacyclododecan
15,74 g (25 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,57 g Natriumbromid (25 mmol) und 5,75 g (50 mmol) N-Hydroxysuccinimid werden in 200 ml Dimethylsulfoxid bei 50°C gelöst. Nach Abkühlen auf Raumtemperatur werden 10,32 g (50 mmol) N,N'-Dicyclohexylcarbodiimid zugesetzt und 60 Minuten voraktiviert. Zu der so hergestellten N-Hydroxysuccinimidester-Lösung gibt man eine Lösung von 3 g (2,75 mmol) der in Beispiel 1e) beschriebenen Titelverbindung und 5,06 g (50 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 6,42 g (87 % d. Th.)
H₂O-Gehalt: 10,1 %

| Elementaranalyse (berechnet auf wasserfreie Substanz): | | | | | |
|---|---|---|---|---|---|
| ber. | C 37,13 | H 4,51 | N 11,48 | F 12,03 | Gd 17,57 |
| gef. | C 37,01 | H 4,71 | N 11,23 | F 11,84 | Gd 17,38 |

### Beispiel 2

1,4,7-Tris{1,4,7-tris(N-carboxylatomethyl)-10-(N-1-methyl-3,6-diaza-2,5,8-trioxo-octan-1,8-diyl)-1,4,7,10-tetraazacyclododecan, Dy-Komplex}-10-(N-2H,2H,4H,4H,5H,5H-3-oxa-perfluor-tridecanoyl)-1,4,7,10-tetraazacyclododecan

10 g (3,72 mmol) der Titelverbindung aus Beispiel 1g) wird in 200 ml Wasser gelöst und 1,52 g (16,86 mmol) Oxalsäure zugegeben. Man rührt 8 Stunden bei 60°C. Es wird auf 0°C abgekühlt und vom ausgefallenen Gadolinium-Oxalat abfiltriert. Zum Filtrat gibt man 2,08 g (5,58 mmol) Dysprosiumoxid und erwärmt 5 Stunden auf 90°C. Die Lösung wird zur Trockne eingedampft und der Rückstand an RP-18 (Laufmittel: Gradient aus Acetonitril/Wasser) chromatographiert.
Ausbeute: 8,74 g (87 % d. Th.) eines glasigen Feststoffs
Wassergehalt: 9,3 %

| Elementaranalyse (berechnet auf wasserfreie Substanz): | | | | | |
|---|---|---|---|---|---|
| ber. | C 36,92 | H 4,48 | N 11,41 | F 11,96 | Dy 18,05 |
| gef. | C 36,81 | H 4,62 | N 11,35 | F 11,81 | Dy 17,84 |

### Beispiel 3

### 1,4,7-Tris{1,4,7-tris(N-carboxylatomethyl)-10-(N-1-methyl-3,6-diaza-2,5,8-trioxo-octan-1,8-diyl)-1,4,7,10-tetraazacyclododecan, Fe-Komplex}-10-(N-2H,2H,4H,4H,5H,5H-3-oxa-perfluortridecanoyl)-1,4,7,10-tetraazacyclododecan

10 g (3,72 mmol) der Titelverbindung aus Beispiel 1g) wird in 200 ml Wasser gelöst und 1,52 mg (16,86 mmol) Oxalsäure zugegeben. Man rührt 8 Stunden bei 80°C. Es wird auf 0°C abgekühlt und vom ausgefallenen Gadolinium-Oxalat abfiltriert. Zum Filtrat gibt man 3,93 g (11,16 mmol) Eisen(III)-acetylacetonat und erwärmt 5 Stunden auf 90°C. Die Lösung wird zur Trockne eingedampft und der Rückstand an RP-18 (Laufmittel: Gradient aus Acetonitril/Wasser) chromatographiert.
Ausbeute. 7,17 g (81 % d. Th.) eines glasigen Feststoffs
Wassergehalt: 7,5 %

| Elementaranalyse (berechnet auf wasserfreie Substanz): | | | | | |
|---|---|---|---|---|---|
| ber. | C 41,88 | H 5,08 | N 12,94 | F 13,57 | Fe 7,08 |
| gef. | C 41,65 | H 5,21 | N 12,75 | F 13,41 | Fe 6,93 |

### Beispiel 4

a) 1,4,7-Tris-(N-Benzyloxycarbonyl)-10-(N-2H,2H,4H,4H,5H,5H-3-oxaperfluortridecanoyl)-1,4,7,10-tetraazacyclododecan
30 g (52,20 mmol) 1,4,7-Tris-(benzyloxycarbonyl)-1,4,7,10-tetraazacyclododecan (≅ Tri-Z-Cyclen) und 54,51 g (104,4 mmol) werden in 300 ml Dimethylformamid gelöst und bei 0°C 25,82 g (104,4 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/2-Propanol= 20:1).
Ausbeute: 44,5 (79 % d. Th.) eines farblosen, zähen Öls

| Elementaranalyse: | | | | |
|---|---|---|---|---|
| ber. | C 48,99 | H 4,02 | N 5,19 | F 29,94 |
| gef. | C 48,75 | H 4,21 | N 5,03 | F 29,75 |

b) 1-(N-H,2H,4H,4H,5H,5H-3-oxa-perfluortridecanoyl)-1,4,7,10-tetraazacyclododecan
30 g (27,81 mmol) der Titelverbindung aus Beispiel 4a) werden in 500 ml Methanol gelöst und 5 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft.
Ausbeute: 18,81 g (quantitativ) eines glasigen, farblosen Feststoffes

| Elementaranalyse: | | | | |
|---|---|---|---|---|
| ber. | C 35,51 | H 3,73 | N 8,28 | F 47,75 |
| gef. | C 35,40 | H 3,91 | N 8,14 | F 47,53 |

c) 1,4,7-Tris[1,4,7-tris(N-carboxylatomethyl)-10-(N-1-methyl-3-aza-2,5-dioxo-pentan-1,5-diyl)-1,4,7,10-tetraazacyclododecan, Gd-Komplex]-10-(2H,3H,4H,4H,5H,5H-3-oxa-perfluor-tridecanoyl)-1,4,7,10-tetraazacyclododecan
16,75 g (25,61 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure und 2,78 Natriumbromid (27 mmol) werden in 200 ml Dimethylsulfoxid bei 50°C gelöst. Nach Abkühlen auf Raumtemperatur werden 12,37 g (50 mmol) 2-Ethoxy-1-ethoxycarbonyl-1,2-dihydrochinolin und 3 g (2,75 mmol) der in Beispiel 4b) beschriebenen Titelverbindung zugegeben und über Nacht bei Raumtemperatur gerührt. Die erhaltene Suspension wird anschließend mit ausreichend Aceton bis zur vollständigen Fällung versetzt, der Niederschlag abgesaugt, getrocknet, in Wasser aufgenommen, und über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 9,70 g (87 % d. Th.)
H₂O-Gehalt: 8,9 %

| Elementaranalyse (berechnet auf wasserfreie Substanz): | | | | | |
|---|---|---|---|---|---|
| ber. | C 36,82 | H 4,37 | N 10,60 | Gd 18,78 | F 12,86 |
| gef. | C 36,70 | H 4,18 | N 10,45 | Gd 18,61 | F 12,71 |

### Beispiel 5

a) 2H,2H,4H,4H,5H,5H-3-oxa-perfluor-pentadecansäure-t.-butylester
Zu einer Mischung aus 12,16 g (21,55 mmol) 1H, 1H, 2H, 2H-Perfluordodecan-1-ol und 0,73 g (2,15 mmol) Tetrabutylammoniumhydrogensulfat in 100 ml 60 %iger Kalilauge/50 ml Toluol tropft man unter starkem Rühren bei 0°C 10,51 g (53,9 mmol) Bromessigsäuretert.-butylester zu. Man rührt 1 Stunde bei 0°C. Es werden 200 ml Toluol zugegeben, die wässrige Phase abgetrennt und 2 mal mit je 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/ Dichlormethan/Aceton= 20:10:1).
Ausbeute: 11,42 g (81 % d. Th.) eines farblosen viskosen Öls

| Elementaranalyse: | | | |
|---|---|---|---|
| ber. | C 29,37 | H 2,31 | F 60,98 |
| gef. | C 29,28 | H 2,42 | F 60,81 |

b) 2H,2H,4H,4H,5H,5H-3-oxa-perfluorpentadecansäure
9,0 g (15,56 mmol) der Titelverbindung aus Beispiel 5a) werden in 180 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 9,29 g (96 % d. Th.) eines farblosen Feststoffes

| Elementaranalyse: | | | |
|---|---|---|---|
| ber. | C 27,03 | H 1,13 | F 64,12 |
| gef | C 26,91 | H 1,24 | F 64,01 |

c) 1,4,7-Tris[N-(2-benzyloxycarbonylamino)-acetyl-]-10-(N-2H,2H,4H,4H,5H,5H-3-oxa-perfluorpentadecanoyl)-1,4,7,10-tetraazacyclododecan
20 g (26,82 mmol) der Titelverbindung aus Beispiel 1c) und 33,35 g (53,6 mmol) der Titelverbindung aus Beispiel 5b) werden in 200 ml Dimethylformamid gelöst und bei 0°C 13,26 g (53,6 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 29,33 (81 % d. Th.) eines farblosen, zähen Öls

| Elementaranalyse: | | | | |
|---|---|---|---|---|
| ber. | C 46,26 | H 3,88 | N 7,26 | F 29,55 |
| gef. | C 46,13 | H 3,68 | N 7,31 | F 29,44 |

d) 1,4,7-Tris-[N-(2-amino)-acetyl]-10-(N-2H,2H,4H,4H,5H,5H-3-oxa-perfluorpentadecanoyl)-1,4,7,10-tetraazacyclododecan, Trihydrobromid
15 g (11,11 mmol) der Titelverbindung aus Beispiel 5c) werden in 100 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1500 ml Diethylether langsam zugetropft. Man filtriert den ausgefallenen Niederschlag ab, wäscht 2 mal mit 200 ml Ether nach und trocknet im Vakuumofen (60°C).
Ausbeute: 12,7 g (96 % d. Th.) cremefarbener, kristalliner Feststoff

| Elementaranalyse: | | | | | |
|---|---|---|---|---|---|
| ber. | C 28,25 | H 3,13 | N 8,24 | F 33,52 | Br 20,14 |
| gef. | C 28,14 | H 3,24 | N 8,13 | F 33,38 | Br 20,01 |

e) 1,4,7-Tris{1,4,7-tris(N-carboxylatomethyl)-10-(N-1-methyl-3,6-diaza-2,5,8-trioxooctan-1,8-diyl)-1,4,7,10-tetraazacyclododecan, Gd-Komplex}-10-(N-2H,2H,4H,4H,5H,5H-3-oxa-perfluor-pentadecanoyl)-1,4,7,10-tetraazacyclododecan
15,74 g (25 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,57 g Natriumbromid (25 mmol) und 5,75 g (50 mmol) N-Hydroxysuccinimid werden in 200 ml Dimethylsulfoxid bei 50°C gelöst. Nach Abkühlen auf Raumtemperatur werden 10,32 g (50 mmol) N,N'-Dicyclohexylcarbodiimid zugesetzt und 60 Minuten voraktiviert. Zu der so hergestellten N-Hydroxysuccinimidester-Lösung gibt man eine Lösung von 3,27 g (2,75 mmol) der in Beispiel 5d) beschriebenen Titelverbindung und 5,06 g (50 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 6,74 g (88 % d. Th.)
H₂O-Gehalt: 6,8 %

| Elementaranalyse (berechnet auf wasserfreie Substanz): | | | | | |
|---|---|---|---|---|---|
| ber. | C 36,66 | H 4,34 | N 11,07 | F 14,33 | Gd 16,94 |
| gef. | C 36,49 | H 4,42 | N 11,01 | F 14,21 | Gd 16,81 |

### Beispiel 6

a) 1,4,7-Tris(N-3,6,9-carboxymethyl-12-aza-11-oxo-3,6,9,12-tetraaza-tetradecan-1,14-dicarbonsäure-monoamid)-10-(N-2H,2H,4H,4H,5H,5H-3-oxa-perfluor-tridecanoyl)-1,4,7,10-tetraazacyclododecan
Zu 5 g (4,59 mmol) der Titelverbindung aus Beispiel 1e) und 50 g Pyridin in 50 ml Dimethylformamid gibt man 10,43 g (27,5 mmol) DTPA-monoanhydrid-ethylester und 1 g (8,19 mmol) 4-(Dimethylamino)-pyridin. Man rührt 24 Stunden bei 50°C. Es wird im Vakuum zur Trockne eingedampft, der Rückstand in 200 ml Wasser aufgenommen und mit 2 N Natronlauge auf pH 13 gebracht. Man rührt 6 Stunden bei Raumtemperatur. Man stellt mit 10 %iger aqu. Salzsäure auf pH 2 und dialysiert die Lösung über eine AMICON ® YM-1 Ultrafiltrationsmembram (cut off 1000 Da) um von niedermolekularen Bestandteilen zu reinigen. Das Retentat wird anschließend gefriergetrocknet.
Ausbeute: 8,24 g (91 % d. Th.)
H₂O-Gehalt: 7,5 %

| Elementaranalyse (berechnet auf wasserfreie Substanz): | | | | |
|---|---|---|---|---|
| ber. | C 41,38 | H 4,95 | N 11,36 | F 16,36 |
| gef. | C 41,28 | H 5,07 | N 11,29 | F 16,27 |

b) 1,4,7-Tris(N-3,6,9-carboxymethyl-12-aza-11-oxo-3,6,9,12-tetraaza-tetradecan-1,14-dicarbonsäure-14-monoamid, Gd-Komplex, Mononatriumsalz)-10-(N-2H,2H,4H,4H,5H,5H-3-oxa-perfluor-tridecanoyl)-1,4,7,10-tetraazacyclododecan
Zu 5 g (2,53 mmol) der Titelverbindung aus Beispiel 6b) gelöst in 200 ml Wasser gibt man 2,54 g (7,59 mmol) Gadoliniumacetat zu. Anschließend rührt man 1 Stunde bei 60°C. Man läßt auf Raumtemperatur abkühlen, stellt mit 2N aqu. Natronlauge auf pH 7,2 und dialysiert die Lösung in einer Ultrafiltrationszelle AMICON® YM-1 (cut off 1000 DA, 6 Durchläufe). Der Inhalt der Ultrafiltrationszelle wird gefriergetrocknet.
Ausbeute: 6,08 g (96 % d. Th.)
Wassergehalt: 9,1 %

| Elementaranalyse (berechnet auf wasserfreie Substanz): | | | | | | |
|---|---|---|---|---|---|---|
| ber. | C 32,64 | H 3,42 | N 8,96 | F 12,91 | Gd 18,85 | Na 2,76 |
| gef. | C 32,47 | H 3,57 | N 8,90 | F 12,84 | Gd 18,67 | Na 2,55 |

### Beispiel 7

a) 1,4,7-Tris{(N-3,6,9-carboxymethyl-12-aza-11-oxo-3,6,9,12-tetraaza-tetradecan-1,14-dicarbonsäure-14-monoamid)}-10-(N-2H,2H,4H,4H,5H,5H-3-oxa-perfluor-pentadecanoyl)-1,4,7,10-tetraazacyclododecan
Zu 5,46 g (4,59 mmol) der Titelverbindung aus Beispiel 5d) und 50 g Pyridin in 50 ml Dimethylformamid gibt man 10,43 g (27,5 mmol) DTPA-monoanhydrid-ethylester und 1 g (8,19 mmol) 4-(Dimethylamino)-pyridin. Man rührt 24 Stunden bei 50°C. Es wird im Vakuum zur Trockne eingedampft, der Rückstand in 200 ml Wasser aufgenommen und mit 2 N Natronlauge auf pH 13 gebracht. Man rührt 6 Stunden bei Raumtemperatur. Man stellt mit 10 %iger aqu. Salzsäure auf pH 2 und dialysiert die Lösung über eine AMICON ® YM-1 Ultrafiltrationsmembram (cut off 1000 Da) um von niedermolekularen Bestandteilen zu reinigen. Das Retentat wird anschließend gefriergetrocknet.
Ausbeute: 8,85 g (93 % d. Th.)
H₂O-Gehalt: 6,3 %

| Elementaranalyse (berechnet auf wasserfreie Substanz): | | | | |
|---|---|---|---|---|
| ber. | C 40,55 | H 4,71 | N 10,81 | F 19,24 |
| gef. | C 40,41 | H 4,85 | N 10,74 | F 19,11 |

b) 1,4,7-Tris[(N-3,6,9-carboxymethyl-12-aza-11-oxo-3,6,9,12-tetraaza-tetradecan-1,14-dicarbonsäure-14-monoamid), Gd-Komplex, Mononatriumsalz]-10-(N-2H,2H,4H,4H,5H,5H-3-oxa-perfluor-pentadecanoyl)-1,4,7,10-tetraazacyclododecan
Zu 5,25 g (2,53 mmol) der Titelverbindung aus Beispiel 7a) gelöst in 200 ml Wasser gibt man 2,54 g (7,59 mmol) Gadoliniumacetat zu. Anschließend rührt man 1 Stunde bei 60°C. Man läßt auf Raumtemperatur abkühlen, stellt mit 2N aqu. Natronlauge auf pH 7,2 und dialysiert die Lösung in einer Ultrafiltrationszelle AMICON® YM-1 (cut off 1000 DA, 6 Durchläufe). Der Inhalt der Ultrafiltrationszelle wird gefriergetrocknet.
Ausbeute: 6,42 g (97 % d. Th.)
Wassergehalt: 10,1 %

| Elementaranalyse (berechnet auf wasserfreie Substanz): | | | | | | |
|---|---|---|---|---|---|---|
| ber. | C 32,16 | H 3,74 | N 8,57 | F 15,26 | Gd 18.04 | Na 2,64 |
| gef. | C 32,01 | H 3,93 | N 8,61 | F 15,10 | Gd 17,91 | Na 2,43 |

### Beispiel 8

a) 1,4,7-Tris{3,9-bis(N-t.butyloxycarbonylmethyl)-6-[N-3-aza-2,5-dioxo-pentan-1,5-diyl]-3,6,9-triazaundecandisäure-di-tbutylester}-10-(N-2H,2H,4H,5H,5H,5H-3-oxaperfluor-tridecanoyl)-1,4,7,10-tetraazacyclododecan
Zu 17 g (27,54 mmol) sym-DTPA-tetra-tert.butylester gelöst in 300 ml Dimethylformamid gibt man bei 0°C 6,34 g (55,1 mmol) N-Hydroxysuccinimid und 11,37 g (55,1 mmol) N,N'-Dicyclohexylcarbodiimid und rührt eine Stunde bei dieser Temperatur. Anschließend rührt man 3 Stunden bei Raumtemperatur. Zu dieser Lösung gibt man 5 g (4,59 mmol) der Titelverbindung aus Beispiel 1e) und 11,13 g (110 mmol) Triethylamin. Man rührt 24 Stunden bei Raumtemperatur. Die Lösung wird zur Trockne eingedampft und der Rückstand in 500 ml Dichlormethan aufgenommen. Man filtriert vom Dicyclohexylhamstoff ab und wäscht das Filtrat 2 mal mit 250 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 9,84 g (81 % d. Th.) eines farblosen, zähen Öls

| Elementaranalyse: | | | | |
|---|---|---|---|---|
| ber. | C 52,64 | H 7,35 | N 8,47 | F 12,20 |
| gef. | C 52,51 | H 7,45 | N 8,38 | F 12,07 |

b) 1,4,7-Tris(N-3,3,6-tris(carboxylatomethyl)-6-[N-3-aza-2,5-dioxo-pentan-1,5-diyl]-3,6,9-triazaundecan-1,11-disäure, Gd-Komplex, Mononatriumsalz}-10-2H,2H,4H,4H,5H,5H-3-oxa-perfluor-tridecanoyl)-1,4,7,10-tetraazacyclododecan
9 g (3,4 mmol) der Titelverbindung aus Beispiel 8a) werden in 200 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 1,85 g (5,1 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 3 g Aktivkohle versetzt, 1 Stunde bei Raumtemperatur gerührt und filtriert. Das Filtrat wird in eine Ultrafiltrationszelle gefüllt und dialysiert (AMICON® YM-1 (cut off 1000 DA)). Man dialysiert solange bis das Fluat eine Leitfähigkeit von 10 µS erreicht hat. Dann wird der Inhalt der Ultrafiltrationszelle gefriergetrocknet.
Ausbeute: 7,91 g (93 % d. Th.) eines farblosen, amorphen Pulvers
Wassergehalt: 7,9 %

| Elementaranalyse (berechnet auf wasserfreie Substanz): | | | | | | |
|---|---|---|---|---|---|---|
| ber. | C 32,64 | H 3,42 | N 8,96 | F 12,91 | Gd 18,85 | Na 2,76 |
| gef. | C 32,48 | H 3,55 | N 8,87 | F 12,80 | Gd 18,79 | Na 2,48 |

### Beispiel 9

a) 1,4,7-Tris{N-3,9-bis(N-t.butyloxycarbonylmethyl)-6-[N-3-aza-2,5-dioxo-pentan-1,5-diyl]-3,6,9-triazaundecan-1,11-disäure-di-t.butylester}-10-(N-2H,2H,4H,4H,5H,5H-3-oxa-perfluor-tridecanoyl)-1,4,7,10-tetraazacyclododecan
Zu 15,54 g (25,20 mmol) sym-DTPA-tetra-tert.butylester gelöst in 200 ml Dimethylformamid gibt man bei 0°C 5,8 g (50,4 mmol) N-Hydroxysuccinimid und 10,4 g (50,4 mmol) N,N'- Dicyclohexylcarbodiimid und rührt eine Stunde bei dieser Temperatur. Anschließend rührt man 3 Stunden bei Raumtemperatur. Zu dieser Lösung gibt man 5 g (4,2 mmol) der Titelverbindung aus Beispiel 5d) und 11,13 g (100 mmol) Triethylamin. Man rührt 24 Stunden bei Raumtemperatur. Die Lösung wird zur Trockne eingedampft und der Rückstand in 300 ml Dichlormethan aufgenommen. Man filtriert vom Dicyclohexylharnstoff ab und wäscht das Filtrat 2 mal mit 150 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 9,76 g (87 % d. Th.) eines farblosen, zähen Öls

| Elementaranalyse: | | | | |
|---|---|---|---|---|
| ber. | C 53,06 | H 7,28 | N 8,39 | F 12,09 |
| gef. | C 52,90 | H 7,41 | N 8,27 | F 12,93 |

b) 1,4,7-Tris{N-3,9-bis(N-carboxylatomethyl)-6-[N-3-aza-2,5-dioxo-pentan-1,5-diyl]-3,6,9-triazaundecan-1-carboxylato-11-säure, Gd-Komplex, Natriumsalz}-10-(N-2H,2H,4H,4H,5H,5H-3-oxa-perfluor-pentadecanoyl)-1,4,7,10-tetraazacyclododecan
9 g (3,37 mmol) der Titelverbindung aus Beispiel 9a) werden in 200 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 1,83 g (5,06 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 3 g Aktivkohle versetzt, 1 Stunde bei Raumtemperatur gerührt und filtriert. Das Filtrat wird in eine Ultrafiltrationszelle gefüllt und dialysiert (AMICON® YM-1 (cut off 1000 DA)). Man dialysiert solange bis das Fluat eine Leitfähigkeit von 10 µS erreicht hat. Dann wird der Inhalt der Ultrafiltrationszelle gefriergetrocknet.
Ausbeute: 8,24 g (94 % d. Th.) eines farblosen, amorphen Pulvers
Wassergehalt: 10,1 %

| Elementaranalyse (berechnet auf wasserfreie Substanz): | | | | | | |
|---|---|---|---|---|---|---|
| ber. | C 32,31 | H 3,29 | N 8,61 | F 15,33 | Gd 18,13 | Na 2,65 |
| gef. | C 32,21 | H 3,42 | N 8,59 | F 15,24 | Gd 18,03 | Na 2,24 |

### Beispiel 10

a) 1,4,7-Tris{N-3,9-bis(N-t.butyloxycarbonylmethyl)-6-[N-(2-oxo)-methyl]-3,6,9-triazaundecan-1,11-disäure-di-t.butylester}-10-(N-2H,2H,4H,4H,5H,5H-3-oxaperfluor-tridecanoyl)-1,4,7,10-tetraazacyclododecan
Zu 27,35 g (44,35 mmol) sym-DTPA-tetra-tert.butylester gelöst in 400 ml Dimethylformamid gibt man bei 0°C 10,21 g (88,7 mmol) N-Hydroxysuccinimid und 18,30 g (88,7 mmol) N,N'-Dicyclohexylcarbodiimid und rührt eine Stunde bei dieser Temperatur. Anschließend rührt man 3 Stunden bei Raumtemperatur. Zu dieser Lösung gibt man 5 g (7,39 mmol) der Titelverbindung aus Beispiel 4b) und 17,91 g (177 mmol) Triethylamin. Man rührt 24 Stunden bei Raumtemperatur. Die Lösung wird zur Trockne eingedampft und der Rückstand in 400 ml Dichlormethan aufgenommen. Man filtriert vom Dicyclohexylharnstoff ab und wäscht das Filtrat 2 mal mit 200 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 16,65 g (91 % d. Th.) eines farblosen, zähen Öls

| Elementaranalyse: | | | | |
|---|---|---|---|---|
| ber. | C 53,37 | H 7,49 | N 7,35 | F 13,05 |
| gef. | C 53,28 | H 7,61 | N 7,27 | F 12,96 |

b) 1,4,7-Tris{N-3,3,9-tris(carboxylatomethyl)-6-[N-(2-oxo)-methyl]-3,6,9-triazaundecan-1-Carboxylato-11-säure, Gd-Komplex, Natriumsalz}-10-(N-2H,2H,4H,4H,5H,5H-3-oxa-perfluor-tridecanoyl)-1,4,7,10-tetraazacyclododecan
9 g (3,64 mmol) der Titelverbindung aus Beispiel 10a) werden in 200 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 1,98 g (5,46 mmol) Gadoliniumoxid zu und rührt 2 Stunden bei 70°C. Man kühlt auf Raumtemperatur ab und stellt mit 10 %iger aqu. 'Natronlauge auf pH 7,2. Die Lösung wird mit 3 g Aktivkohle versetzt, 1 Stunde bei Raumtemperatur gerührt und filtriert. Das Filtrat wird in eine Ultrafiltrationszelle gefüllt und dialysiert (AMICON® YM-1 (cut off 1000 DA)). Man dialysiert solange bis das Fluat eine Leitfähigkeit von 10 µS erreicht hat. Dann wird der Inhalt der Ultrafiltrationszelle gefriergetrocknet.
Ausbeute: 8,05 g (95 % d. Th.) eines farblosen, amorphen Pulvers
Wassergehalt: 6,9 %

| Elementaranalyse (berechnet auf wasserfreie Substanz): | | | | | | |
|---|---|---|---|---|---|---|
| ber. | C 31,95 | H 3,29 | N 7,81 | F 13,86 | Gd 20,24 | Na 2,96 |
| gef. | C 31,88 | H 3,41 | N 7,72 | F 13,74 | Gd 20,11 | Na 2,73 |

### Beispiel 11

a) 1,4,7-Tris(N-benzyloxycarbonyl)-10-N-(2H,2H,4H,4H,5H,5H-3-oxa-perfluorpentadecanoyl)-1,4,7,10-tetraazacyclododecan
21,65 g (34,8 mmol) der Titelverbindung aus Beispiel 5b) und 10 g (17,4 mmol) 1,4,7-Tris-(benzyloxycarbonyl)-1,4,7,10-tetraazacyclododecan werden in 200 ml Dimethylformamid gelöst und bei 0°C 8,61 g (34,8 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel Dichlormethan/2-Propanol= 20:1).
Ausbeute: 17,02 (83 % d. Th.) eines farblosen, zähen Öls

| Elementaranalyse: | | | | |
|---|---|---|---|---|
| ber. | C 46,87 | H 3,68 | N 4,75 | F 33,84 |
| gef. | C 46,63 | H 3,84 | N 4,61 | F 33,72 |

b) 1-N-(2H,2H,4H,4H,5H,5H-3-oxa-perfluor-pentadecanoyl)-1,4,7,10-tetraazacyclododecan
20 g (16,97 mmol) der Titelverbindung aus Beispiel 11a) werden in 400 ml Methanol gelöst und 3 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft.
Ausbeute: 13,17 g (quantitativ) eines glasigen, farblosen Feststoffes

| Elementaranalyse: | | | | |
|---|---|---|---|---|
| ber. | C 34,03 | H 3,25 | N 7,22 | F 51,38 |
| gef. | C 33,91 | H 3,42 | N 7,11 | F 51,24 |

c) 1,4,7-Tris{1,4,7-Tris[(N-carboxylatomethyl)]-10-[N-methyl-3-aza-2,5-dioxo-pentan-1,5-diyl]-1,4,7,10-tetraazacyclododecan, Gd-Komplex}-10-(N-2H,2H,4H,4H,5H,5H-3-oxa-perfluor-pentadecanoyl)-1,4,7,10-tetraazacyclododecan
40,55 g (64,4 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure und 6,63 g Natriumbromid (64,4 mmol) werden in 500 ml Dimethylsulfoxid bei 50°C gelöst. Nach Abkühlen auf Raumtemperatur werden 10,0 g (12,88 mmol) der in Beispiel 11b) beschriebenen Titelverbindung und 31,9 g (129 mmol) 2-Ethoxy-1-ethoxycarbonyl-1,2-dihydrochirolin zugegeben. Man rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, und über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 30,61 g (91 % d. Th.)
H₂O-Gehalt: 11,4 %

| Elementaranalyse (berechnet auf wasserfreie Substanz): | | | | | |
|---|---|---|---|---|---|
| ber. | C 36,33 | H 4,21 | N 10,19 | F 15,28 | Gd 18,06 |
| gef. | C 36,18 | H 4,40 | N 10,03 | F 15,17 | Gd 17,91 |

### Beispiel 12

a) N-Ethyl-N-(perfluoroctylsulfonyl)-amino-essigsäure-t-butylester
20 g (37,94 mmol) N-Ethylperfluorooctylsulfonamid und 15,73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton= 10:10:1). Nach Eindampfen der produkthaltigen Fraktionen, kristallisiert man den Rückstand aus Methanol/Ether um.
Ausbeute: 21,66 g (89 % d. Th.) eines wachsartigen farblosen Feststoffes

| Elementaranalyse: | | | | | |
|---|---|---|---|---|---|
| ber. | C 29,96 | H 2,51 | F 50,36 | N 2,18 | S 5,00 |
| gef. | C 29,81 | H 2,70 | F 50,15 | N 2,30 | S 4,83 |

b) N-Ethyl-N-(perfluoroctylsulfonyl)-amino-essigsäure
20 g (31,18 mmol) der Titelverbindung aus Beispiel 12a) werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 17,34 g (95 % d. Th.) eines farblosen kristallinen Feststoffes

| Elementaranalyse: | | | | | |
|---|---|---|---|---|---|
| ber. | C 24,63 | H 1,38 | F 55,19 | N 2,39 | S 5,48 |
| gef. | C 24,48 | H 1,50 | F 55,01 | N 2,17 | S 5,59 |

c) 1,4,7-Tris(N-benzyloxycarbonyl)-10-[2-(N-ethyl-N-perfluoroctylsulfonyl)-amino]acetyl-1,4,7,10-tetraazacyclododecan
17 g (29,04 mmol) der Titelverbindung aus Beispiel 12c) und 8,34 g (14,52 mmol) 1,4,7-Tris-(benzyloxycarbonyl)-1,4,7,10-tetraazacyclododecan werden in 200 ml Dimethylformamid gelöst und bei 0°C 7,18 g (29,04 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 20:1).
Ausbeute: 13,1 (79 % d. Th.) eines farblosen, zähen Öls

| Elementaranalyse: | | | | | |
|---|---|---|---|---|---|
| ber. | C 46,28 | H 3,88 | N 6,13 | F 28,28 | S 2,81 |
| gef. | C 46,17 | H 3,99 | N 6,04 | F 28,17 | S 2,74 |

d) 1-[2-(N-ethyl-N-perfluoracetylsulfonyl)-amino]-acetyl-1,4,7,10-tetraazacyclododecan
12 g (10,5 mmol) der Titelverbindung aus Beispiel 12c) werden in 200 ml Methanol gelöst und 2 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft.
Ausbeute: 7,77 g (quantitativ) eines glasigen, farblosen Feststoffes

| Elementaranalyse: | | | | | |
|---|---|---|---|---|---|
| ber. | C 32,48 | H 3,54 | N 9,47 | F 43,67 | S 4,34 |
| gef. | C 32,36 | H 3,61 | N 9,38 | F 43,58 | S 4,27 |

e) 1,4,7-Tris{1,4,7-tris[(N-carboxylatomethyl)]-10-[N-1-methyl-3-aza-2,5-dioxopentan-1,5-diyl]-1,4,7,10-tetraazacyclododecan, Gd-Komplex}-10-[2-(N-ethyl-N-perfluoroctylsulfonyl)-amino]-acetyl-1,4,7,10-tetraazacyclododecan
29,8 g (47,33 mmol) des in Beispiel 1f beschriebenen Gd-Komplexes der 10-(4-Carboxy-1-methyl-2-oxo-3-azabutyl)-1,4,7,10-tetraazacyclododecan-1,4,7-triessigsäure und 4,87 g Natriumbromid (47,33 mmol) werden in 400 ml Dimethylsulfoxid bei 50°C gelöst. Nach Abkühlen auf Raumtemperatur werden 7 g (9,47 mmol) der in Beispiel 12d beschriebenen Titelverbindung und 12,12 g (49 mmol) 2-Ethoxy-1-ethoxycarbonyl-1,2-dihydrochirolin zugegeben. Die erhaltene Suspension wird anschließend mit ausreichend Aceton bis zur vollständigen Fällung versetzt, der Niederschlag abgesaugt, getrocknet, in Wasser aufgenommen, und über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 22,43 g (92 % d. Th.)
H₂O-Gehalt: 10,6 %

| Elementaranalyse (berechnet auf wasserfreie Substanz): | | | | | | |
|---|---|---|---|---|---|---|
| ber. | C 35,92 | H 4,31 | N 10,88 | F 12,54 | Gd 18,32 | S 1,25 |
| gef. | C 35,86 | H 4,40 | N 10,82 | F 15,47 | Gd 18,28 | S 1,16 |

### Beispiel 13

a) Gadolinium-Komplex der 10-[4-Carboxy-2-oxo-3-aza-butyl]-1,4,7,10-tetraazacyclododecan-1,4,7-triessigsäure
25 g (36,45 mmol) 10-[4-(t-Butyloxycarbonyl)-2-oxo-3-azabutyl]-1,4,7,10-tetraazacyclododecan-1,4,7-triessigsäure-tri-tert.-butylester werden in 300 ml Trifluoressigsäure gelöst und 3 Stunden bei Raumtemperatur gerührt. Man dampft zur Trockne ein, nimmt den Rückstand in 300 ml Wasser auf und gibt die Lösung auf eine Säule, gefüllt mit Reillex®-425 PVP. Man eluiert mit Wasser. Die produkthaltigen Fraktionen werden vereinigt und zur Trockne eingedampft, der Rückstand wird aus Methanol/Aceton umkristallisiert. Ausbeute: 15,24 g (84 % d. Th.) eines farblosen, hygroskopischen Feststoffes Wassergehalt: 7,3 %

| Elementaranalyse (berechnet auf wasserfreie Substanz): | | | |
|---|---|---|---|
| ber. | C 46,85 | H 6,77 | N 15,18 |
| gef. | C 46,61 | H 6,95 | N 15,02 |

Zu 15 g (32,50 mmol) der oben beschriebenen Säure, gelöst in 200 ml Wasser, gibt man 5,86 g (16,25 mmol) Gadoliniumoxid und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Diethylether gewaschen und im Vakuumofen bei 130°C getrocknet (24 Stunden).
Ausbeute: 18,92 g (92 % d. Th.) eines farblosen, kristallinen Pulvers
Wassergehalt: 2,7 %

| Elementaranalyse (berechnet auf wasserfreie Substanz): | | | | |
|---|---|---|---|---|
| ber. | C 35,11 | H 4,58 | N 11,37 | Gd 25,54 |
| gef. | C 34,92 | H 4,71 | N 11,14 | Gd 25,33 |

b) 1,4,7-Tris{1,4,7-tris-(carboxylatomethyl)-10-(3,6-diaza-2,5,8-trioxo-octan-1,8-diyl)-1,4,7,10-tetraazacyclododecan, Gd-Komplex}-10-(N-2H,2H,4H,4H,5H,5H-3-oxaperfluor-tridecanoyl)-1,4,7,10-tetraazacyclododecan
16,94 g (27,5 mmol) des in Beispiel 13a) beschriebenen Gd-Komplexes der 10-(4-Carboxy-2-oxo-3-azabutyl)-1,4,7,10-tetraazacyclododecan-1,4,7-triessigsäure, 2,83 g Natriumbromid (27,5 mmol) und 6,33 g (55 mmol) N-Hydroxysuccinimid werden in 300 ml Dimethylsulfoxid bei 50°C gelöst. Nach Abkühlen auf Raumtemperatur werden 11,35 g (55 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (4,59 mmol) der in Beispiel 1e) beschriebenen Titelverbindung und 11,13 g (110 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylhamstoff abfiltriert und das Filtrat über eine AMICON® YM-1 Ultrafiltrationsmembran (cut off/1000 Da) entsalzt und von niedermolekularen Bestandteilen gereinigt. Das Retentat wird anschließend gefriergetrocknet.
Ausbeute: 11,16 g (92 % d. Th.)
H₂O-Gehalt: 9,5 %

| Elementaranalyse (berechnet auf wasserfreie Substanz): | | | | | |
|---|---|---|---|---|---|
| ber. | C 36,36 | H 4,35 | N 11,66 | Gd 17,85 | F 12,22 |
| gef. | C 36,28 | H 4,42 | N 11,58 | Gd 17,75 | F 12,14 |

### Beispiel 14

a) 22F,22F,22F,21F,21F,20F,20F,19F,19F,18F,18F,17F,17F,16F,16F,15F,15Fheptadecafluor-12oxa-docosan-carbonsaure-benzylester
Zu einer Mischung aus 10 g (21,55 mmol) 1H,1H,2H,2H-Perfluordodecanol und 0,73 g (2,15 mmol) Tetrabutylammoniumhydrogensulfat in 100 ml 60 %iger Kalilauge/50 ml Toluol tropft man unter starkem Rühren bei 0°C 18,83 g (53 mmol) 11-Bromundecansäurebenzylester zu. Man rührt 1 Stunde bei 0°C. Es werden 200 ml Toluol zugegeben, die wässrige Phase abgetrennt und 2 mal mit je 50 ml Toluol extrahiert. Die vereinigten organischen Phasen werden über Magnesiumsulfat getrocknet und im Vakuum eingedampft. Der Rückstand wird an Kieselgel chromatographiert (Laufmittel: Hexan/ Dichlormethan/Aceton= 20:10:1).
Ausbeute: 8,75 g (55 % d. Th.) eines farblosen viskosen Öls

| Elementaranalyse: | | | |
|---|---|---|---|
| ber. | C 45,54 | H 4,23 | F 43,73 |
| gef. | C 45,48 | H 4,31 | F 43,68 |

b) 22F,22F,22F,21F,21F,20F,20F,19F,19F,18F,18F,17F,17F,16F,16F,15F,15Fheptadecafluor-12oxa-docosan-carbonsäure
8 g (10,83 mmol) der Titelverbindung aus Beispiel 14a) werden in 200 ml Methanol gelöst und 2 g Palladiumkatalysator (10 % Pd/C) zugegeben. Man hydriert über Nacht bei Raumtemperatur. Es wird vom Katalysator abfiltriert und das Filtrat im Vakuum zur Trockne eingedampft.
Ausbeute: 7,02 g (quantitativ) eines glasigen, farblosen Feststoffes

| Elementaranalyse: | | | |
|---|---|---|---|
| ber. | C 38,90 | H 3,89 | F 49,81 |
| gef. | C 38,75 | H 3,98 | F 49,72 |

c) 1,4,7-Tris[N-(2-benzyloxycarbonylamino)-acetyl]-10-(N-22F,22F,22F,21F,21F, 20F,20F,19F,19F,18F,18F,17F,17F,16F,16F,15F,15F-heptadecafluor-12oxadocosanoyl)-1,4,7,10-tetraazacyclododecan
7 g (10,08 mmol) der Titelverbindung aus Beispiel 14b) und 4,02 g (5,4 mmol) der Titelverbindung aus Beispiel 14b) werden in 100 ml Dimethylformamid gelöst und bei 0°C 4,2 g (17 mmol) 2-Ethoxy-1-ethoxycarbonyl-1.2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 6,17 (83 % d. Th.) eines farblosen, zähen Öls

| Elementaranalyse: | | | | |
|---|---|---|---|---|
| ber. | C 51,49 | H 5,13 | N 7,12 | F 23,47 |
| gef. | C 51,39 | H 5,20 | N 7,19 | F 23,38 |

d) 1,4,7-Tris[N-(2-amino)-acetyl]-10-(N-22F,22F,22F,21F,21F,20F,20F, 19F,19F, 18F,18F,17F,17F,16F,16F,15F,15F-heptadecafiuor-12-oxa-docosanoyl-1,4,7,10-tetraazacyclododecan, Trihydrobromid
6 g (4,36 mmol) der Titelverbindung aus Beispiel 14c) werden in 50 ml Essigsäure gelöst und zu einer 60°C heißen Lösung aus 50 ml Bromwasserstoff in Eisessig (33 %ig) getropft. Man rührt 1 Stunde bei 60°C. Es wird auf 0°C abgekühlt und unter Rühren 1000 ml Diethylether langsam zugetropft. Man filtriert den ausgefallenen Niederschlag ab, wäscht 2 mal mit 200 ml Ether nach und trocknet im Vakuumofen (60°C).
Ausbeute: 4,93 g (93 % d. Th.) cremefarbener, kristalliner Feststoff

| Elementaranalyse: | | | | | |
|---|---|---|---|---|---|
| ber. | C 34,56 | H 4,56 | N 8,06 | F 26,55 | Br 19,70 |
| gef. | C 34,48 | H 4,70 | N 8,00 | F 26,48 | Br 19,46 |

e) 1,4,7-Tris{1,4,7-tris-(N-carboxylatomethyl)-10-[N-(4,7-diaza-3,6,9-trioxo)-nonan-2,9-diyl]-1,4,7,10-tetraazacyclododecan, Gd-Komplex-10-(N-22F,22F,22F,21F,21F, 20F,20F,19F, 19F, 18F, 18F, 17F,17F, 16F, 16F, 15F, 15F-heptadecafluor-12-oxadocosanoyl)-1,4,7,10-tetraazacyclododecan
13,98 g (22,9 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,36 g Natriumbromid (22,9 mmol) und 5,29 g (46 mmol) N-Hydroxysuccinimid werden in 200 ml Dimethylsulfoxid bei 50°C gelöst. Nach Abkühlen auf Raumtemperatur werden 9,49 g (46 mmol) N,N'-Dicyclohexylcarbodiimid zugesetzt und 60 Minuten voraktiviert. Zu der so hergestellten N-Hydroxysuccinimidester-Lösung gibt man eine Lösung von 4,5 g (3,7 mmol) der in Beispiel 14d) beschriebenen Titelverbindung und 9,31 g (92 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 9,78 g (94 % d. Th.)
H₂O-Gehalt: 6,8 %

| Elementaranalyse (berechnet auf wasserfreie Substanz): | | | | | |
|---|---|---|---|---|---|
| ber. | C 39,31 | H 4,95 | N 10,96 | F 11,49 | Gd 16,78 |
| gef. | C 39,24 | H 5,02 | N 10,87 | F 11,41 | Gd 16,69 |

### Beispiel 15

a) N-(Hexyl)-perfluoroctansulfonamid
Zu einer Mischung von 10,62 g (105 mmol) Triethylamin und 10,12 g (100 mmol) Benzylamin tropft man bei 80°C unter kräftigem Rühren 50.21 g (100 mmol) Perfluoroctansulfonylfluorid. Man rührt 2 Tage bei 80°C, versetzt das Reaktionsgemisch mit 300 ml Wasser und extrahiert 3 mal mit Ethylacetat. Die vereinigten organischen Extrakte werden über Natriumsulfat getrocknet, filtriert und eingeengt. Der Rückstand wird an Kieselgel chromatographiert (Laufmittel: Dichlormethan/Methanol= 4:1).
Ausbeute: 45,50 g (78 % d. Th.) einer farblosen Flüssigkeit

| Elementaranalyse: | | | | | |
|---|---|---|---|---|---|
| ber. | C 28,83 | H 2,42 | N 2,40 | S 5,50 | F 55,37 |
| gef. | C 28,29 | H 2,39 | N 2,44 | S 5,55 | F 55,50 |

b) N-(Hexyl)-(t-butyloxycarbonylmethyl)-perfluoroctylsulfonamid
22,13 g (37,94 mmol) der Titelverbindung aus Beispiel 15a) und 15,73 g (113,8 mmol) Kaliumcarbonat werden in 200 ml Aceton suspendiert und bei 60°C 14,80 g (75,87 mmol) Bromessigsäure-tert.-butylester zugetropft. Man rührt 3 Stunden bei 60°C. Man filtriert von den Salzen ab und dampft das Filtrat im Vakuum zur Trockne ein Der Rückstand wird an Kieselgel chromatographiert (Laufmittel: Hexan/Dichlormethan/Aceton= 10:10:1). Nach Eindampfen der produkthaltigen Fraktionen, kristallisiert man den Rückstand aus Methanol/Ether um.
Ausbeute: 23,02 g ( 87 % d. Th.) eines wachsartigen farblosen Feststoffes

| Elementaranalyse: | | | | | |
|---|---|---|---|---|---|
| ber. | C 34,34 | H 3,47 | N 2,01 | S 4,60 | F 46,31 |
| gef. | C 34,31 | H 3,61 | N 1,97 | S 4,65 | F 46,25 |

c) N-(Hexyl)-(perfluoroctylsulfonamid)-aminoessigsäure
20 g (28,43 mmol) der Titelverbindung aus Beispiel 15b) werden in 200 ml Trifluoressigsäure gelöst und über Nacht bei Raumtemperatur gerührt. Man dampft im Vakuum zur Trockne ein. Der Rückstand wird aus Methanol/Ether umkristallisiert.
Ausbeute: 16,74 g (91 % d. Th.) eines farblosen kristallinen Feststoffes

| Elementaranalyse: | | | | | |
|---|---|---|---|---|---|
| ber. | C 29,96 | H 2,51 | N 2,18 | S 5,00 | F 50,36 |
| gef. | C 29,87 | H 2,70 | N 2,05 | S 4,84 | F 50,17 |

d) 1,4,7-Tris[N-(2-benzyloxycarbonylamino)-acetyl]-10-[N-acetyl-(2-amino-N-hexyl-N-perfluoroctylsulfonyl)]-1,4,7,10-tetraazacyclododecan 16 g (24,95 mmol) der Titelverbindung aus Beispiel 15c) und 9,3 g (12,48 mmol) der Titelverbindung aus Beispiel 1c) werden in 200 ml Dimethylformamid gelöst und bei 0°C 3,46 g (14 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 13,5 g (79 % d. Th.) eines farblosen, zähen Öls

| Elementaranalyse: | | | | | |
|---|---|---|---|---|---|
| ber. | C 47,37 | H 4,49 | N 8,18 | F 23,59 | S 2,34 |
| gef. | C 47,29 | H 4,57 | N 8,09 | F 23,48 | S 2,28 |

e) 1,4,7-Tris[N-(2-amino)-acetyl]-10-[N-acetyl-(2-amino)-N-hexyl-N-pes-fluoroctylsulfonyl)]-1,4,7,10-tetraazacyclododecan, Trihydrobromid
10 g (7,30 mmol) der Titelverbindung aus Beispiel 15d) werden in 100 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1300 ml Diethylether langsam zugetropft. Man filtriert den ausgefallenen Niederschlag ab, wäscht 2 mal mit 150 ml Ether nach und trocknet im Vakuumofen (60°C).
Ausbeute: 8,48 g (96 % d. Th.) cremefarbener, kristalliner Feststoff

| Elementaranalyse: | | | | | | |
|---|---|---|---|---|---|---|
| ber. | C 29,79 | H 3,83 | N 9,26 | F 26,70 | Br 19,82 | S 2,65 |
| gef. | C 29,68 | H 3,95 | N 9,20 | F 26,62 | Br 19,73 | S 2,58 |

f) 1,4,7-Tris[1,4,7-tris(N-carboxylatomethyl)-10-(3,6-diaza-2,5-dioxo-octan-1,8-diyl)-1,4,7,10-tetraazacyclododecan, Gd-Komplex]-10-[2-(N-acethyl-(2-amino)-N-hexyl-N-perfluoroctylsulfonyl)]-1,4,7,10-tetraazacyclododecan
15,62 g (24,8 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,55 g Natriumbromid (24,8 mmol) und 5,75 g (50 mmol) N-Hydroxysuccinimid werden in 200 ml Dimethylsulfoxid bei 50°C gelöst. Nach Abkühlen auf Raumtemperatur werden 10,32 g (50 mmol) N,N'-Dicyclohexylcarbodiimid zugesetzt und 60 Minuten voraktiviert.
Zu der so hergestellten N-Hydroxysuccinimidester-Lösung gibt man eine Lösung von 5 g (4,13 mmol) der in Beispiel 15e) beschriebenen Titelverbindung und 10,12 g (100 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 10,6 g (93 % d. Th.)
H₂O-Gehalt: 11,0 %

| Elementaranalyse (berechnet auf wasserfreie Substanz): | | | | | | |
|---|---|---|---|---|---|---|
| ber. | C 36,56 | H 4,42 | N 11,67 | Gd 17,09 | F 11,70 | S 1,16 |
| gef. | C 36,48 | H 4,50 | N 11,61 | Gd 16,98 | F 11,61 | S 1,13 |

### Beispiel 16

a) 1,4,7-Tris(N-carboxylatomethyl)-10-[N-(6-aza-8-hydroxy-3-oxa-5-oxo)-nonan-9-ylsäure]-1,4,7,10-tetraazacyclododecan, Gd-Komplex
Zu einer Lösung aus 23,21 g (200 mmol) Diglycolsäureanhydrid und 102 g (177,76 mmol) 10-[3-Amino-2-hydroxypropyl]-1,4,7-triscarboxymethyl-1,4,7,10-tetraazacyclododecan-Gadolinium-Komplex in 1000 ml Formamid gibt man 36 g (355,5 mmol) Triethylamin. Man rührt über Nacht bei Raumtemperatur. In diese Lösung wird unter Rühren 8000 ml Aceton eingetropft, der ausgefallene Niederschlag abfiltriert, mit Aceton gewaschen und im Vakuum getrocknet. Das so getrocknete Material wird in 2000 ml Wasser gelöst und mit 2 N Salzsäure auf pH 3,2 eingestellt, anschließend wird gefriergetrocknet. Das gefriergetrocknete Pulver wird in sehr wenig Wasser gelöst und auf eine RP18-Säule gebracht. Man chromatographiert mit einem Laufmittelgemisch, bestehend aus Wasser/Tetrahydrofuran (Gradient). Die produkthaltigen Fraktionen werden im Vakuum zur Trockne eingeengt und der Rückstand aus 2-Propanol umkristallisiert. Nach Trocknen im Vakuumofen bei 120°C (24 Stunden) erhält man 100,54 g (82 % d. Th.) der Titelverbindung als farbloses, kristallines Pulver.
Wassergehalt: 3,1 %

| Elementaranalyse (berechnet auf wasserfreie Substanz): | | | | |
|---|---|---|---|---|
| ber. | C 36,57 | H 4,97 | N 10,15 | Gd 22,80 |
| gef. | C 36,41 | H 5,12 | N 9,96 | Gd 22,59 |

b) 1,4,7-Tris{1,4,7-tris(N-carboxylatomethyl)-10-[N-4,10-diaza-2-hydroxy-5,8,12-trioxo-7-oxa-dodecan-1,12-diyl], Gd-Komplex}-10-(N-2H,2H,4H,4H,5H,5H-3-oxaperfluortridecanoyl)-1,4,7,10-tetraazacyclododecan
19 g (27,52 mmol) des in Beispiel 16a) beschriebenen Gd-Komplexes und 2,81 g Natriumbromid (27,52 mmol) und 3,17 g (27,52 mmol) N-Hydroxysuccinimid werden in 250 ml Dimethylsulfoxid bei 50°C gelöst. Nach Abkühlen auf Raumtemperatur werden 6,19 g (30 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (4,59 mmol) der in Beispiel 1e) beschriebenen Titelverbindung und 6,07 g (60 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 12,35 g (94 % d. Th.)
H₂O-Gehalt: 9,6 %

| Elementaranalyse (berechnet auf wasserfreie Substanz): | | | | | |
|---|---|---|---|---|---|
| ber. | C 37,34 | H 4,58 | N 10,76 | Gd 16,48 | F 11,28 |
| gef. | C 37,25 | H 4,71 | N 10,60 | Gd 16.37 | F 11,13 |

### Beispiel 17

1,4,7-Tris{1,4,7-tris(N-carboxylatomethyl)-10-[N-(4-aza-2-hydroxy-5,9-dioxo-7-oxa)-nonan-1,9-diyl]-1,4,7,10-tetraazacyclododecan, Gd-Komplex}-10-2H,2H,4H,4H,5H,5H-3-oxa-perfluor-tridecanoyl)-1,4,7,10-tetraazacyclododecan

19 g (27,52 mmol) des in Beispiel 16a) beschriebenen Gd-Komplexes und 2,83 g Natriumbromid (27,52 mmol) werden in 200 ml Dimethylsulfoxid bei 50°C gelöst. Nach Abkühlen auf Raumtemperatur werden 3,1 g (4,59 mmol) der in Beispiel 4b) beschriebenen Titelverbindung und 9,89 g (40 mmol) 2-Ethoxy-1-ethoxycarbonyl-1,2-dihydrochinolin zugegeben und über Nacht bei Raumtemperatur gerührt. Die erhaltene Suspension wird anschließend mit ausreichend Aceton bis zur vollständigen Fällung versetzt, der Niederschlag abgesaugt, getrocknet, in Wasser aufgenommen, und über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 11,37 g (92 % d. Th.)
H₂O-Gehalt: 8,9 %

| Elementaranalyse (berechnet auf wasserfreie Substanz): | | | | | |
|---|---|---|---|---|---|
| ber. | C 37,04 | H 4,53 | N 9,89 | Gd 17,53 | F 12,00 |
| gef. | C 36,95 | H 4,65 | N 9.78 | Gd 17.37 | F 11,87 |

### Beispiel 18

a) 1,4,7-Tris[N-(2-benzyloxycarbonylamino)-acetyl]-10-[N-acetyl-(2-amino-N-ethyl-N-perfluoroctylsulfonyl)]-1,4,7,10-tetraazacyclododecan
20 g (39,48 mmol) der Titelverbindung aus Beispiel 12b) und 10,99 g (14,74 mmol) der Titelverbindung aus Beispiel 1c) werden in 200 ml Dimethylformamid gelöst und bei 0°C 3,46 g (14 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 16,06 g (83 % d. Th.) eines farblosen, zähen Öls

| Elementaranalyse: | | | | | |
|---|---|---|---|---|---|
| ber. | C 45,74 | H 4,07 | N 8,53 | F 24,60 | S 2,44 |
| gef. | C 45,68 | H 4,19 | N 8,48 | F 24,45 | S 2,36 |

b) 1,4,7-Tris[N-(2-amino)-acetyl]-10-[N-acetyl-(2-amino-N-ethyl-N-perfluoroctylsulfonyl)]-1,4,7,10-tetraazacyclododecan, Trihydrobromid
10 g (7,62 mmol) der Titelverbindung aus Beispiel 18a) werden in 100 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1600 ml Diethylether langsam zugetropft. Man filtriert den ausgefallenen Niederschlag ab, wäscht 2 mal mit 200 ml Ether nach und trocknet im Vakuumofen (60°C).
Ausbeute: 8 g (91 % d. Th.) cremefarbener, kristalliner Feststoff

| Elementaranalyse: | | | | | | |
|---|---|---|---|---|---|---|
| ber. | C 27,08 | H 3,32 | N 9,72 | F 28,00 | Br 20,78 | S 2,78 |
| gef. | C 26,94 | H 3,42 | N 9,63 | F 27,91 | Br 20,46 | S 2,74 |

c) 1,4,7-Tris{N-3,9-bis(N-t.butyloxycarbonylmethyl)-6-[N-(3-aza-2,5-dioxo-pentan1,5-diyl]-3,6,9-triazaundecan-1,11-disäure-di-tbutylester}-10-[N-acetyl-(2-amino-N-ethyl-N-perfluoroctylsulfonyl)]-1,4,7,10-tetraazacyclododecan
Zu 16,04 g (26 mmol) sym-DTPA-tetra-tert.butylester gelöst in 200 ml Dimethylformamid gibt man bei 0°C 5,98 g (52 mmol) N-Hydroxysuccinimid und 10,73 g (52 mmol) N,N'-Dicyclohexylcarbodiimid und rührt eine Stunde bei dieser Temperatur Anschließend rührt man 3 Stunden bei Raumtemperatur. Zu dieser Lösung gibt man 5 g (4,34 mmol) der Titelverbindung aus Beispiel 18b) und 10,12 g (100 mmol) Triethylamin. Man rührt 24 Stunden bei Raumtemperatur. Die Lösung wird zur Trockne eingedampft und der Rückstand in 300 ml Dichlormethan aufgenommen. Man filtriert vom Dicyclohexylharnstoff ab und wäscht das Filtrat 2 mal mit 150 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 11,68 g (95 % d. Th.) eines farblosen, zähen Öls

| Elementaranalyse: | | | | | |
|---|---|---|---|---|---|
| bei. | C 49,20 | H 6,55 | N 11,38 | F 11,40 | S 1,13 |
| gef. | C 49,10 | H 6,70 | N 11,31 | F 11,29 | S 1,06 |

d) 1,4,7-Tris{N-3,9-bis(carboxylatomethyl)-6-[N-3-aza-2,5-dioxo-pentan-1,5-diyl]-3,6,9-triazaundecan-1-carboxylato-11-säure, Gd-Komplex, Natriumsalz}-10-[Nacetyl-(2-amino-N-ethyl-N-perfluoroctylsulfonyl)]-1,4,7,10-tetraazacyclododecan
11 g (3,88 mmol) der Titelverbindung aus Beispiel 18c) werden in 200 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 2,11 g (5,82 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 2 g Aktivkohle versetzt, l Stunde bei Raumtemperatur gerührt und filtriert. Das Filtrat wird in eine Ultrafiltrationszelle gefüllt und dialysiert (AMICON® YM-1 (cut off 1000 DA)). Man dialysiert solange bis das Eluat eine Leitfähigkeit von 10 µS erreicht hat. Dann wird der Inhalt der Ultrafiltrationszelle gefriergetrocknet.
Ausbeute: 9,67 g (97 % d. Th.) eines farblosen, amorphen Pulvers
Wassergehalt: 10,1 %

| Elementaranalyse (berechnet auf wasserfreie Substanz): | | | | | | | |
|---|---|---|---|---|---|---|---|
| ber. | C 31,84 | H 3,38 | N 9,28 | F 12,59 | S 1,25 | Gd 20,24 | Na 2,96 |
| gef. | C 31,70 | H 3,51 | N 9,14 | F 12,45 | S 1,16 | Gd 20,11 | Na 2,73 |

### Beispiel 19

### 1,4,7-Tris{1,4,7-tris(N-carboxylatomethyl)-10-[N-(4,7-diaza-3,6,9-trioxo)-nonan-2,9-diyl]-1,4,7,10-tetraazacyclododecan, Gd-Komplex}-10-[N-acetyl-(2-amino-N-ethyl-N-perfluor-octylsulfonyl)]-1,4,7,10-tetraazacyclododecan

16,38 g (26,0 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,68 g Natriumbromid (26 mmol) und 5,98 g (52 mmol) N-Hydroxysuccinimid werden in 200 ml Dimethylsulfoxid bei 50°C gelöst. Nach Abkühlen auf Raumtemperatur werden 10,73 g (52 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (4,34 mmol) der in Beispiel 18b) beschriebenen Titelverbindung und 10,12 g (100 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 11,08 g (93 % d. Th.)
H₂O-Gehalt: 6,8 %

| Elementaranalyse (berechnet auf wasserfreie Substanz): | | | | | | |
|---|---|---|---|---|---|---|
| ber. | C 36,31 | H 4,37 | N 11,73 | F 11,76 | S 1,17 | Gd 17,18 |
| gef. | C 36,25 | H 4,46 | N 11,68 | F 11,70 | S 1,10 | Gd 17,09 |

### Beispiel 20

a) 1,4,7-Tris{N-[3-aza-(N-benzyloxycarbonyl)-5-oxo]-pent-5-yl-säure}-10-(N-2H,2H,4H,4H,5H,5H-3-oxa-perfluor-tridecanoyl)-1,4,7,10-tetraazacyclododecan
Zu 11,85 g (44,35 mmol) N-(Benzyloxycarbonyl)-aminodiessigsäure gelöst in 200 ml Tetrahydrofuran gibt man 9,28 (45 mmol) N,N'-Dicyclohexylcarbodiimid und rührt 3 Stunden bei Raumtemperatur. Man filtriert vom ausgefallenen Dicyclohexylharnstoff ab und gibt zum Filtrat 5 g (7,39 mmol) der Titelverbindung aus Beispiel 4b), 15,18 g (150 mmol) Triethylamin und 200 mg 4-(Dimethylamino)pyridin zu. Man erhitzt 12 Stunden auf 50°C. Man dampft zur Trockne ein, nimmt den Rückstand in 300 ml 10 %iger aqu. Zitronensäurelösung auf und extrahiert 3 mal mit je 250 ml Chloroform. Die vereinigten organischen Phasen werden über Magnesiumsulfat getrocknet und im Vakuum zur Trockne eingedampft. Der Rückstand wird an Kieselgel chromatographiert (Laufmittel: Chloroform/Methanol= 5:1, (+ 1 % Eisessig)).
Ausbeute: 8,52 g (81 % d. Th.) eines farblosen Feststoffes

| Elementaranalyse: | | | | |
|---|---|---|---|---|
| ber. | C 47,23 | H 4,10 | N 6,88 | F 22,68 |
| gef. | C 47,19 | H 4,21 | N 6,74 | F 22,57 |

b) 1,4,7-Tris[N-(3-aza-5-oxo)-pent-5-yl-säure]-10-(N-2H,2H,4H,4H,5H,5H-3-oxaperfluortridecanoyl)-1,4,7,10-tetraazacyclododecan, Trihydrobromid
8 g (5,62 mmol) der Titelverbindung aus Beispiel 20a) werden in 80 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1200 ml Diethylether langsam zugetropft. Man filtriert den ausgefallenen Niederschlag ab, wäscht 2 mal mit 150 ml Ether nach und trocknet im Vakuumofen (60°C).
Ausbeute: 6,75 g (95 % d. Th.) cremefarbener, kristalliner Feststoff

| Elementaranalyse: | | | | | |
|---|---|---|---|---|---|
| ber. | C 30,40 | H 3,43 | N 7,75 | F 25,54 | Br 18,96 |
| gef. | C 30,31 | H 3,51 | N 7,68 | F 25,47 | Br 18,74 |

c) 1,4,7-Tris{1,4,7-tris(N-carboxylatomethyl)-10-[N-(4,7-diaza-7-N-carboxymethyl-3,6,9-trioxo)-nonan-2,9-diyl]-1,4,7,10-tecraazacyclododecan, Gd-Komplex, Mononatriumsalz}-10-(N-2H,2H,4H,4H,5H,5H-3-oxa-perfluortridecanoyl)-1,4,7,10-tetraazacyclododecan
19,92 g (31,63 mmol) des in Beispiel 1f) beschriebenen Gd-Komplexes der 10-(4-Carboxy-1-methyl-2-oxo-3 -azabutyl)-1,4,7,10-tetraazacyclododecan-1,4,7-triessigsäure, 3,25 g Natriumbromid (31,63 mmol) und 3,64 g (31,63 mmol) N-Hydroxysuccinimid werden in 250 ml Dimethylsulfoxid bei 50°C gelöst. Nach Abkühlen auf Raumtemperatur werden 6,53 g (31,63 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (3,95 mmol) der in Beispiel 20b) beschriebenen Titelverbindung und 10,12 g (100 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird mit 1 N Natronlauge auf pH 7,2 eingestellt, anschließend gefriergetrocknet.
Ausbeute: 10,39 g (90 % d. Th.)
H₂O-Gehalt: 10,1 %

| Elementaranalyse (berechnet auf wasserfreie Substanz): | | | | | | |
|---|---|---|---|---|---|---|
| ber. | C 36,59 | H 4,14 | N 10,55 | F 11,05 | Gd 16,15 | Na 2,36 |
| gef. | C 36,50 | H 4,28 | N 10,47 | F 10,95 | Gd 16,03 | Na 2,14 |

### Beispiel 21

a) 1,4,7-Tris[N-(2-benzyloxycarbonylamino-asparaginsäure-benzylester-4-amid)]-10-[(N-2H,2H,4H,4H,5H,5H-3-oxa-perfluor-tridecanoyl)]-1,4,7,10-tetraazacyclododecan
5 g (7,39 mmol) der Titelverbindung aus Beispiel 4b) und 15,72 g (44 mmol) N-(Benzyloxycarbonyl-asparaginsäure-1-benzylester werden in 100 ml Dimethylformamid gelöst und bei 0°C 19,8 g (80 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 20:1).
Ausbeute: 9,64 g (77 % d. Th.) eines farblosen, zähen Öls

| Elementaranalyse: | | | | |
|---|---|---|---|---|
| ber. | C 54,58 | H 4,52 | N 5,79 | F 19,06 |
| gef. | C 54,48 | H 4,61 | N 5,71 | F 18,94 |

b) 1,4,7-Tris[N-(2-amino-asparaginsäure-4-amid)]-10-(N-2H,2H,4H,4H,5H,5H-3-oxaperfluor-tridecanoyl)-1,4,7,10-tetraazacyclododecan
8 g (4,72 mmol) der Titelverbindung aus Beispiel 21a) werden in 80 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1600 ml Diethylether langsam zugetropft. Man filtriert den ausgefallenen Niederschlag ab, wäscht 2 mal mit 150 ml Ether nach und trocknet im Vakuumofen (60°C).
Ausbeute: 5,67 g (95 % d. Th.) cremefarbener, kristalliner Feststoff

| Elementaranalyse: | | | | | |
|---|---|---|---|---|---|
| ber. | C 30,40 | H 3,43 | N 7,75 | F 25,54 | Br 18,96 |
| gef. | C 30,28 | H 3,51 | N 7,69 | F 25,47 | Br 18,87 |

c) 1,4,7-Tris{1,4,7-tris(N-carboxylatomethyl)-10-[N-(4,7-diaza-8-carboxy-3,6,9-trioxo)-decan-2,10-diyl]-1,4,7,10-tetraazacyclododecan, Gd-Komplex, Mononatriumsalz}-10-2H,2H,4H,4H,5H,5H-3-oxo-perfluortridecanoyl)-1,4,7,10-tetraazacyclododecan 14,94 g (23,7 mmol) des in Beispiel 1f) beschriebenen Gd-Komplexes der 10-(4-Carboxy-1-methyl-2-oxo-3 -azabutyl)-1,4,7,10-tetraazacyciododecan-1,4,7-triessigsäure, 2,44 g Natriumbromid (23,7 mmol) und 2,73 g (23,7 mmol) N-Hydroxysuccinimid werden in 200 ml Dimethylsulfoxid bei 50°C gelöst. Nach Abkühlen auf Raumtemperatur werden 4,89 g (23,7 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (3,95 mmol) der in Beispiel 21b) beschriebenen Titelverbindung und 10,12 g (100 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird mit 1 N Natronlauge auf pH 7,2 eingestellt, anschließend gefriergetrocknet.
Ausbeute: 10,73 g (93 % d. Th.)
H₂O-Gehalt: 8,5 %

| Elementaranalyse (berechnet auf wasserfreie Substanz): | | | | | | |
|---|---|---|---|---|---|---|
| ber. | C 36,59 | H 4,14 | N 10,55 | F 11,05 | Gd 16,15 | Na 2,36 |
| gef. | C 36,50 | H 4,23 | N 10,48 | F 10,96 | Gd 16,07 | Na 2,13 |

### Beispiel 22

a) 1,4,7-Tris[2,6-bis(benzyloxycarbonylamino)-hexanoyl]-10-(N-2H,2H,4H, 4H, 5H,5H-3-oxa-perfluortridecanoyl)-1,4,7,10-tetraazacyclododecan
5 g (7,39 mmol) der Titelverbindung aus Beispiel 4b) und 15,33 g (37 mmol) Di-Z-Lysin werden in 150 ml Dimethylformamid gelöst und bei 0°C 11,13 g (45 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 25:1).
Ausbeute: 10,34 g (75 % d. Th.) eines farblosen, zähen Öls

| Elementaranalyse: | | | | |
|---|---|---|---|---|
| ber. | C 55,36 | H 5,24 | N 7,51 | F 17,31 |
| gef. | C 55,28 | H 5,31 | N 7,43 | F 17,23 |

b) 1,4,7-Tris(2,6-diamino-hexanoyl)-10-N-2H,2H,4H,4H,5H,5H-3-oxa-perfluortridecanoyl)-1,4,7,10-tetraazacyclododecan, Hexahydrobromid
8 g (4,29 mmol) der Titelverbindung aus Beispiel 22a) werden in 80 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 1600 ml Diethylether langsam zugetropft. Man filtriert den ausgefallenen Niederschlag ab, wäscht 2 mal mit 200 ml Ether nach und trocknet im Vakuumofen (60°C).
Ausbeute: 5,38 g (96 % d. Th.) cremefarbener, kristalliner Feststoff

| Elementaranalyse: | | | | | |
|---|---|---|---|---|---|
| ber. | C 34,93 | H 5,17 | N 10.72 | F 24,72 | Br 18,34 |
| gef. | C 34,87 | H 5,25 | N 10,65 | F 24,57 | Br 18,15 |

c) 1,4,7-Tris{2,6-bis〈amino-[(3-aza-1,4-dioxohexan-1,5-diyl)-10-(N-1,4,7,10-tetraazacyclododecan-1,4,7-tris(carboxylatomethyl), Gd-Komplex]〉-hexanoyl}-10-(N-2H,2H,4H,4H,5H,5H-3-oxa-perfluor-tridecanoyl)-1,4,7,10-tetraazacyclododecan, Hexahydrobromid
28,97 g (46 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 4,7 g Natriumbromid (46 mmol) und 10,59 g (92 mmol) N-Hydroxysuccinimid werden in 300 ml Dimethylsulfoxid bei 50°C gelöst. Nach Abkühlen auf Raumtemperatur werden 18,98 g (92 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (3,83 mmol) der in Beispiel 22b) beschriebenen Titelverbindung und 20,34 g (200 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird mit 1 N Natronlauge auf pH 7,2 eingestellt, anschließend gefriergetrocknet.
Ausbeute: 16,49 g (91 % d. Th.)
H₂O-Gehalt: 10,2 %

| Elementaranalyse (berechnet auf wasserfreie Substanz): | | | | | |
|---|---|---|---|---|---|
| ber. | C 38,59 | H 4,88 | N 11,84 | F 6,83 | Gd 19,94 |
| gef. | C 38,50 | H 4,97 | N 11,68 | F 6,70 | Gd 19,82 |

### Beispiel 23

a) 1,4,7,-Tris[(N-(3-benzyloxycarbonylaminoglutarsäure-monoamid)]-10-(N-2H-2H-4H,4H,5H,5H-3-oxa-perfluor-tridecanoyl)-1,4,7,10-tetraazacyclododecan
Zu 12,47 g (44,35 mmol) 3-[N-(Benzyloxycarbonyl)amino]-glutarsäure gelöst in 200 ml Tetrahydrofuran gibt man 9,28 g (45 mmol) Dicyclohexylcarbodiimid und rührt 3 Stunden bei Raumtemperatur. Man filtriert vom ausgefallenen Dicyclohexylharnstoff ab und gibt zum Filtrat 5 g (7,39 mmol) der Titelverbindung aus Beispiel 4b), 15,18 g (150 mmol) Triethylamin und 200 mg 4-(Dimethylamino)pyridin zu. Man erhitzt 12 Stunden auf 50°C. Man dampft zur Trockne ein, nimmt den Rückstand in 300 ml 10 %iger aqu. Zitronensäurelösung auf und extrahiert 3 mal mit je 250 ml Chloroform. Die vereinigten organischen Phasen werden über Magnesiumsulfat getrocknet und im Vakuum zur Trockne eingedampft. Der Rückstand wird an Kieselgel chromatographiert (Laufmittel:
Chloroform/Methanol= 5:1, (+ 1 % Eisessig).
Ausbeute: 8,57 g (81 % d. Th.) eines farblosen Feststoffes

| Elementaranalyse: | | | | |
|---|---|---|---|---|
| ber. | C 48,27 | H 4,53 | N 6,68 | F 22,00 |
| gef. | C 48,10 | H 4,71 | N 6,47 | F 21,81 |

b) 1,4,7,-Tris(N-(3-amino-glutarsäure-monoamid)]-10-(N-2H-2H-4H,4H,5H,5H-3-oxaperfluor-tridecanoyl)-1,4,7,10-tetraazacyclododecan
5 g (3,41 mmol) der Titelverbindung aus Beispiel 23a) werden in 50 ml Essigsäure gelöst und zu einer 60°C heißen Lösung aus 100 ml Bromwasserstoff in Eisessig (33 %ig) getropft. Man rührt 1 Stunde bei 60°C. Es wird auf 0°C abgekühlt und unter Rühren 1000 ml Diethylether langsam zugetropft. Man filtriert den ausgefallenen Niederschlag ab, wäscht 2 mal mit 100 ml Ether nach und trocknet im Vakuumofen (60°C). Ausbeute: 4,19 g (94 % d. Th.) cremefarbener, kristalliner Feststoff

| Elementaranalyse: | | | | | |
|---|---|---|---|---|---|
| ber. | C 32,18 | H 3,78 | N 7,50 | F 24,72 | Br *18,35* |
| gef. | C 32,10 | H 3,94 | N 7,35 | F 24,51 | Br 18,14 |

c) 1,4,7,-Tris{1,4,7-tris(N-carboxylatomethyl)-10-[N-(4,7-diaza-8-carboxymethyl-3,6,10-trioxo)-decan-2,10-diyl]-1,4,7,10-tetraazacyclododecan, Gd-Komplex, Mononatriumsalz}-10-(N-2H-2H-4H,4H,5H,5H-3-oxa-perfluor-tridecanoyl)-1,4,7,10-tetraazacyclododecan
11,57 g (18,4 mmol) des in Beispiel 1f) beschriebenen Gd-Komplexes der 10-(4-Carboxy-1-methyl-2-oxo-3-azabutyl)-1,4,7,10-tetraazaryclododecan-1,4, 7-triessigsaure, 1,89 g Natriumbromid (18,4 mmol) und 2,12 g (18,4 mmol) N-Hydroxysuccinimid werden in 150 ml Dimethylsulfoxid bei 50°C gelöst. Nach Abkühlen auf Raumtemperatur werden 3,80 g (18,4 mmol) N,N'-Dicyclohexylcarbodiimid zugesetzt und 60 Minuten voraktiviert. Zu der so hergestellten N-Hydroxysuccinimidester-Lösung gibt man eine Lösung von 4 g (3,06 mmol) der in Beispiel 23b) beschriebenen Titelverbindung und 4.05 g (40 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird mit 1 N Natronlauge auf pH 7,2 eingestellt, anschließend gefriergetrocknet.
Ausbeute: 8,62 g (95 % d. Th.)
H₂O-Gehalt: 9,6 %

| Elementaranalyse (berechnet auf wasserfreie Substanz): | | | | | | |
|---|---|---|---|---|---|---|
| ber. | C 37,28 | H 4,28 | N 10,40 | F 10,90 | Gd 15,93 | Na 2,33 |
| gef. | C 37,14 | H 4,41 | N 10,25 | F 10,73 | Gd 15,75 | Na 2,10 |

### Beispiel 24

a) 4-[(N-Ethyl-N-perfluoroctylsulfonyl)-aminomethyl]-benzoesäuremethylester
20 g (37,94 mmol) N-Ethylperfluorooctylsulfonamid und 15,73 g (113,8 mmol) Kaliumcarbonat werden in 200 ml Aceton suspendiert und bei 60°C (75,87 mmol) 4-(Brommethyl)-benzoesäuremethylester zugetropft. Man rührt 3 Stunden bei 60°C. Man filtriert von den Salzen ab und dampft das Filtrat im Vakuum zur Trockne ein. Der Rückstand wird an Kieselgel chromatographiert (Laufmittel: Hexan/Dichlormethan/Aceton= 10:10:1). Nach Eindampfen der produkthaltigen Fraktionen, kristallisiert man den Rückstand aus Methanol/Ether um.
Ausbeute: 22,80 g (89 % d. Th.) eines wachsartigen farblosen Feststoffes

| Elementaranalyse: | | | | | |
|---|---|---|---|---|---|
| ber. | C 33,79 | H 2,09 | F 47,82 | N 2,07 | S 4,75 |
| gef. | C 33,61 | H 2,28 | F 47,65 | N 2,01 | S 4,68 |

b) 4-[(N-Ethyl-N-perfluoroctylsulfonyl)-amino-methyl]-benzoesäure
22 g (32,58 mmol) der Titelverbindung aus Beispiel 24a) werden in einer Mischung aus 100 ml H₂O/200 ml Ethanol gelöst und 4 g (100 mmol) Natriumhydroxid zugesetzt. Man erwärmt 5 Stunden auf 60°C. Es wird im Vakuum zur Trockne eingedampft und der Rückstand in 400 ml 5 %iger aqu. Salzsäure aufgenommen. Man extrahiert 2 mal mit je 250 ml Dichlormethan. Die organische Phase wird über Magnesiumsulfat getrocknet und im Vakuum zur Trockne eingedampft, der Rückstand wird aus Diethylether/n-Hexan umkristallisiert.
Ausbeute: 21,12 g (98 % d. Th.) farbloser, wachsartiger Kristalle

| Elementaranalyse: | | | | | |
|---|---|---|---|---|---|
| ber. | C 32,69 | H 1,83 | N 2,12 | F 48,84 | S 4,85 |
| gef. | C 32,47 | H 2,02 | N 2,02 | F 48,65 | S 4,68 |

c) 1,4,7-Tris[N-(2-benzyloxycarbonylamino)-acetyl]-10-{[4-(N-Ethyl-N -perfluoroctylsulfonyl)-aminomethyl]-benzoyl}-1,4,7,10-tetraazacyclododecan 21 g (31,75 mmol) der Titelverbindung aus Beispiel 24b) und 11,86 g (15,9 mmol) der Titelverbindung aus Beispiel 1c) werden in 200 ml Dimethylformamid gelöst und bei 0°C 8,66 g (35 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1). Ausbeute: 18,7 g (85 % d. Th.) eines farblosen, zähen Öls

| Elementaranalyse: | | | | | |
|---|---|---|---|---|---|
| ber. | C 48,42 | H 4,14 | N 8,07 | F 23,25 | S 2,31 |
| gef. | C 48,25 | H 4,02 | N 7,92 | F 23,04 | S 2,18 |

d) 1,4,7,-Tris-[N-(2-amino)-acetyl]-10-{4-[(N-ethyl-N-perfluoroctylsulfonyl)-aminomethyl]-benzoyl}-1,4,7,10-tetraazacyclododecan, Trihydrobromid
10 g (7,2 mmol) der Titelverbindung aus Beispiel 24c) werden in 100 ml Essigsäure gelöst und zu einer 60°C heißen Lösung aus 100 ml Bromwasserstoff in Eisessig (33 %ig) getropft. Man rührt 1 Stunde bei 60°C. Es wird auf 0°C abgekühlt und unter Rühren 1600 ml Diethylether langsam zugetropft. Man filtriert den ausgefallenen Niederschlag ab, wäscht 2 mal mit 200 ml Ether nach und trocknet im Vakuumofen (60°C).
Ausbeute: 8,59 g (97 % d. Th.) cremefarbener, kristalliner Feststoff

| Elementaranalyse: | | | | | | |
|---|---|---|---|---|---|---|
| ber. | C 31,26 | H 3,44 | N 9,11 | F 26,27 | S 2,61 | Br 19,50 |
| gef. | C 31,11 | H 3,60 | N 9,02 | F 26,09 | S 2,48 | Br 19,27 |

e) 1,4,7-Tris{1,4,7-tris(N-carboxylatomethyl)-10-[N-(4,7-diaza-3,6,9-trioxo)-nonan-1,9-diyl]-Gd-Komplex}-10-{4-[(N-ethyl-N-perfluoroctylsulfonyl)-aminomethyl]-benzoyl}-1,4,7,10-tetraazacyclododecan
15,74 g (25 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 2,57 g Natriumbromid (25 mmol) und 5,75 g (18,4 mmol) N-Hydroxysuccinimid werden in 200 ml Dimethylsulfoxid bei 50°C gelöst. Nach Abkühlen auf Raumtemperatur werden 10,32 g (50 mmol) N,N'-Dicyclohexylcarbodiimid zugesetzt und 60 Minuten voraktiviert. Zu der so hergestellten N-Hydroxysuccinimidester-Lösung gibt man eine Lösung von 5 g (4,07 mmol) der in Beispiel 24d) beschriebenen Titelverbindung und 10,12 g (100 mmol) Triethylamin in 25 ml Wasser und rührt über Nacht bei Raumtemperatur. Die erhaltene Suspension wird anschließend mit ausreichend Aceton bis zur vollständigen Fällung versetzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird mit 1 N Natronlauge auf pH 7,2 eingestellt, anschließend gefriergetrocknet.
Ausbeute: 10,91 g (95 % d. Th.)
H₂O-Gehalt: 9,6 %

| Elementaranalyse (berechnet auf wasserfreie Substanz): | | | | | | |
|---|---|---|---|---|---|---|
| ber. | C 37,88 | H 4,39 | N 11,42 | F 11,45 | S 1,14 | Gd 16.72 |
| gef. | C 37,64 | H 4,61 | N 11,27 | F 11,28 | S 1,08 | Gd 16,58 |

### Beispiel 25

a) 1,4,7-Tris[N-(2-benzyloxycarbonylamino)-acetyl]-10-N-[(11-t.butyloxycarbonylamino)-undecanoyl]-1,4,7,10-tetraazacyclododecan
10 g (13,41 mmol) der Titelverbindung aus Beispiel 1c) und 8,10 g (26,8 mmol) 11-N-(tert.butoxycarbonyl)-aminoundecansäure werden in 200 ml Dimethylformamid gelöst und bei 0°C 9,89 g (40 mmol) 2-Ethoxy-1-ethoxycarbonyl-1,2-dihydrochinolin zugegeben. Man rührt 24 Stunden bei Raumtemperatur. Es wird im Vakuum zur Trockne eingedampft und der Rückstand an Kieselgel chromatographiert (Laufmittel: Dichlormethan/Aceton= 15:1).
Ausbeute: 18,7 g (85 % d. Th.) eines farblosen, zähen Öls

| Elementaranalyse: | | | |
|---|---|---|---|
| ber. | C 63,02 | H 7,44 | N 10,89 |
| gef. | C 62,90 | H 7,61 | N 10,63 |

b) 1,4,7-Tris[N-(2-benzyloxycarbonylamino)-acetyl]-10-N-(11-aminoundecanoyl)-1,4,7,10-tetraazacyclododecan
12 g (11,66 mmol) der Titelverbindung aus Beispiel 25a) werden in 180 ml Trifluoressigsäure gelöst und über Nacht bei Raumtemperatur gerührt. Man dampft im Vakuum zur Trockne ein. Der Rückstand wird in 200 ml 2 N-Natronlauge aufgenommen und 2 mal mit je 150 ml Dichlormethan extrahiert. Die vereinigten organischen Phasen werden über Magnesiumsulfat getrocknet und im Vakuum eingedampft. Der Rückstand wird an Kieselgel chromatogaphiert (Laufmittel: Dichlormethan/Methanol= 15:1, (+ 2 Triethylamin)). Ausbeute: 10,18 g (94 % d. Th.) eines farblosen Feststoffes

| Elementaranalyse: | | | |
|---|---|---|---|
| ber. | C 63,34 | H 7,38 | N 12,06 |
| gef. | C 63,15 | H 7,57 | N 11,84 |

c) 1,4,7-Tris[N-(2-benzyloxycarbonylamino)-acetyl]-10-N-{undecanoyl-〈11-amino-N-[acetyl-(2-amino-N-ethyl-N-perfluoroctylsulfonyl)]〉}-1,4,7,10-tetraazacyclododecan
11,70 g (20 mmol) der Titelverbindung aus Beispiel 12b) und 2,30 g (20 mmol) N-Hydroxysuccinimid werden in 100 ml Dimethylformamid gelöst und auf 0°C abgekühlt. Man gibt 4,13 g (20 mmol) N,N'-Dicyclohexylcarbodiimid hinzu und rührt 30 Min. bei 0°C, anschließend 2 Stunden bei Raumtemperatur. Man kühlt auf 0°C und gibt 10 g (10,76 mmol) der Titelverbindung aus Beispiel 25b) zu, anschließend 4,05 g (40 mmol) Triethylamin und läßt langsam auf Raumtemperatur kommen (ca. 4 Stunden). Es wird im Vakuum zur Trockne eingedampft, der Rückstand in 400 ml Methylenchlorid aufgenommen und vom Harnstoff abfiltriert. Das Filtrat wird 3 mal mit einer wässrigen Salzsäure (pH 3,0 je 600 ml) extrahiert, über Magnesiumsulfat getrocknet und zur Trockne eingedampft. Der Rückstand wird in Kieselgel chromatographiert (Laufmittel: Methylenchlorid/Methanol = 20:1, (+ 2 % Essigsäure)).
Ausbeute: 14,22 g (91 % d. Th.) eines farblosen Feststoffes

| Elementaranalyse: | | | | | |
|---|---|---|---|---|---|
| ber. | C 50,45 | H 5,14 | N 8,68 | F 17,01 | S 2,21 |
| gef. | C 50,27 | H 5,31 | N 8,49 | F 16,83 | S 2,07 |

d) 1,4,7-Tris[N-(2-amino)-acetyl]-10-N-{undecanoyl-〈11-amino-N-[acetyl-(2-amino-N-ethyl-N-perfluoroctylsulfonyl)]〉}-1,4,7,10-tetraazacyclododecan, Trihydrobromid 10 g (6,89 mmol) der Titelverbindung aus Beispiel 25c) werden in 100 ml Essigsäure gelöst und zu einer 60°C heißen Lösung aus 200 ml Bromwasserstoff in Eisessig (33 %ig) getropft. Man rührt 1 Stunde bei 60°C. Es wird auf 0°C abgekühlt und unter Rühren 2000 ml Diethylether langsam zugetropft. Man filtriert den ausgefallenen Niederschlag ab, wäscht 2 mal mit 200 ml Ether nach und trocknet im Vakuumofen (60°C).
Ausbeute: 8,93 g (97 % d. Th.) cremefarbener, kristalliner Feststoff

| Elementaranalyse: | | | | | | |
|---|---|---|---|---|---|---|
| ber. | C 33,25 | H 4,45 | N 9,43 | F24,16 | S 2,40 | Br 17,93 |
| gef. | C 33,07 | H 4,68 | N 9,30 | F 24,03 | S 2,17 | Br 17,68 |

e) 1,4,7-Tris{N-[3,9-bis(N-t.butyloxycarbonylmethyl)]-6-[N-(3-aza-2,5-dioxo-pentan)-1,5-diyl]-3,6,9-triazaundecan-1,11-disaure-di-t.butylester}-10-N-{undecanoyl-〈11-amino-N-[acetyl-(2-amino-N-ethyl-N-perfluoroctylsulfonyl)]〉}-1,4,7,10-tetraazacyclododecan
Zu 27,35 g (44,35 mmol) sym-DTPA-tetra-tert.butylester gelöst in 400 ml Dimethylformamid gibt man bei 0°C 10,21 g (88,7 mmol) N-Hydroxysuccinimid und 18,30 g (88,7 mmol) N.N'-Dicyclohexylcarbodiimid und rührt eine Stunde bei dieser Temperatur. Anschließend rührt man 3 Stunden bei Raumtemperatur. Zu dieser Lösung gibt man 9,88 g (7,39 mmol) der Titelverbindung aus Beispiel 25d) und 17,91 g (177 mmol) Triethylamin. Man rührt 24 Stunden bei Raumtemperatur. Die Lösung wird zur Trockne eingedampft und der Rückstand in 400 ml Dichlormethan aufgenommen. Man filtriert vom Dicyclohexylharnstoff ab und wäscht das Filtrat 2 mal mit 200 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 19,67 g (92 % d. Th.) eines farblosen, zähen Öls

| Elementaranalyse: | | | | | |
|---|---|---|---|---|---|
| ber. | C 52,72 | H 7,49 | N 8,71 | F 11,16 | S 1,11 |
| gef. | C 52,60 | H 7.62 | N 8,60 | F 11,01 | S 1,04 |

f) 1,4,7-Tris{N-[3,9-bis{N-carboxylatomethyl)]-6-[N-{3-aza-2,5-dioxo-pentan)-1,5-diyl]-3,6,9-triazaundecan-1-carboxylato-11-säure, Gd-Komplex, Mononatriumsalz}-10-N-{undecanoyl-〈11-amino-N-[acetyl-(2-amino-N-ethyl-N-perfluoroctylsulfonyl)]) }-1,4,7,10-tetraazacyclododecan
10,53 g (3,64 mmol) der Titelverbindung aus Beispiel 25e) werden in 200 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 1,98 g (5,46 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 3 g Aktivkohle versetzt, 1 Stunde bei Raumtemperatur gerührt und filtriert. Das Filtrat wird in eine Ultrafiltrationszelle gefüllt und dialysiert (AMICON® YM-1 (cut off 1000 DA)). Man dialysiert solange bis das Eluat eine Leitfähigkeit von 10 µS erreicht hat. Dann wird der Inhalt der Ultrafiltrationszelle gefriergetrocknet.
Ausbeute: 9,50 g (95 % d. Th.) eines farblosen, amorphen Pulvers
Wassergehalt: 7,5 %

| Elementaranalyse (berechnet auf wasserfreie Substanz): | | | | | | | |
|---|---|---|---|---|---|---|---|
| ber. | C 34,52 | H 3,92 | N 9,17 | F 11,75 | S 1,17 | Gd 17,16 | Na 2,51 |
| gef. | C 34,37 | H 4,08 | N 9,09 | F 11,69 | S 1,08 | Gd 17,05 | Na 2,33 |

### Beispiel 26

a) 1,4,7-Tris{N-2,6-[bis(benzyloxycarbonylamino)]-hexanoyl}-10-N-[acetyl-(2-amino-N-ethyl-N-perfluoroctylsulfonyl)]}-1,4,7,10-tetraazacyclododecan
5,46 g (7,39 mmol) der Titelverbindung aus Beispiel 12d) und 15,33 g (37 mmol) Di-Z-Lysin werden in 150 ml Dimethylformamid gelöst und bei 0°C 11,13 g (45 mmol) 2-Ethoxy-1-ethoxycarbonyl-1,2-dihydrochinolin zugegeben. Man rührt 24 Stunden bei Raumtemperatur Es wird im Vakuum zur Trockne eingedampft und der Rückstand an Kieselgel chromatographiert (Laufmittel: Dichlormethan/Aceton= 25:1).
Ausbeute: 11,12 g (78 % d. Th.) eines farblosen, zähen Öls

| Elementaranalyse: | | | | | |
|---|---|---|---|---|---|
| ber. | C 53,55 | H 5,12 | N 7,99 | F 16,74 | S 1,66 |
| gef. | C 53,37 | H 5,31 | N 7,82 | F 16,55 | S 1,49 |

b) 1,4,7-Tris{[N-(2,6-diamino)-hexanoyl]-10-[N-acetyl-(2-amino-N-ethyl-N-perfluoroctylsulfonyl)]}-1,4,7,10-tetraazacyclododecan
8,27 g (4,29 mmol) der Titelverbindung aus Beispiel 26a) werden in 80 ml Essigsäure gelöst und zu einer 60°C heißen Lösung aus 150 ml Bromwasserstoff in Eisessig (33 %ig) getropft. Man rührt 1 Stunde bei 60°C. Es wird auf 0°C abgekühlt und unter Rühren 600 ml Diethylether langsam zugetropft. Man filtriert den ausgefallenen Niederschlag ab, wäscht 2 mal mit 200 ml Ether nach und trocknet im Vakuumofen (60°C).
Ausbeute: 6,56 g (95 % d. Th.) cremefarbener, kristalliner Feststoff

| Elementaranalyse: | | | | | | |
|---|---|---|---|---|---|---|
| ber. | C 28,36 | H 4,26 | N 9,57 | F 20,07 | S 1,99 | Br 29,79 |
| gef. | C 28,17 | H 4,41 | N 9,41 | F 19,92 | S 1,85 | Br 29,45 |

c) 1,4,7-Tris{N-hexanoyl-2,6-bis(amino-N-acetyl-2-[3,9-bis(N-t.butyloxy-carbonylmethyl)-6-yl-3,6,9-triaazaundecan-1,11-dicarbonsäure-di-t.butylester]〉}-10-[N-Acetyl-(2-amino-N-ethyl-N-perfluoroctylsulfonyl)]}-1,4,7,10-tetraazacyclododecan
Zu 54,7 g (88,7 mmol) sym-DTPA-tetra-tertbutylester gelöst in 500 ml Dimethylformamid gibt man bei 0°C 20,42 g (177,4 mmol) N-Hydroxysuccinimid und 36,6 g (177,4 mmol) N,N'-Dicyclohexylcarbodiimid und rührt eine Stunde bei dieser Temperatur. Anschließend rührt man 3 Stunden bei Raumtemperatur. Zu dieser Lösung gibt man 11,89 g (7,39 mmol) der Titelverbindung aus Beispiel 26b) und 35,42 g (350 mmol) Triethylamin. Man rührt 24 Stunden bei Raumtemperatur. Die Lösung wird zur Trockne eingedampft und der Rückstand in 800 ml Dichlormethan aufgenommen. Man filtriert vom Dicyclohexylharnstoff ab und wäscht das Filtrat 2 mal mit 400 ml 5 %iger aqu. Natriumcarbonat-Lösung. Die organische Phase wird über Magnesiumsulfat getrocknet und im Vakuum zur Trockne eingedampft. Der Rückstand wird an Kieselgel chromatographiert (Laufmittel: Dichlormethan/Methanol= 20:1).
Ausbeute: 30,68 g (92 % d. Th.) eines farblosen, zähen Öls

| Elementaranalyse: | | | | | |
|---|---|---|---|---|---|
| ber. | C 53,23 | H 8,62 | N 9,00 | F 7,16 | S 0,71 |
| gef. | C 53,05 | H 8,84 | N 8,81 | F 7,03 | S 0,65 |

d) 1,4,7-Tris{N-hexanoyl-2,6-bis〈*amino-N-acetyl-2-[3,9-bis(N-carboxylato-methyl)-6-yl-3,6,9-triazaundecan-1-carboxylato-11-säure, Gd-Komplex, Mononatriumsalz]〉}-10-[N-acetyl-(2-amino-N-ethyl-N-perfluoroctylsulfonyl)]-1,4,7,10-tetraazacyclododecan
16,43 g (3,64 mmol) der Titelverbindung aus Beispiel 26c) werden in 300 ml Trifluoressigsäure gelöst und 12 Stunden bei Raumtemperatur gerührt. Man dampft im Vakuum zur Trockne ein. Der Rückstand wird in 200 ml Wasser gelöst und mit 10 %iger aqu. Natronlauge auf pH 4 gestellt. Anschließend gibt man 3,96 g (10,92 mmol) Gadoliniumoxid zu und rührt 2 Stunden bei 70°C. Man kühlt auf Raumtemperatur ab und stellt mit 10 %iger aqu. Natronlauge auf pH 7,2. Die Lösung wird mit 3 g Aktivkohle versetzt, 1 Stunde bei Raumtemperatur gerührt und filtriert. Das Filtrat wird in eine Ultrafiltrationszelle gefüllt und dialysiert (AMICON® YM-1 (cut off 1000 DA)) Anschließend wird der Inhalt der Ultrafiltrationszelle gefriergetrocknet.
Ausbeute: 14,76 g (96 % d. Th.) eines farblosen, amorphen Pulvers
Wassergehalt: 9,5 %

| Elementaranalyse (berechnet auf wasserfreie Substanz): | | | | | | | |
|---|---|---|---|---|---|---|---|
| ber. | C 29,58 | H 4,06 | N 9,62 | F 7,65 | S 0,76 | Gd 22,34 | Na 3,27 |
| gef. | C 29,39 | H 4,24 | N 9,43 | F 7,48 | S 0,69 | Gd 22,17 | Na 3,11 |

### Beispiel 27

1,4,7-Tris{N-hexanoyl-2,6-bis〈amino-N-acetyl-2-[aminopropanoyl-(2-yl)]-[1,4,7-tris(Ncarboxylatomethyl)-1,4,7,10-tetraazacyclododecan-10-yl, Gd-Komplex]〉}-10-[N-acetyl-(2-amino-N-ethyl-N-perfluoroctylsulfonyl)]-1,4,7,10-tetraazacyclododecan

28,97 g (46 mmol) des in Beispiel 1f) beschriebenen Gd-Komplexes der 10-(4-Carboxy-1-methyl-2-oxo-3-azabutyl)-1,4,7,10-tetraazacyclododecan-1,4,7-triessigsäure, 4,7 g Natriumbromid (46 mmol) und 10,59 g (92 mmol) N-Hydroxysuccinimid werden in 300 ml Dimethylsulfoxid bei 50°C gelöst. Nach Abkühlen auf Raumtemperatur werden 18,98 g (92 mmol) N,N'-Dicyclohexylcarbodiimid zugesetzt und 60 Minuten voraktiviert. Zu der so hergestellten N-Hydroxysuccinimidester-Lösung gibt man eine Lösung von 6,16 g (3,83 mmol) der in Beispiel 26b) beschriebenen Titelverbindung und 20,34 g (200 mmol) Triethylamin in 25 ml Wasser und rührt über Nacht bei Raumtemperatur. Die erhaltene Suspension wird anschließend mit ausreichend Aceton bis zur vollständigen setzt, der Niederschlag abgesaugt, getrocknet, in Wasser aufgenommen, von unlöslichem Dicyclohexylharnstoff abfiltriert und das Filtrat über eine AMICON® YM-1 Ultrafiltrationsmembran (cut off 1000 Da) entsalzt und von niedermolekularen Bestandteilen gereinigt. Das Retentat wird anschließend gefriergetrocknet.
Ausbeute: 16,15 g (92 % d. Th.)
H₂O-Gehalt: 11,3 %

| Elementaranalyse (berechnet auf wasserfreie Substanz): | | | | | | |
|---|---|---|---|---|---|---|
| ber. | C 35,11 | H 5.19 | N 12,53 | S 0,70 | F 7,05 | Gd 20,58 |
| gef. | C 35,01 | H 5,32 | N 11,27 | S 0,64 | F 6,91 | Gd 20,39 |

**Tabelle 1:**

| **Bestimmung der T**^{**1**}**-Relaxivity ausgewählter Verbindungen** | |
|---|---|
| Die Relaxivity der folgenden Verbindungen wurde mit einem Minispec pc 20 (20 MHz, 0,47T) bei 37 °C in Humanplasma bestimmt und mit der von Gd-DTPA-Polylysin und Magnevist ® als Vegleichssubstanzen verglichen. Da die erfindungsgemäßen Substanzen mehr als ein Metallatom enthalten, wurde die Relaxivity für Vergleichszwecke auf ein Metallatom normiert. | |

| Substanz Beispiel Nr. | R¹ [L/mmol * sec] bei 0.47 T und 37 °C |
|---|---|
| 14 e) | 19,3 |
| 8 b) | 20,6 |
| 18 d) | 23,0 |
| 19 | 21,4 |
| 25 f) | 15,1 |
| 1 g) | 19,5 |
| 26 d) | 13,0 |
| 27 | 16,1 |
| Magnevist ® | 4,8 |
| Gd-DTPA-Polylysin | 16,8 |

### Beispiel 28

### Lymphknotenanreicherung am Meerschweinchen

Der Gadolinium-Komplex des Beispiels 19 wurde 30 Min./ 90 Min. nach subkutaner Gabe (10 µmol Gesamtgadolinium/kg KG, Hinterpfote s.c.) an stimulierten Meerschweinchen (komplettes Freund-Adjuvant; jeweils 0,1 ml i.m. in den rechten und linken Ober- und Unterschenkel; 2 Wochen vor Gabe der Prüfsubstanz) hinsichtlich ihrer Lymphknotenanreicherung in drei aufeinanderfolgenden Lymphknotenstationen (popliteal, inguinal, iliakal) untersucht. Hierbei wurden die nachfolgend aufgelisteten Ergebnisse (Ermittlung der Gadolinium-Konzentration mittels ICP-AES) erhalten:

| Gd-Anreicherung [µmol/l] in drei aufeinanderfolgenden Lymphknotenstationen 30 und 90 min nach interstitieller Applikation von 10 µmol/kg (MW ± SD. n=3) | | |
|---|---|---|
| Lymphknoten | **Gd-Konzentration in [µmol/l]** | |
| | **30 min p.i.** | **90 min p.i.** |
| popliteal | 345 ± 319 | 75 ± 20 |
| inguinal profund | 43 ± 41 | 9 ± 2 |
| iliakal | 50 ± 18 | 20 ± 8 |
| Blut | 24 ± 3 | 25 ± 4 |
| Urin | 261 ± 167 | 348 ± 119 |

Die mit dieser Verbindung erreichten lymphonodalen Akkumulationen übersteigen bei gleicher Dosierung die mit einem extrazellulären Kontrastmittel (Gd-DTPA) erzielten Anreicherungen um den Faktor 5 - 7.

### Beispiel 29

### Retention des kontrastgebenden Metalls am Injektionsort

Nach s.c. Gabe von 10 µmol Gesamtgadolinium (Beispiel 19)/kg Körpergewicht in die Meerschweinchenpfote wurde die Retention des Metalls am Injektionsort zu verschiedenen Zeitpunkten untersucht.

| **Gd-Anreicherung [% Dosis] am Applikationsort (Pfote), nach interstitieller Applikation von 10 µmol/kg (MW ± SD, n=3)** | | | |
|---|---|---|---|
| **Substanz** | **Gadolinium-Konzentration an der Injektionsstelle (Pfote) [% Dosis]** | | |
| | **30 min p.i.** | **90 min p.i.** | **7 d p.i.** |
| Beispiel 19 | 48,4 ± 10,4 % | 8,0 ± 1,7 % | 0,7 ± 0,6 % |

### Beispiel 30

### Ausscheidung des Kontrastmittels nach s.c. Gabe

Nach subkutaner Gabe von 10 µmol Gesamtgadolinium (Beispiel 19) /kg Körpergewicht in die Hinterpfote des Meerschweines wurde 7 Tage nach Applikation die Retention des Metalls in der Leber, in den Nieren sowie in der Milz untersucht.

| **Gd-Anreicherung [% Dosis] in Leber, Nieren, Milz (7 d p.i.) nach interstitieller Applikation von 10 µmol/kg (MW ± SD, n=2)** | | | |
|---|---|---|---|
| **Substanz** | **Gadolinium-Gehalt in den Organen 7 d p.i. [% Dosis]** | | |
| | **Leber** | **Nieren** | **Milz** |
| Beispiel 19 | 0.09 ± 0.09 % | 0.06 ± 0.04 % | 0.00 ± 0.01 % |

### Beispiel 31

### Beispiel für einen in-vivo-Vergleich mit einem intravasalen und einem extrazellulären Kontrastmittel bei abgebundenen Nieren (Diffusibilität) an der Ratte

Die Eignung der im Beispiel 1g beschriebenen Verbindung als blood-pool-agent wird im folgenden Versuch gezeigt.

Als Versuchstiere dienten drei 250 g schwere, männliche (Schering-SPF-) Ratten. Den Tieren wurden die Nierengefäße abgebunden, um eine renale Eliminierung zu verhindern und bei angenommener vernachlässigbarer faekaler Eliminierung ein Maß für Diffusibilität der erfindungsgemäßen Formulierung aus dem Blut in das umgebende Gewebe zu erhalten. Je Tier wurden 0.5 ml (jeweils 10 mmol/L) der folgenden Kontrastmittel-Lösung intravenös appliziert: Gemisch aus je 1 Teil der Verbindung des Beispiels 1g, im folgenden Verbindung 1 genannt, des intravasalen Kontrastmittels Eu-DTPA-Albumin, im folgenden Verbindung 2 genannt, und des extrazellulären Kontrastmittels Dy-DTPA, im folgenden Verbindung 3 genannt. Über einen Katheter in der Arteria carotis communis wurden Blutproben zu folgenden Zeitpunkten entnommen: 15, 30, 45, 60, 90 sec, 3, 5, 10, 15 min p.i. In den gewonnenen Blutproben wurden jeweils parallel die Konzentrationen an Gadolinium (Gd), Europium (Eu) und Dysprosium (Dy) mittels Atomemissionsspektrometrie (ICP-AES) gemessen. Der im Blutraum verbliebene Anteil des intravasalen Kontrastmittels Verbindung 2 (Eu) wurde als 100 % gesetzt. Der im Blutraum verbliebene Anteil der erfindungsgemäßen Formulierung Verbindung 1 (Gd) und der Verbindung 3 (Dy, extrazelluläre Vergleichssubstanz) konnte durch die unterschiedliche Markierung im gleichen Tier verglichen werden und wurde prozentual ins Verhältnis zur Konzentration der Verbindung 2 gesetzt. Damit liefern diese Daten Angaben über den Verbleib der Verbindungen im Intravasalraum.

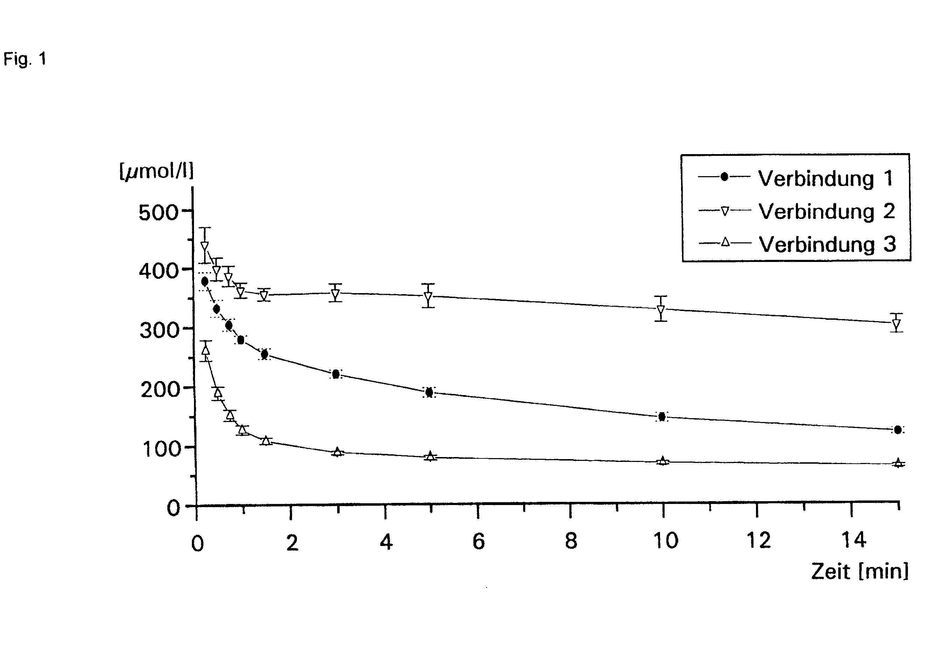

Ergebnisse: Figur 1 zeigt den Blutspiegel (in µmol/l) der erfindungsgemäßen Formulierung im Vergleich zu Eu-DTPA-BSA und Dy-DTPA (jeweils 20 µmol/kg) an Ratten. Die Diffusion der erfindungsgemäßen Formulierung (Verbindung 1) in das Interstitium war im Vergleich zu Eu-DTPA-BSA (Verbindung 2) in der ersten Minute p.i. nur geringfügig schneller, nahm im weiteren Versuchsverlauf jedoch deutlich zu. Im Vergleich zu Dy-DTPA (Verbindung 3) war sie während der gesamten Versuchsdauer langsamer. Vor allem zu den frühen Zeitpunkten wurden deutlich höhere Blutkonzentrationen der Verbindung 1 verglichen mit dem extrazellulären Kontrastmittel (Verbindung 2) erhalten.

Die Diffusibilität der erfindungsgemäßen Formulierung (Verbindung 1) aus dem Vasalraum in das umgebende Gewebe ist zwar größer verglichen zu dem "rein" intravasalen Kontrastmittel (Verbindung 2), jedoch deutlich geringer verglichen zu der frei diffusiblen, extrazellulären Verbindung 3 (siehe Tabelle 2). Dieser Diffusibilitätsversuch verdeutlicht die besondere Eignung der erfindungsgemäßen Formulierung als blood-pool-Kontrastmittel zur diagnostischen Darstellung der Gefäße bzw. pathologischer Gefäßveränderungen (Angiographie).

**Tabelle 2:**

| Prozentsatz der im Blutraum verbliebenen Dosis. | | | |
|---|---|---|---|
| | **30 sec** | **3 min** | **10 min** |
| Eu (Verbindung 2) | 100% | 100% | 100% |
| Gd (Verbindung 1) | 84% | 62% | 44% |
| Dy (Verbindung 3) | 48% | 25% | 21% |

### Beispiel 32

### Bluteliminationskinetik

Die Blut-Eliminationskinetik der Verbindung des Beispiels 1g wurde an Ratten (Han. Wistar, Schering SPF, ≈ 250 g Körpergewicht) untersucht. Hierfür wurde nach einmaliger intravenöser Applikation (via einer Caudalvene) der Substanzen (Dosis: ≈ 100 µmol Metall pro kg Körpergewicht) die Substanzkonzentration im Blut (basierend auf dem Gd- bzw. Dy-Gehalt) über einen Zeitraum bis zu 120 Minuten p. i. mittels ICP-AES bestimmt. Die pharmakokinetischen Parameter: Verteilungsvolumen (Vss), Gesamtclearance (CLtot) und Eliminationshalbwertszeit (tb) wurden mit einem speziellen Computerprogramm (TOPFIT 2.0; Thomae, Schering, Gödecke) berechnet, wobei ein Zwei-Kompartment-Distributionsmodell zu Grunde gelegt wurde.

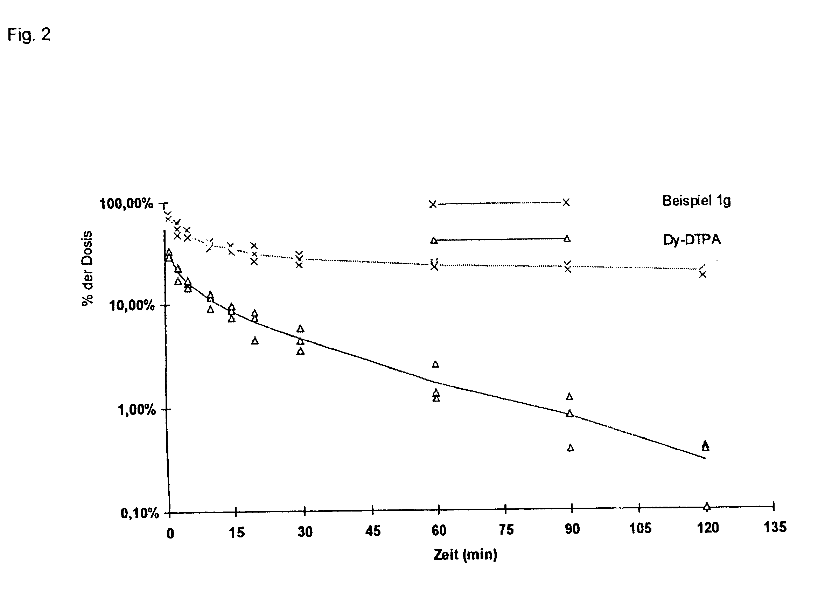

Figur 2 zeigt die Elimination aus dem Blut (in % der injizierten Dosis) von Dy-DTPA (Dosis: 101 µmol Dy pro kg Körpergewicht, n=3) und der Verbindung des Beispiels 1g (Dosis: 96 µmol Gd pro kg Körpergewicht, n=3) nach einmaliger intravenöser Applikation der Substanzen bei Ratten (Han Wistar, Schering SPF, ≈ 250 g Körpergewicht).
Die Gd- und Dy-Gehalte im Blut wurden mittels ICP-AES bestimmt.

Im Vergleich zu Dy-DTPA (dem Dysprosium Analogon von Magnevist®) zeigte die Verbindung des Beispiels 1g eine deutlich langsamere Elimination aus dem Blut und außerdem ein kleineres Verteilungsvolumen. Es ist daher festzustellen, daß diese Verbindung überraschenderweise eine verlängerte Retention im Blutraum hat und daher als "blood pool Kontrastmittel", z. B. zur Darstellung von Blutgefäßen mit geeigneten Techniken, und aufgrund der überraschend hohen Relaxivity (r_{1, Plasma} : ≈19.5 [1*mmol⁻ ¹*s⁻¹]) hier auch in relativ kleinen Dosierungen von ≤ 50 µmol Gd pro kg Körpergewicht geeignet sein dürfte.

**Tabelle 3:**

| Pharmakokinetikparameter: Verteilungsvolumen (Vss), Gesamtclearance (CLtot) und Eliminationshalbwertszeit (tβ) von Dy-DTPA und Beispiel 1g. (Berechnet mit TOPFIT 2.0; Zwei-Kompartmentmodell). | | | | | | | | |
|---|---|---|---|---|---|---|---|---|
| | **Vss (l/kg)** | | **CL tot (ml/(min kg))** | | **t α (min)** | | **t β (min)** | |
| | **MEAN** | **SD** | **MEAN** | **SD** | **MEAN** | **SD** | **MEAN** | **SD** |
| **Dy-DTPA** | 0.17 | 0.03 | 6.75 | 1.31 | 2.07 | 0.46 | 20.7 | 3.9 |
| **Beispiel 1g** | 0.09 | 0.01 | 0.41 | 0.08 | 4.06 | 0.80 | 168.8 | 49.3 |

### Beispiel 33

### Bluteliminationskinetik

Die Blut-Eliminationskinetik der Verbindung des Beispiels 19 wurde an Ratten (Han. Wistar, Schering SPF, ≈ 250 g Körpergewicht) untersucht. Hierfür wurde nach einmaliger intravenöser Applikation (via einer Caudalvene) der Substanzen (Dosis: ≈ 100 µmol Metall pro kg Körpergewicht) die Substanzkonzentration im Blut (basierend auf dem Metallgehalt) über einen Zeitraum bis zu 120 Minuten p. i. mittels ICP-AES bestimmt. Die pharmakokinetischen Parameter: Verteilungsvolumen (Vss), Gesamtclearance (CLtot) und Eliminationshalbwertszeit (tb) wurden mit einem speziellen Computerprogramm (TOPFIT 2.0; Thomae, Schering, Gödecke) berechnet, wobei ein Zwei-Kompartment-Distributionsmodell zu Grunde gelegt wurde.

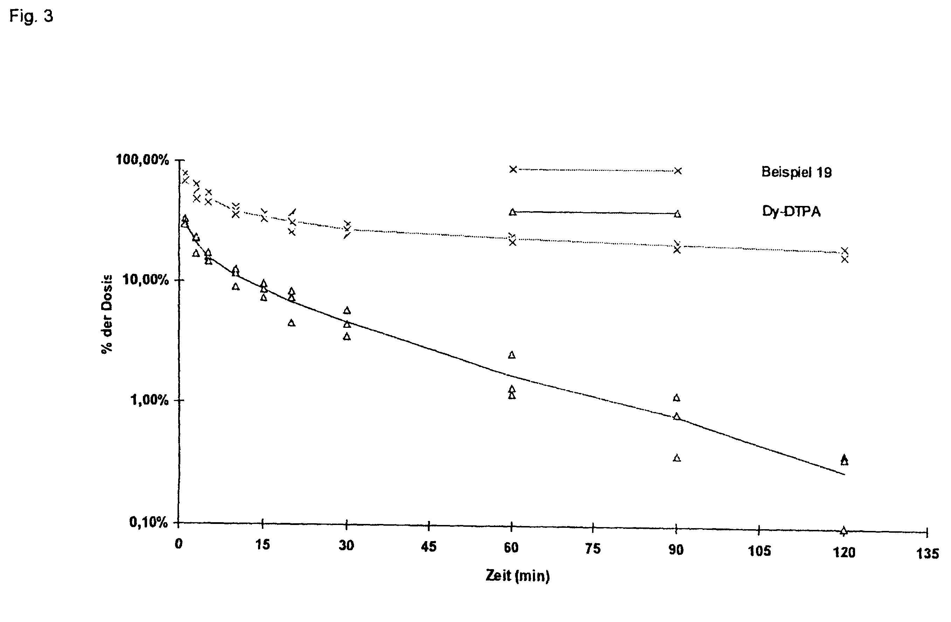

Figur 3 zeigt die Elimination aus dem Blut (in % der injizierten Dosis) von Dy-DTPA (Dosis: 101 µmol Dy pro kg Körpergewicht, n=3) und der Verbindung des Beispiels 19 (Dosis: 96 µmol Gd pro kg Körpergewicht, n=3) nach einmaliger intravenöser Applikation der Substanzen bei Ratten (Han Wistar, Schering SPF, ≈ 250 g Körpergewicht).
Die Gd- und Dy-Gehalte im Blut wurden mittels ICP-AES bestimmt.
Im Vergleich zu Dy-DTPA (dem Dysprosium Analogon von Magnevist®) zeigte die Verbindung des Beispiels 19 eine deutlich langsamere Elimination aus dem Blut und außerdem ein kleineres Verteilungsvolumen. Es ist daher festzustellen, daß diese Verbindung überraschenderweise eine verlängerte Retention im Blutraum hat und daher als "blood pool Kontrastmittel", z. B. zur Darstellung von Blutgefäßen mit geeigneten Techniken, und aufgrund der überraschend hohen Relaxivity (r₁, _{Plasma}: ≈19.5 [1*mmol⁻ ¹*s⁻¹]) hier auch in relativ kleinen Dosierungen von ≤ 50 µmol Gd pro kg Körpergewicht geeignet sein dürfte.

**Tabelle 4:**

| Pharmakokinetikparameter: Verteilungsvolumen (Vss), Gesamtclearance (CLtot) und Eliminationshalbwertszeit (tβ) von Dy-DTPA und von Beispiel 1g. (Berechnet mit TOPFIT 2.0; Zwei-Kompartmentmodell). | | | | | | | | |
|---|---|---|---|---|---|---|---|---|
| | **Vss** ^{**(l/kg)**} | | **CL tot** ^{**(ml/(min·kg))**} | | **t α** ^{**(min)**} | | **t β** ^{**(min)**} | |
| | **MEAN** | **SD** | **MEAN** | **SD** | **MEAN** | **SD** | **MEAN** | **SD** |
| **Dy-DTPA** | 0.17 | 0.01 | 6.90 | 0.31 | 1.48 | 0.27 | 18.5 | 0.6 |
| **Beispiel 19** | 0.12 | 0.01 | 0.94 | 0.06 | 2.52 | 0.29 | 89.2 | 9.6 |

### Beispiel 34

### Kontrastmittelgestützte MR Angiographie

Die Untersuchungen zur kontrastmittelgetützten MR Angiographie wurden an einem experimentellen MRT System (SISCO SIS 85, 2.0 Tesla) mit einer 3 D FLASH Technik (10/2.6/40°) durchgeführt.
Um die Wirksamkeit von Kontrastmitteln zur Darstellung von Blutgefäßen zu bestimmen, wurden narkotisierte (Rompun® + Ketavet®,1 + 1, v/v; ≈ 1 ml pro kg Körpergewicht, i. m.) Ratten (Han. Wistar; ≈ 200 g Körpergewicht) MR-tomographisch untersucht.

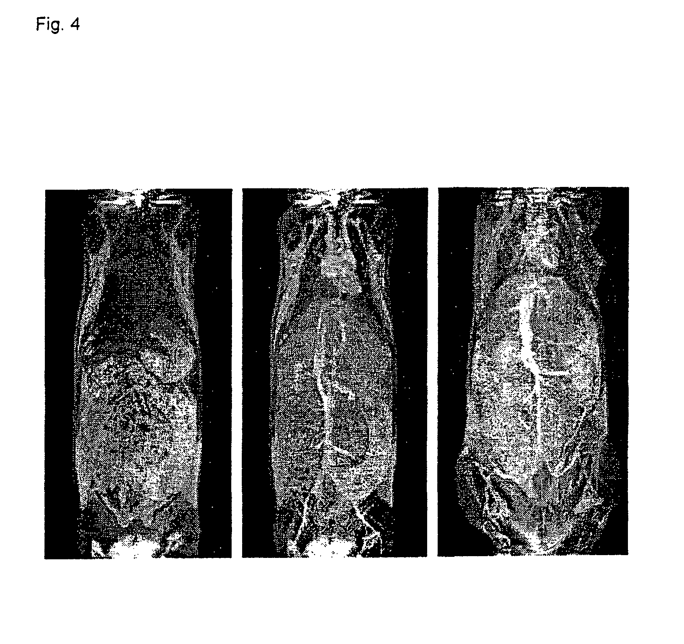

Figur 4 zeigt Abbildungen dieses Experiments. Die MIP (maximal intensity projection)-Bilder wurden berechnet aus den 3 D FLASH Experimenten vor (links) und direkt nach einmaliger i. v. Applikation von 50 µmol / kg (mitte) bzw. 100 µmol /kg (rechts) der Substanz aus Beispiel 1 g.

Vor Kontrastmittelapplikation waren keinerlei Blutgefäße zu erkennen (links). Nach einmaliger intravenöser Applikation war ein ausgeprägter Signalanstieg in den Blutgefäßen und eine deutliche Kontrastierung zum Normalgewebe zu erkennen (mitte und rechts).

Es konnte ein ausgeprägter Signalanstieg in den Blutgefäßen nach der Applikation festgestellt werden, der die Darstellung einer Vielzahl von Blutgefäßen ermöglichte.

## Patentansprüche

1. Oligomere, perfluoralkylhaltige Verbindungen der allgemeinen Formel I
A-R^{F} (I)
worin
• A ein Molekülteil ist, der 2 - 6 Metallkomplexe enthält, die direkt oder über einen Linker an ein Stickstoffatom einer ringförmigen Gerüstkette gebunden sind
und
• R^{F} eine perfluorierte, geradkettige oder verzweigte Kohlenstoffkette mit der Formel -CₙF₂ₙE ist, in der
E ein endständiges Fluor-, Chlor-, Brom-, Jod- oder Wasserstoffatom darstellt und n fiir die Zahlen 4 - 30 steht,
**dadurch gekennzeichnet,**
**daß** das Molekülteil A die folgende Struktur aufweist: 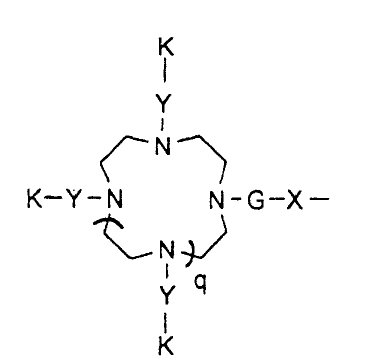 wobei
• q eine Zahl 0,1,2 oder 3 ist,
• K für einen Komplexbildner oder Metallkomplex oder deren Salze organischer und/oder anorganischer Basen oder Aminosäuren oder Aminosäureamide steht,
• X eine direkte Bindung zur Perfluoralkylgruppe, eine Phenylengruppe oder eine C₁-C₁₀-Alkylenkette ist, die gegebenenfalls 1 - 15 Sauerstoff-, 1 - 5 Schwefelatome, 1 - 10 Carbonyl-, 1 - 10 (NR)-, 1 - 2 NRSO₂-, 1 - 10 CONR-. 1 Piperidin-, 1 - 3 SO₂-, 1 - 2 Phenylengruppen enthält oder gegebenenfalls durch 1 - 3 Reste R^{F} substituiert ist, worin R für ein Wasserstoffatom, eine Phenyl-, Benzyl- oder eine C₁-C₁₅-Alkylgruppe steht, die gegebenenfalls 1 - 2 NHCO-, 1 - 2 CO-Gruppen, 1 - 5 Sauerstoffatome enthält und gegebenenfalls durch 1 - 5 Hydroxy-, 1 - 5 Methoxy-, 1 - 3 Carboxy-, 1 - 3 R^{F}-Reste substituiert ist
• Y eine direkte Bindung oder eine Kette der allgemeinen Formel II oder III ist: 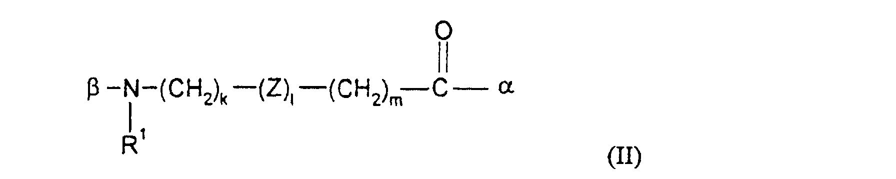 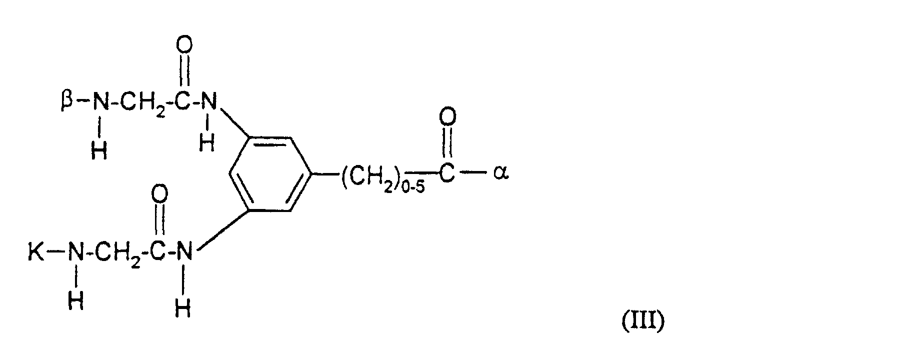 worin
■ R¹ ein Wasserstoffatom, eine Phenylgruppe, eine Benzylgruppe oder eine C₁-C₇ Alkylgruppe ist, die gegebenenfalls substituiert ist mit einer Carboxy-, einer Methoxy- oder einer Hydroxygruppe,
■ Z eine direkte Bindung, eine Polyglycolethergruppe mit bis zu 5 Glycoleinheiten oder ein
Molekülteil der allgemeinen Formel IV ist
―CH(R²)- (IV)
worin R² eine C₁-C₇-Carbonsäure, eine Phenylgruppe, eine Benzylgruppe oder eine -(CH₂)₁₋₅-NH-K-Gruppe ist,
■ α die Bindung an das Stickstoffatom der Gerüstkette, β die Bindung zum Komplexbildner oder Metallkomplex K darstellt,
■ und in der die Variablen k und m für natürliche Zahlen zwischen 0 und 10 und 1 für 0 oder 1 stehen,
und wobei
• G eine CO- oder SO₂-Gruppe ist.

2. Verbindungen gemäß Anspruch 1,
**dadurch gekennzeichnet,**
**daß** q die Zahl 1 ist.

3. Verbindungen gemäß Anspruch 1,
**dadurch gekennzeichnet,**
**daß** der Molekülteil X eine Alkylenkette ist, die 1 - 10 CH₂CH₂O- oder 1 - 5 COCH₂NH-Gruppen enthält.

4. Verbindungen gemäß Anspruch 1,
**dadurch gekennzeichnet,**
**daß** der Molekülteil X eine direkte Bindung ist oder eine der folgenden Strukturen aufweist: 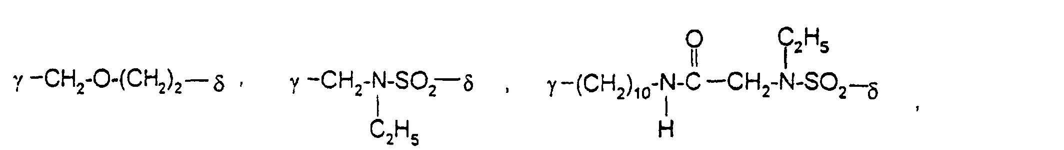
γ-(CH₂)₁₀-O-(CH₂)₂―δ , 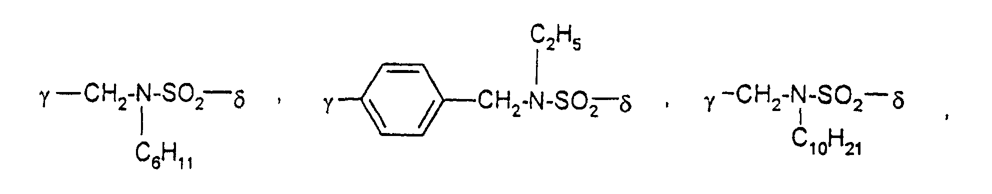 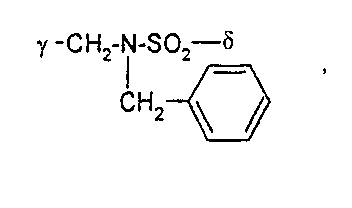
wobei
γ an G und δ an R^{F} bindet.

5. Verbindungen gemäß Anspruch 1,
**dadurch gekennzeichnet,**
**daß** Y ein Molekülteil mit einer der folgenden Strukturen ist:

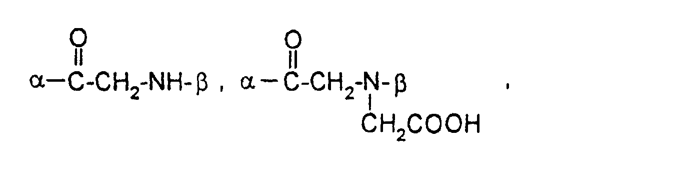

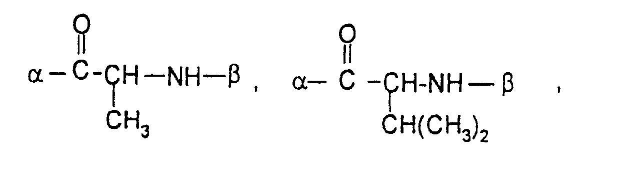

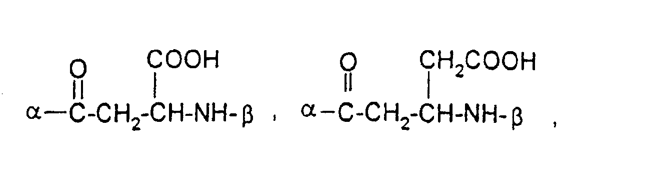

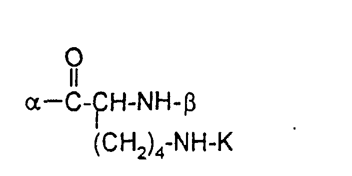

6. Verbindungen gemäß Anspruch 1,
**dadurch gekennzeichnet,**
**daß** K einen Komplex der allgemeinen Formel V, VI, VII oder VIII darstellt, 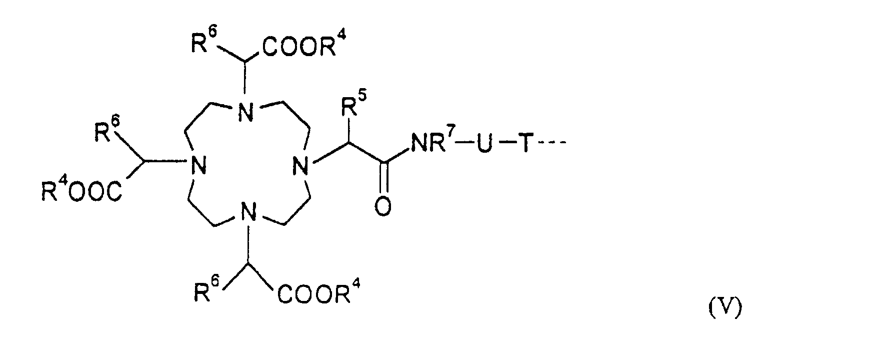 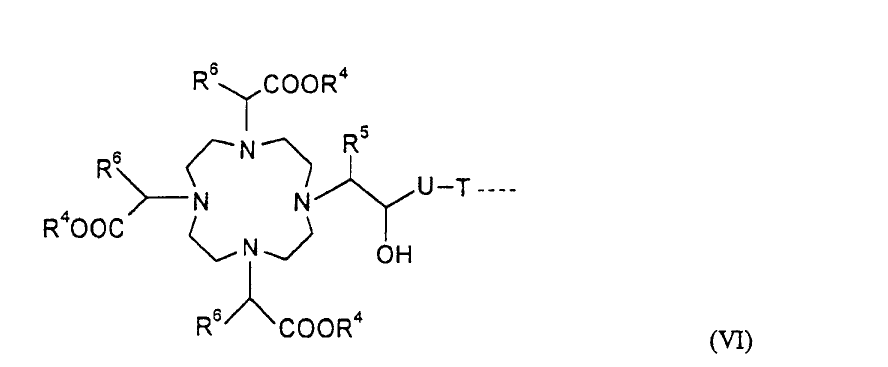 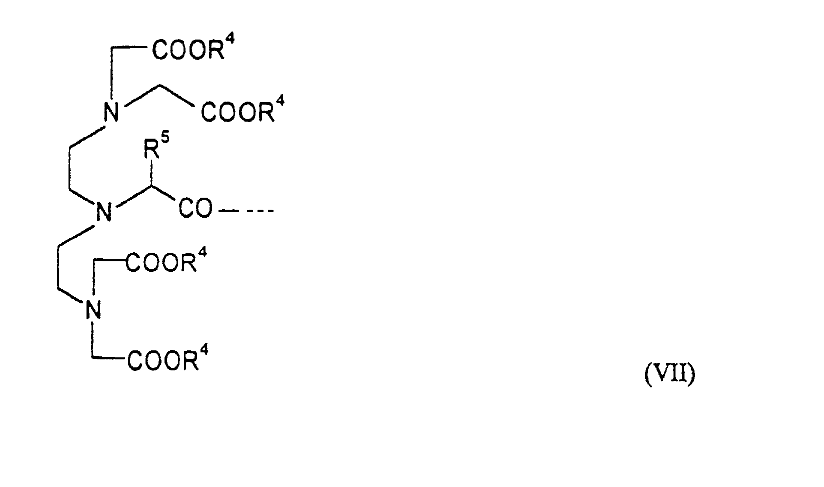 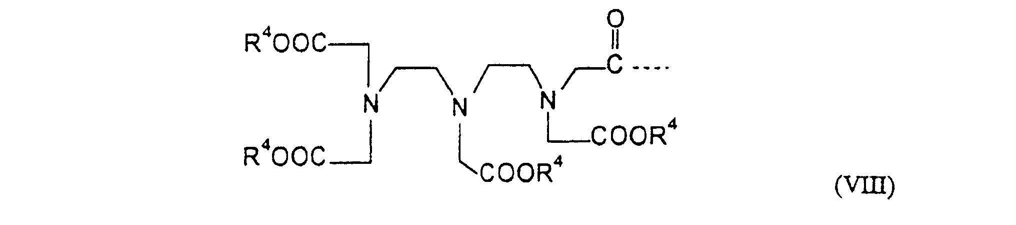 wobei
• R⁴ unabhängig voneinander ein Wasserstoffatom oder ein Metallionenäquivalent der Elemente der Ordnungszahlen 20 - 32, 37 - 39, 42 - 44, 49 oder 57 - 83 ist,
• R⁵ ein Wasserstoffatom oder eine geradkettige, verzweigte, gesättigte oder ungesättigte C₁-C₃₀-Alkylkette ist, die gegebenenfalls substituiert ist durch 1 -5 Hydroxy-, 1 - 3 Carboxy- oder 1 Phenylgruppe(n) und/oder gegebenenfalls durch 1 - 10 Sauerstoffatome, 1 Phenylen- oder 1 Phenylenoxygruppe unterbrochen ist
• R⁶ ein Wasserstoffatom, ein geradkettiger oder verzweigter C₁-C₇-Alkylrest, ein Phenyl- oder Benzylrest ist,
• R⁷ ein Wasserstoffatom, eine Methyl- oder Ethylgruppe ist, die gegebenenfalls substituiert ist durch eine Hydroxy- oder Carboxygruppe,
• U eine gegebenenfalls 1 - 5 Imino-, 1 - 3 Phenylen-, 1 - 3 Phenylenoxy-, 1 - 3 Phenylenimino-, 1 - 5 Amid-, 1 - 2 Hydrazid-, 1 - 5 Carbonyl-, 1 - 5 Ethylenoxy-, 1 Harnstoff-, 1 Thioharnstoff-, 1 - 2 Carboxyalkylimino-, 1 - 2 Estergruppen, 1 - 10 Sauerstoff-, 1 - 5 Schwefel- und/oder 1 - 5 Stickstoffatome enthaltende und/oder gegebenenfalls durch 1 - 5 Hydroxy-, 1 - 2 Mercapto-, 1 - 5 Oxo-, 1 - 5 Thioxo-, 1 - 3 Carboxy-, 1 - 5 Carboxyalkyl-, 1 - 5 Ester- und/oder 1 - 3 Aminogruppen substituierte, geradkettige, verzweigte, gesättigte oder ungesättigte C₁-C₂₀-Alkylengruppe ist, wobei die gegebenenfalls enthaltenen Phenylengruppen durch 1 - 2 Carboxy-, 1 - 2 Sulfon- oder 1 - 2 Hydroxygruppen substituiert sein können
• T für eine -CO-β, -NHCO-β oder -NHCS-β-Gruppe steht, wobei β die Bindungsstelle an Y darstellt.

7. Verbindungen gemäß Anspruch 6,
**dadurch gekennzeichnet,**
**daß** die für U stehende C₁-C₂₀-Alkylenkette die Gruppen
-CH₂NHCO-, -NHCOCH₂O-, -NHCOCH₂OC₆H₄-, -N(CH₂CO₂H)-,
-CH₂OCH₂-,
-NHCOCH₂C₆H₄-, -NHCSNHC₆H₄-, -CH₂OC₆H₄-, -CH₂CH₂Oenthält und/oder durch die Gruppen -COOH, -CH₂COOH substituiert ist.

8. Verbindungen gemäß Anspruch 6,
**dadurch gekennzeichnet,**
**daß** U fiir eine
-CH₂-, -CH₂CH₂-, -CH₂CH₂CH₂-, -C₆H₄-, -C₆H₁₀-, -CH₂C₆H₄-,
-CH₂NHCOCH₂CH(CH₂CO₂H)-C₆H₄-,
-CH₂NHCOCH₂OCH₂-,
-CH₂NHCOCH₂C₆H₄-gruppe steht.

9. Verbindungen gemäß Anspruch 1,
**dadurch gekennzeichnet,**
**daß** K eine der folgenden Strukturen aufweist:

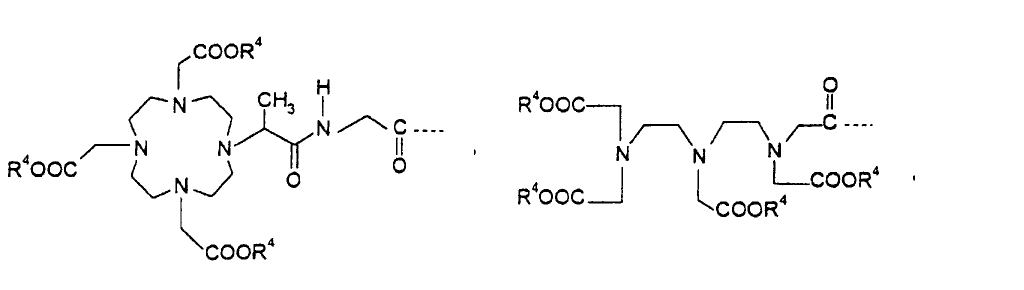

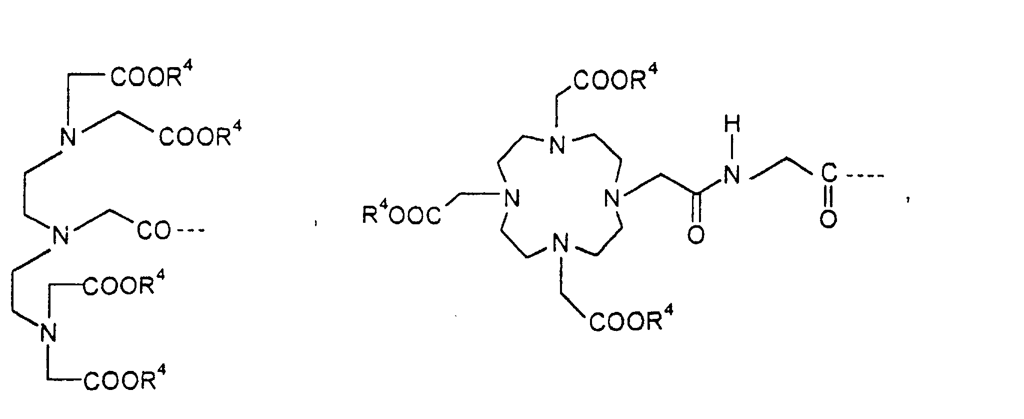

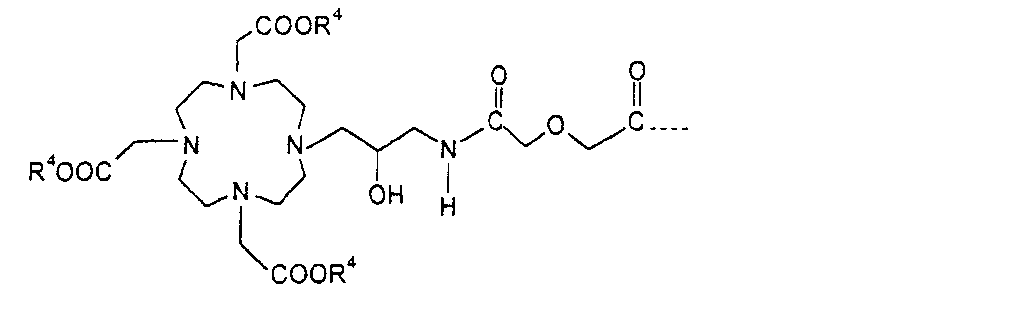

10. Verbindungen gemäß einem der Ansprüche 1 bis 9,
**dadurch gekennzeichnet,**
**daß** die Perfluoralkylkette R^{F} -C₆F₁₃, -C₈F₁₇, -C₁₀F₂₁ oder -C₁₂F₂₅ ist.

11. Verfahren zur Herstellung von perfluorhaltigen Verbindungen der allgemeinen Formel I,
**dadurch gekennzeichnet,**
**daß** man Verbindungen der allgemeinen Formel I'
A'-R^{F} (I')
worin A' für einen Rest 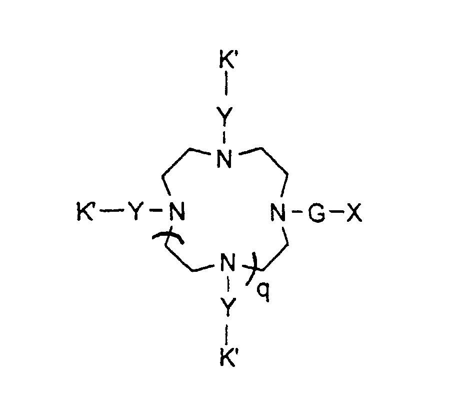 steht und K' für K steht mit R⁴ in der Bedeutung eines Wasserstoffatoms oder einer Carboxyschutzgruppe,
nach Abspaltung der gegebenenfalls vorhandenen Schutzgruppen in an sich bekannter Weise mit mindestens einem Metalloxid oder Metallsalz eines Elementes der Ordnungszahlen 20 - 32, 37 - 39, 42 - 44, 49 oder 57 - 83 umsetzt und gegebenenfalls anschließend in den so erhaltenen Komplexverbindungen noch vorhandene acide Wasserstoffatome ganz oder teilweise durch Kationen von anorganischen und/oder organischen Basen, Aminosäuren oder Aminosäureamiden substituiert.

12. Verfahren zur Herstellung von perfluoralkylhaltigen Verbindungen der allgemeinen Formel I,
**dadurch gekennzeichnet,**
**daß** man eine Verbindung der allgemeinen Formel I" 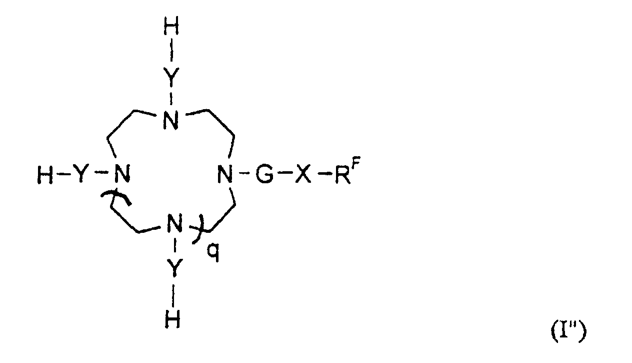 mit einem Komplex V' oder VI' umsetzt, 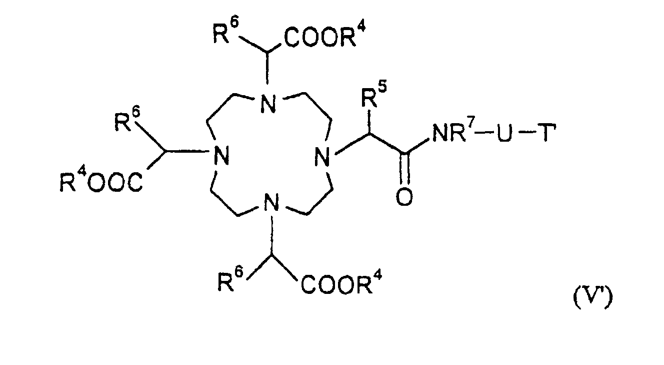 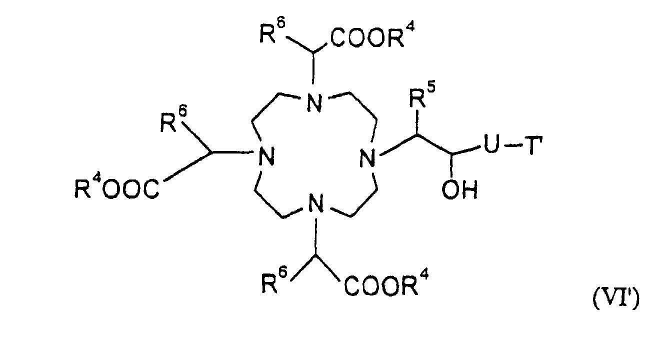 wobei T' für eine -C*O-, -COOH, -N=C=O- oder -N=C=S-Gruppe und -C*O fiir eine aktivierte Carboxylgruppe steht,
mit der Maßgabe, daß mindestens zwei (bei zweiwertigen Metallen) bzw. drei (bei dreiwertigen Metallen) der Substituenten R⁴ für ein Metallionenäquivalent der oben genannten Elemente stehen und daß gewünschtenfalls weitere Carboxylgruppen in Form ihrer Salze mit anorganischen und/oder organischen Basen, Aminosäuren oder Aminosäureamiden vorliegen.

13. Pharmazeutische Mittel enthaltend mindestens eine physiologisch verträgliche Verbindung gemäß Anspruch 1, gegebenenfalls mit den in der Galenik üblichen Zusätzen.

14. Verwendung von mindestens einer physiologisch verträglichen Verbindung gemäß Anspruch 1 oder eines pharmazeutischen Mittels gemäß Anspruch 13 zur Herstellung von Kontrastmitteln in der ¹H-NMR-Diagnostik und -Spektroskopie.

15. Verwendung von mindestens einer physiologisch verträglichen Verbindung gemäß Anspruch 1 oder eines pharmazeutischen Mittels gemäß Anspruch 13 zur Herstellung von Kontrastmitteln in der Röntgendiagnostik.

16. Verwendung von mindestens einer physiologisch verträglichen Verbindung gemäß Anspruch 1 oder eines pharmazeutischen Mittels gemäß Anspruch 13 zur Herstellung von pharmazeutisches Mitteln für Radio-Diagnostik und -Therapie.

17. Verwendung gemäß Anspruch 14 oder 15 zur Herstellung von blood-pool-agents.

18. Verwendung gemäß Anspruch 14 oder 15 zur Herstellung von Lymphographika.

19. Verfahren zur Herstellung der pharmazeutischen Mittel gemäß Anspruch 13,
**dadurch gekennzeichnet, daß** man die in Wasser oder physiologischer Salzlösung vorliegenden perfluoralkylhaltigen Verbindungen, gegebenenfalls mit den in der Galenik üblichen Zusätzen, in eine für die enterale oder parenterale Applikation geeignete Form bringt.

## Claims

1. Oligomeric compounds that contain perfluoroalkyl of general formula I:
A-R^{F} (I)
in which
• A is a molecule portion that contains 2-6 metal complexes that are connected directly or via a linker to a nitrogen atom of an annular skeleton chain,
and
• R^{F} is a perfluorinated, straight-chain or branched carbon chain with formula -CₙF₂ₙE, in which E represents a terminal fluorine, chlorine, bromine, iodine, or hydrogen atom and n stands for numbers 4-30,
**characterized in that** molecule portion A has the following structure: 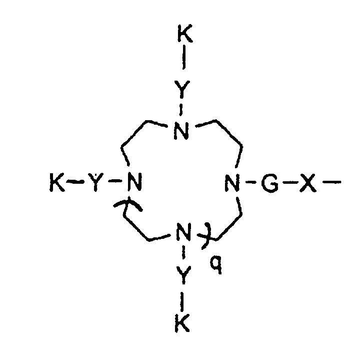 whereby
• q is a number 0, 1, 2 or 3,
• K stands for a complexing agent or metal complex or their salts of organic and/or inorganic bases or amino acids or amino acid amides,
• X is a direct bond to the perfluoroalkyl group, a phenylene group, or a C₁-C₁₀ alkylene chain, which optionally contains 1-15 oxygen atoms, 1-5 sulfur atoms, 1-10 carbonyl groups, 1-10 (NR) groups, 1-2 NRSO₂ groups, 1-10 CONR groups, 1 piperidine group, 1-3 SO₂ groups, 1-2 phenylene groups or optionally is substituted by 1-3 radicals R^{F}, in which R stands for a hydrogen atom, a phenyl, benzyl or a C₁-C₁₅ alkyl group, which optionally contains 1-2 NHCO groups, 1-2 CO groups, 1-5 oxygen atoms, and optionally is substituted by 1-5 hydroxy radicals, 1-5 methoxy radicals, 1-3 carboxy radicals, 1-3 R^{F} radicals,
• Y is a direct bond or a chain of general formula II or III: 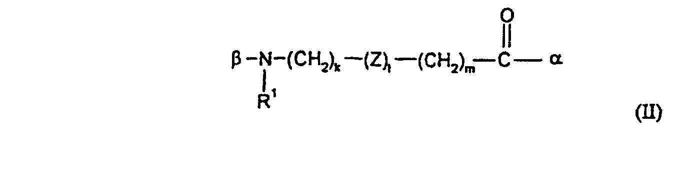 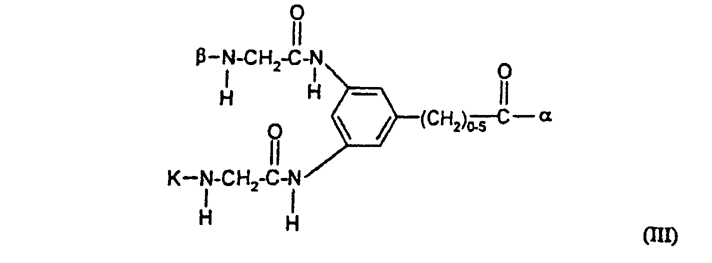 in which
■ R¹ is a hydrogen atom, a phenyl group, a benzyl group or a C₁-C₇ alkyl group, which optionally is substituted with a carboxy group, a methoxy group, or a hydroxy group,
■ Z is a direct bond, a polyglycol ether group with up to 5 glycol units, or a molecule portion of general formula IV
-CH(R²)- (IV)
in which R² is a C₁-C₇ carboxylic acid, a phenyl group, a benzyl group, or a -(CH₂)₁₋₅-NH-K group,
■ α represents the bond to the nitrogen atom of the skeleton chain, and β is the bond to the complexing agent or metal complex K,
■ and in which variables k and m stand for natural numbers between 0 and 10 and 1 stands for 0 or 1,
and whereby
• G is a CO or SO₂ group.

2. Compounds according to claim 1, **characterized in that** q is the number 1.

3. Compounds according to claim 1, **characterized in that** molecule portion X is an alkylene chain, which contains 1-10
CH₂CH₂O groups or 1-5 COCH₂NH groups.

4. Compounds according to claim 1, **characterized in that** molecule portion X is a direct bond or has one of the following structures: 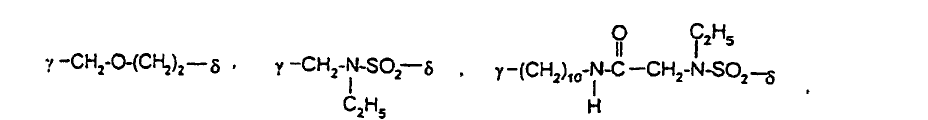
γ-(CH₂)₁₀-O-(CH₂)₂-δ , 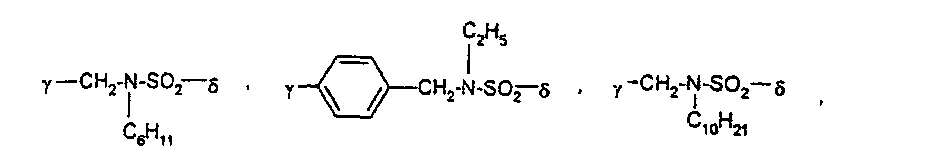 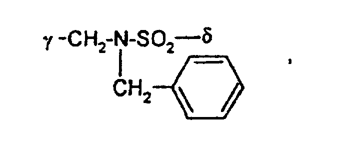
whereby γ binds to G and δ binds to R^{F}.

5. Compounds according to the claim 1, **characterized in that** Y is a molecule portion with one of the following structures:

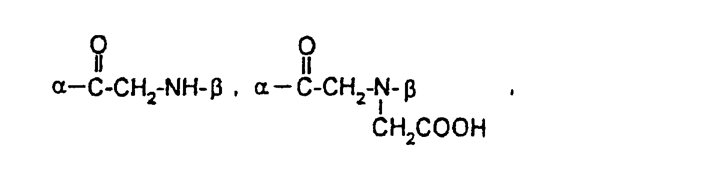

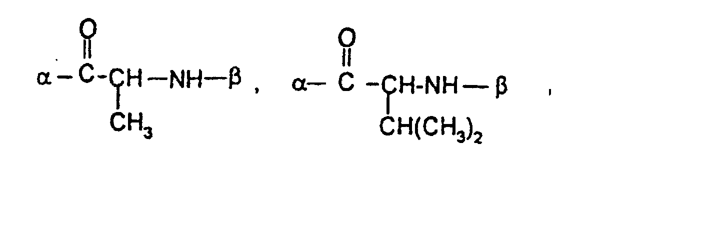

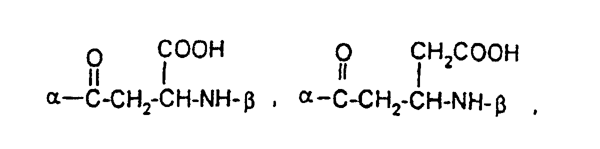

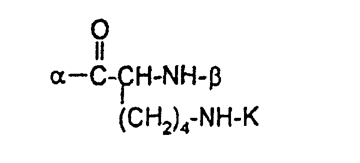

6. Compounds according to claim 1, **characterized in that** K represents a complex of general formula V, VI, VII or VIII, 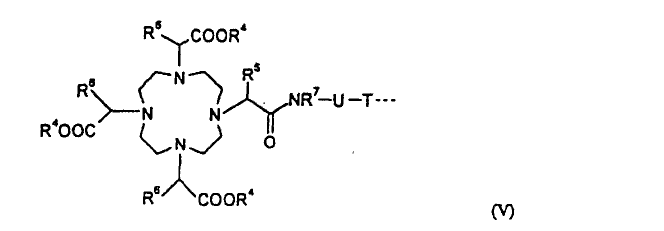 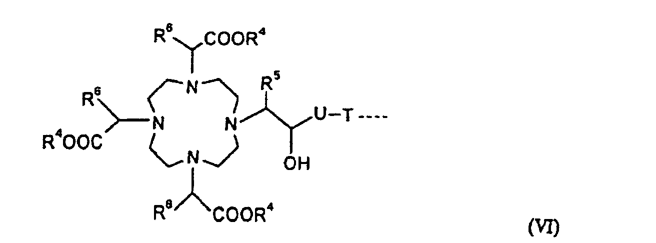 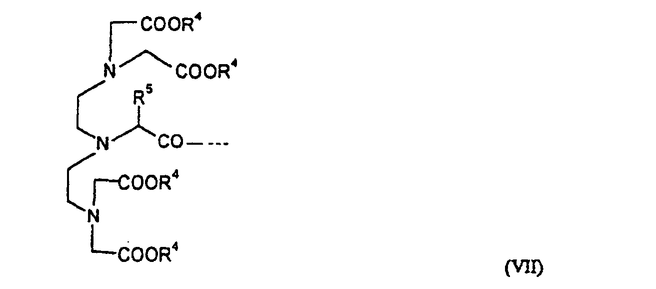 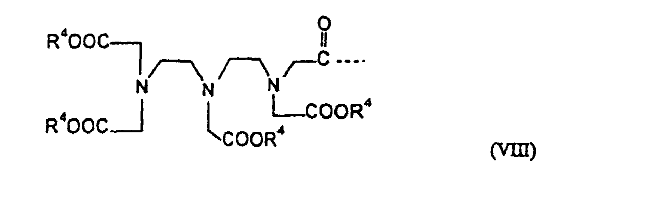 whereby
• R⁴, independently of one another, are a hydrogen atom or a metal ion equivalent of the elements of atomic numbers 20-32, 37-39, 42-44, 49 or 57-83,
• R⁵ is a hydrogen atom or a straight-chain, branched, saturated or unsaturated C₁-C₃₀ alkyl chain, which optionally is substituted by 1-5 hydroxy groups, 1-3 carboxy groups or 1 phenyl group and/or optionally is interrupted by 1-10 oxygen atoms, 1 phenylene group or 1 phenylenoxy group,
• R⁶ is a hydrogen atom, a straight-chain or branched C₁-C₇ alkyl radical, a phenyl or benzyl radical,
• R⁷ is a hydrogen atom, a methyl or ethyl group, which optionally is substituted by a hydroxy or carboxy group,
• U is a straight-chain, branched, saturated or unsaturated C₁-C₂₀ alkylene group that optionally contains 1-5 imino, 1-3 phenylene, 1-3 phenylenoxy, 1-3 phenylenimino, 1-5 amide, 1-2 hydrazide, 1-5 carbonyl, 1-5 ethylenoxy, 1 urea, 1 thiourea, 1-2 carboxyalkylimino, 1-2 ester groups, 1-10 oxygen, 1-5 sulfur and/or 1-5 nitrogen atoms, and/or optionally is substituted by 1-5 hydroxy, 1-2 mercapto, 1-5 oxo, 1-5 thioxo, 1-3 carboxy, 1-5 carboxyalkyl, 1-5 ester and/or 1-3 amino groups, whereby the optionally contained phenylene groups can be substituted by 1-2 carboxy, 1-2 sulfone or 1-2 hydroxy groups,
T stands for a -CO-β, -NHCO-β or -NHCS-β group, whereby β represents the binding site to Y.

7. Compounds according to claim 6, **characterized in that** the C₁-C₂₀ alkylene chain that stands for U contains the groups -CH₂NHCO-, -NHCOCH₂O-, -NHCOCH₂OC₆H₄-, -N(CH₂CO₂H)-, -CH₂OCH₂-, -NHCOCH₂C₆H₄-, -NHCSNHC₆H₄-,-CH₂OC₆H₄-, -CH₂CH₂O-, and/or is substituted by the groups -COOH, -CH₂COOH.

8. Compounds according to claim 6, **characterized in that** U stands for a -CH₂, -CH₂CH₂, -CH₂CH₂CH₂, -C₆H₄, -C₆H₁₀, -CH₂C₆H₄, -CH₂NHCOCH₂CH(CH₂CO₂H)-C₆H₄-, -CH₂NHCOCH₂-OCH₂, -CH₂NHCOCH₂C₆H₄ group.

9. Compounds according to claim 1, **characterized in that** K has one of the following structures:

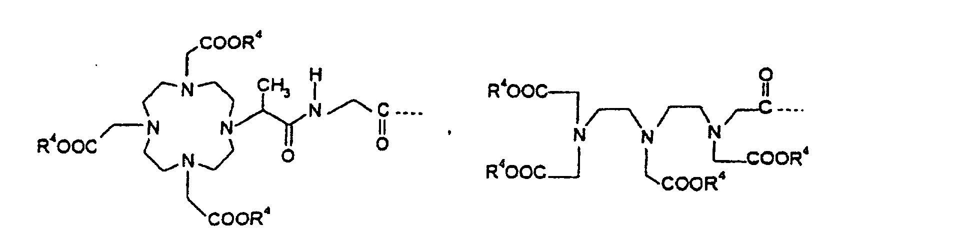

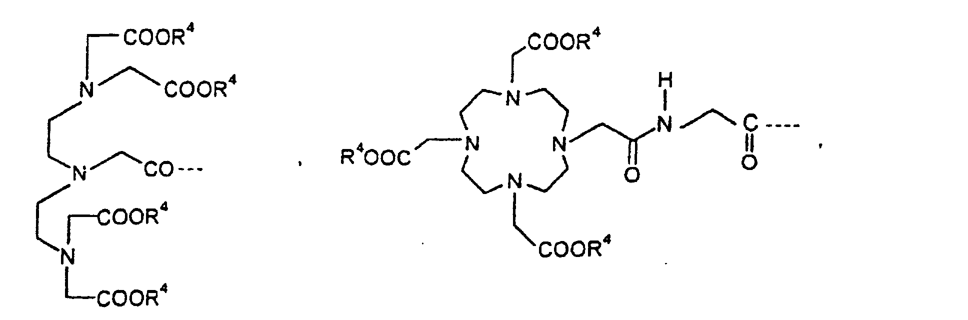

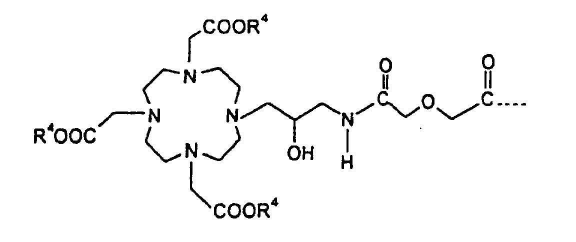

10. Compounds according to any one of claims 1 to 9, **characterized in that** the perfluoroalkyl chain R^{F} is -C₆F₁₃, -C₈F₁₇, -C₁₀F₂₁ or -C₁₂F₂₅.

11. Process for the production of compounds that contain perfluoroalkyl of general formula I, **characterized in that** compounds of general formula I'
A'-R^{F} (I')
in which A' stands for a radical 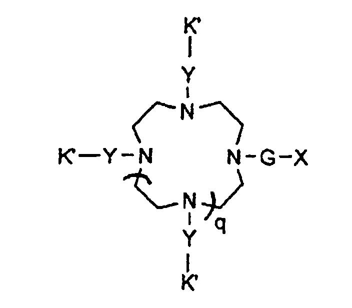 and K' stands for K with R⁴ in the meaning of a hydrogen atom or a carboxy protective group,
are reacted after cleavage of the optionally present protective groups in a way that is known in the art with at least one metal oxide or metal salt of an element of atomic numbers 20-32, 37-39, 42-44, 49 or 57-83 and optionally then still present acidic hydrogen atoms in the complex compounds that are thus obtained are substituted completely or partially by cations of inorganic and/or organic bases, amino acids or amino acid amides.

12. Process for the production of compounds that contain perfluoroalkyl of general formula I, **characterized in that** a compound of general formula I" 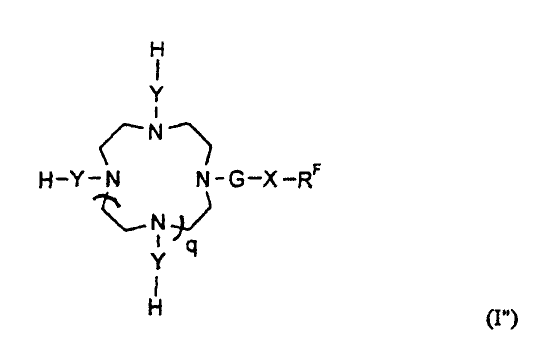 is reacted with a complex V' or VI', 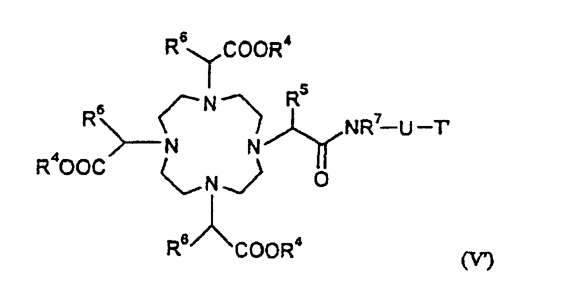 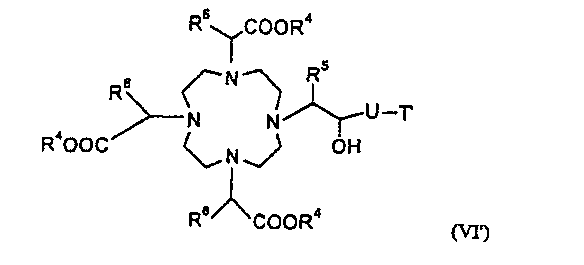 whereby T' stands for a -C*O, -COOH, -N=C=O or -N=C=S group and -C*O stands for an activated carboxyl group, provided that at least two (in the case of divalent metals) or three (in the case of trivalent metals) of substituents R⁴ stand for a metal ion equivalent of the above-mentioned elements, and that optionally other carboxyl groups are present in the form of their salts with inorganic and/or organic bases, amino acids or amino acid amides.

13. Pharmaceutical agents that contain at least one physiologically compatible compound according to claim 1, optionally with the additives that are commonly used in galenicals.

14. Use of at least one physiologically compatible compound according to claim 1 or of a pharmaceutical agent according to claim 13 for producing contrast agents in ¹H NMR diagnosis and ¹H NMR spectroscopy.

15. Use of at least one physiologically compatible compound according to claim 1 or of a pharmaceutical agent according to claim 13 for producing contrast agents in diagnostic radiology.

16. Use of at least one physiologically compatible compound according to claim 1 or of a pharmaceutical agent according to claim 13 for producing pharmaceutical compositions for radiodiagnosis and radiotherapy.

17. Use according to claim 14 or 15 for producing blood pool agents.

18. Use according to claim 14 or 15 for producing lymphographic agents.

19. Process for the production of pharmaceutical agents according to claim 13, **characterized in that** the compounds that contain perfluoroalkyl and that are present in water or physiological salt solution, are brought into a form that is suitable for enteral or parenteral administration optionally with the additives that are commonly used in galenicals.

## Revendications

1. Composés oligomères perfluoralkylés de formule générale I
A-R^{F} (I)
dans laquelle
• A représente une partie de molécule contenant 2-6 complexes métalliques liés directement ou via un lieur à un atome d'azote d'une chaîne de squelette en forme de noyau
et
• R^{F} est une chaîne carbonée perfluorée, linéaire ou ramifiée, de formule -CₙF₂ₙE, dans laquelle E représente un atome terminal de fluor, de chrome, de brome, d'iode ou d'hydrogène, et n représente les nombres de 4 à 30,
**caractérisés en ce que**
la partie de molécule A présente la structure suivante: 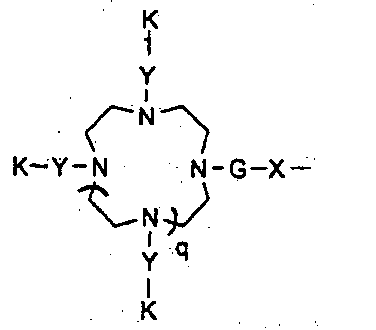 dans laquelle
• q représente un nombre 0, 1, 2 ou 3,
• K représente un complexant ou un complexe métallique
ou ses sels de bases organiques et/ou inorganiques ou d'acides aminés ou d'amides d'acides aminés,
• X représente une liaison directe avec le groupe perfluoralkyle, un groupe phénylène ou une chaîne alkylène en C₁ à C₁₀, qui contient éventuellement 1 à 15 atomes d'oxygène, 1 à 5 atomes de soufre, ou 1 à 10 groupes carbonyle, 1 à 10 groupes (NR)-, 1 à 2 groupes NRSO₂-, 1 à 10 groupes CONR-, 1 groupe pipéridine, 1 à 3 groupes SO₂-, 1 à 2 groupes phénylène, ou est éventuellement substituée par 1 à 3 restes R^{F}, où R représente un atome d'hydrogène ou un groupe phényle, benzyle ou alkyle en C₁ à C₁₅, qui contient éventuellement 1 à 2 groupes NHCO-, 1 à 2 groupes CO-, 1 à 5 atomes d'oxygène et est éventuellement substitué par 1 à 5 restes hydroxy, 1 à 5 restes méthoxy, 1 à 3 restes carboxy, 1 à 3 restes R^{F},
• Y représente une liaison directe ou une chaîne des formules générales II ou III: 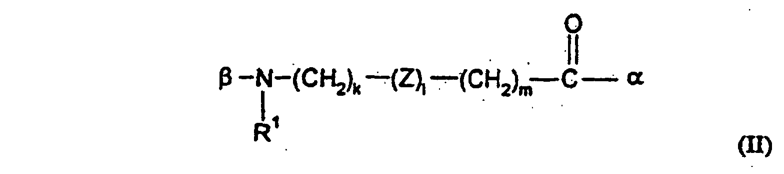 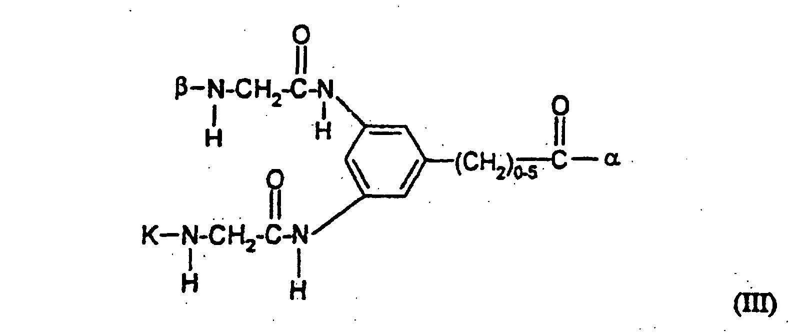
dans lesquelles
• R¹ représente un atome d'hydrogène, un groupe phényle, un groupe benzyle ou un groupe alkyle en C₁ à C₇, qui est éventuellement substitué par un groupe carboxy, méthoxy ou hydroxy,
• Z représente une liaison directe, un groupe polyglycoléther ayant jusqu'à 5 unités glycol ou une partie de molécule de formule générale IV
-CH(R²)- (IV)
dans laquelle R² représente un acide carboxylique en C₁ à C₇, un groupe phényle, un groupe benzyle ou un groupe -(CH₂)₁₋₅-NH-K,
• α représente la liaison à l'atome d'azote de la chaîne de squelette, β la liaison au complexant ou au complexe métallique K,
• et les variables k et m représentent des nombres naturels entre 0 et 10 et 1 a une valeur de 0 ou 1, et dans laquelle
• G représente un groupe CO- ou SO₂-.

2. Composés selon la revendication 1, **caractérisés en ce que** q représente le nombre 1.

3. Composés selon la revendication 1, **caractérisés en ce que** la partie de molécule X est une chaîne alkylène qui contient de 1 à 10 groupes CH₂CH₂O ou de 1 à 5 groupes COCH₂NH.

4. Composés selon la revendication 1, **caractérisés en ce que** la partie de molécule X est une liaison directe ou présente l'une des structures suivantes: 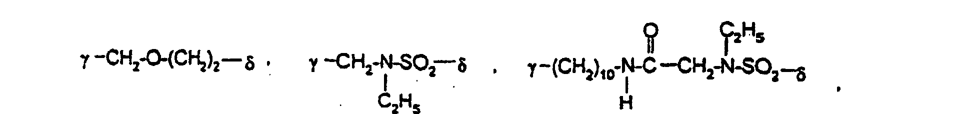
γ-(CH₂)₁₀-O-(CH₂)₂―δ . 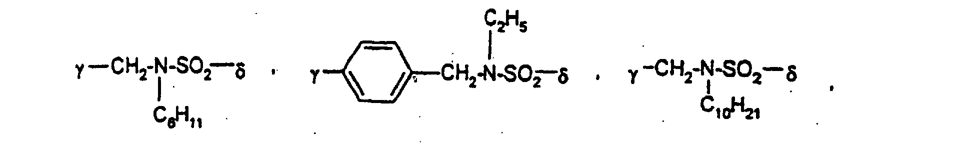 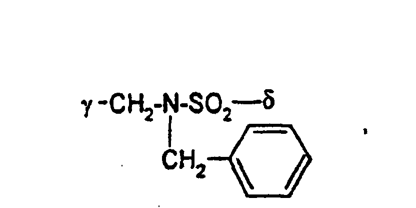
où γ se lie à G et δ à R^{F}.

5. Composés selon la revendication 1, **caractérisés en ce que** Y est une partie de molécule de l'une des structures suivantes:

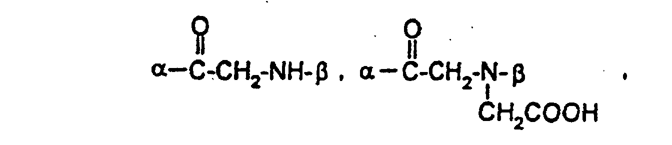

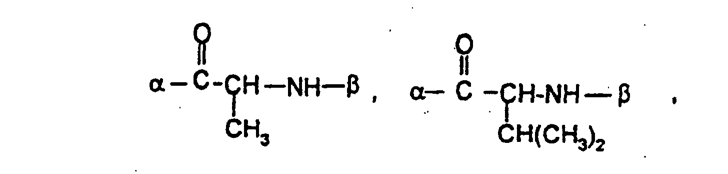

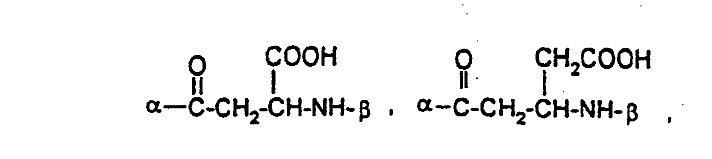

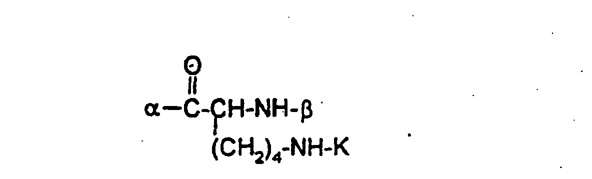

6. Composés selon la revendication 1, **caractérisés en ce que** K représente un complexe des formules générales V, VI, VII ou VIII, > 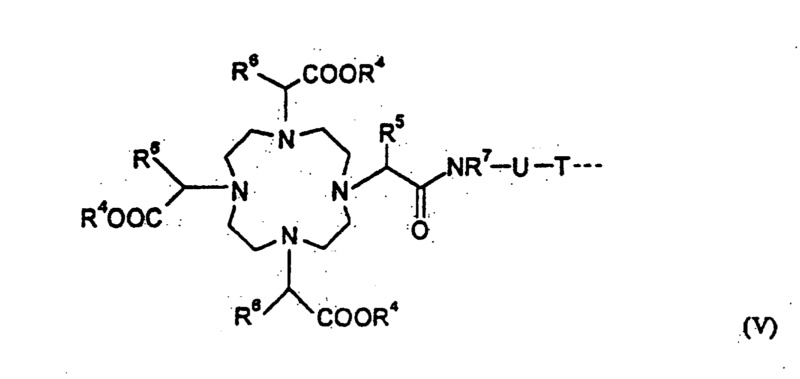 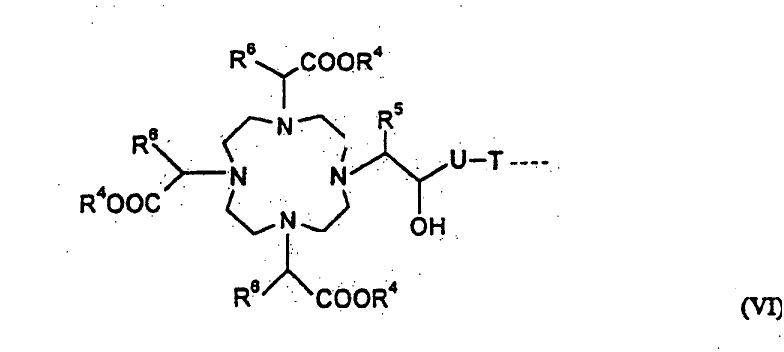 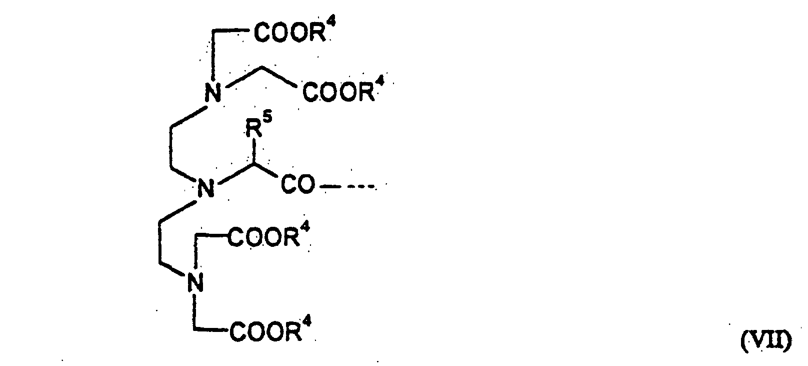 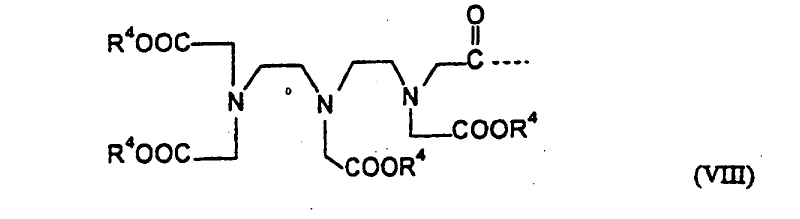 dans lesquelles
• R⁴ représente indépendamment un atome d'hydrogène ou un équivalent d'ions métalliques des éléments des numéros atomiques 20 - 32, 37 - 39, 42 - 44, 49 ou 57 - 83,
• R⁵ représente un atome d'hydrogène ou une chaîne alkyle en C₁ à C₃₀ linéaire, ramifiée, saturée ou insaturée, qui est éventuellement substituée par 1 à 5 groupes hydroxy, 1 à 3 groupes carboxy ou 1 groupe phényle et/ou éventuellement interrompue par 1 à 10 atomes d'oxygène, 1 groupe phénylène ou 1 groupe oxyphénylène,
• R⁶ représente un atome d'hydrogène, un reste alkyle en C₁ à C₇ à chaîne linéaire ou ramifiée, un reste phényle ou benzyle,
• R⁷ représente un atome d'hydrogène, un groupe méthyle
ou éthyle, qui est éventuellement substitué par un groupe hydroxy ou carboxy,
• U représente un groupe alkylène en C₁ à C₂₀ linéaire, ramifié, saturé ou insaturé, qui contient éventuellement 1 à 5 groupes imino, 1 à 3 phénylène, 1 à 3 oxyphénylène, 1 à 3 iminophénylène, 1 à 5 amide, 1 à 2 hydrazide, 1 à 5 carbonyle, 1 à 5 oxyéthylène, 1 urée, 1 thiourée, 1 à 2 carboxyalkylimino, 1 à 2 ester, 1 à 10 atomes d'oxygène, 1 à 5 de soufre et/ou 1 à 5 d'azote et/ou qui est éventuellement substitué par 1 à 5 groupes hydroxy, 1 à 2 mercapto, 1 à 5 oxo, 1 à 5 thioxo, 1 à 3 carboxy, 1 à 5 carboxyalkyle, 1 à 5 ester et/ou 1 à 3 amino, les groupes phénylène éventuellement contenus pouvant être substitués par 1 à 2 groupes carboxy, 1 à 2 sulfone ou 1 à 2 hydroxy,
• T représente un groupe -CO-β, -NHCO-β ou- NHCS-β, où β représente le site de liaison à Y.

7. Composés selon la revendication 6, **caractérisés en ce que** la chaîne alkylène en C₁ à C₂₀ représentant U contient les groupes -CH₂NHCO-, -NHCOCH₂O-, -NHCOCH₂OC₆H₄-, -N(CH₂CO₂H)-, -CH₂OCH₂-, -NHCOCH₂C₆H₄-, -NHCSNHC₆H₄-, -CH₂OC₆H₄-, -CH₂CH₂O- et/ou est substituée par les groupes -COOH, -CH₂COOH.

8. Composés selon la revendication 6, **caractérisés en ce que** U représente un groupe -CH2-, -CH₂CH₂-, -CH₂CH₂CH₂-, -C₆H₄-, -C₆H₁₀-, -CH₂C₆H₄-, -CH₂NHCOCH₂CH(CH₂CO₂H)-C₆H₄-, -CH₂NHCOCH₂OCH₂-, -CH₂NHCOCH₂C₆H₄-.

9. Composés selon la revendication 1, **caractérisés en ce que** K présente l'une des structures suivantes:

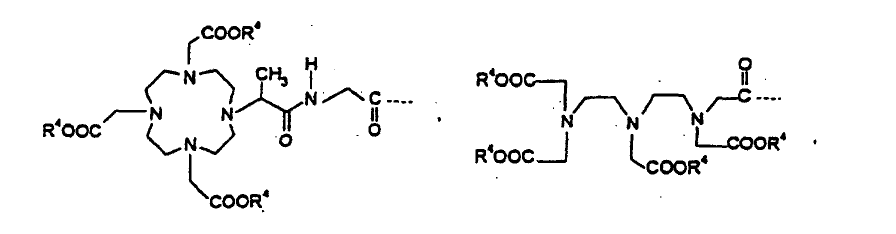

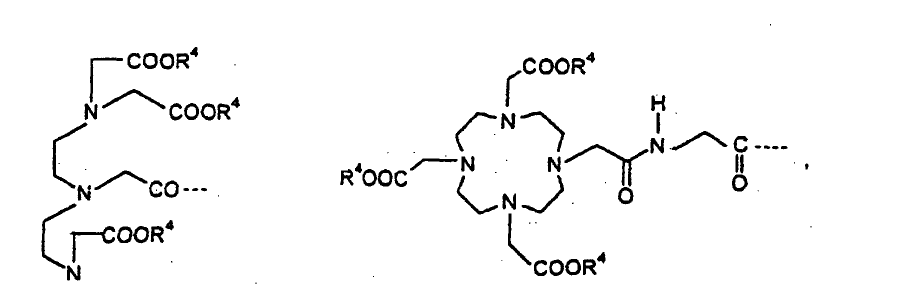

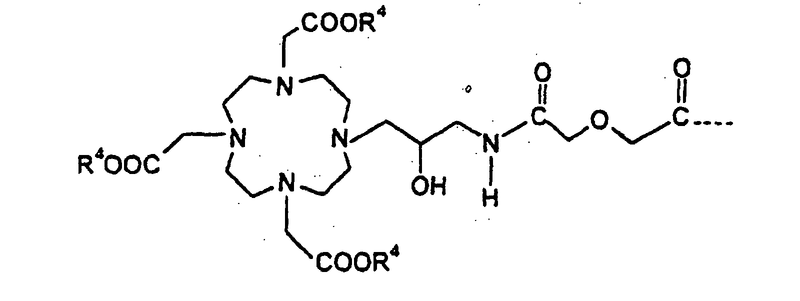

10. Composés selon l'une des revendications 1 à 9, **caractérisés en ce que** la chaîne perfluoralkylée R^{F} est -C₆F₁₃, -C₈F₁₇, -C₁₀F₂₁ ou -C₁₂F₂₅.

11. Procédé de préparation de composés perfluorés de formule générale I, **caractérisé en ce que** l'on fait réagir des composés de formule générale I'
A'-R^{F} (I')
dans laquelle A' représente un reste 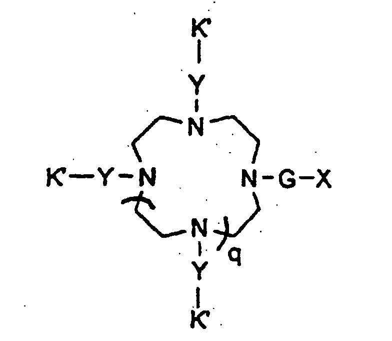 et K' représente K avec R⁴ dans le sens d'un atome d'hydrogène ou d'un groupe protecteur carboxy,
après scission des éventuels groupes protecteurs présents, de manière en soi connue, avec au moins un oxyde métallique ou un sel métallique d'un élément des numéros atomiques 20 - 32, 37 - 39, 42 - 44, 49 ou 57 - 83 et qu'éventuellement ensuite, dans les composés complexes ainsi obtenus, on substitue totalement ou partiellement les atomes d'hydrogènes acides encore présents par des cations de bases inorganiques et/ou organiques, d'acides aminés ou d'amides d'acides aminés.

12. Procédé de préparation de composés perfluoralkylés de formule générale I, **caractérisé en ce que** l'on fait réagir un composé de formule générale I" 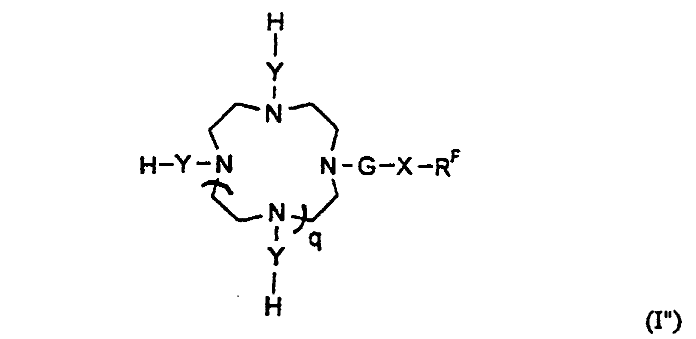 avec un complexe V' ou VI', 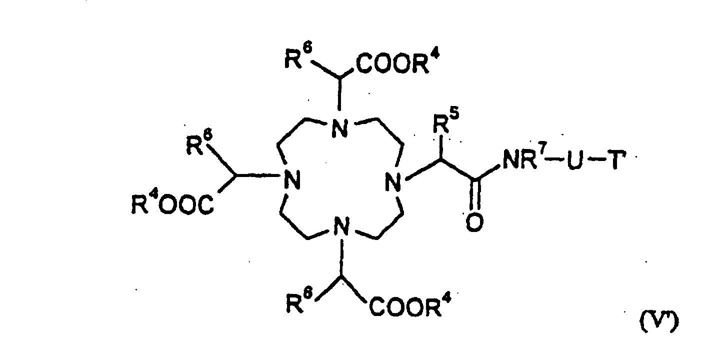 où T' représente un groupe -C*O, -COOH, -N=C=O ou -N=C=S et -C*O un groupe carboxyle activé, avec la condition qu'au moins deux (pour des métaux bivalents) ou trois (pour des métaux trivalents) des substituants R⁴ représentent un équivalent d'ions métalliques des éléments précités et qu'il se trouve éventuellement d'autres groupes carboxyle sous forme de leurs sels avec des bases inorganiques et/ou organiques, d'acides aminés ou d'amides d'acides aminés.

13. Agent pharmaceutique contenant au moins un composé physiologiquement compatible selon la revendication 1, éventuellement avec les additifs usuels en galénique.

14. Utilisation d'au moins un composé physiologiquement compatible selon la revendication 1, ou d'un agent pharmaceutique selon la revendication 13, pour la préparation d'agents de contraste pour le diagnostic et la spectroscopie RMN au ¹H.

15. Utilisation d'au moins un composé physiologiquement compatible selon la revendication 1, ou d'un agent pharmaceutique selon la revendication 13, pour la préparation d'agents de contraste pour diagnostic radiographique.

16. Utilisation d'au moins un composé physiologiquement compatible selon la revendication 1, ou d'un agent pharmaceutique selon la revendication 13, pour la préparation d'agents pharmaceutiques pour radiodiagnostic et radiothérapie.

17. Utilisation selon la revendication 14 ou 15 pour la préparation d'agents de contraste intravasculaires (agents Blood Pool).

18. Utilisation selon la revendication 14 ou 15 pour la préparation de produits pour lymphographie.

19. Procédé de préparation des agents pharmaceutiques selon la revendication 13, **caractérisé en ce que** l'on met les composés perfluoralkylés se trouvant dans de l'eau ou dans une solution saline physiologique, éventuellement avec les additifs usuels en galénique, sous une forme appropriée pour application entérale ou parentérale.
